# EUROPÄISCHE PATENTSCHRIFT

(11) **EP 2 673 640 B1**
(45) Veröffentlichungstag und Bekanntmachung des Hinweises auf die Patenterteilung: **30.09.2020**
(21) Anmeldenummer: 12704511.0
(22) Anmeldetag: 10.02.2012
(51) Int. Cl.: G01N 33/68, C12N 9/64

(54) **DETEKTION UND ENTFERNUNG VON MISSGEFALTETEN PROTEINEN/PEPTIDEN**
DETECTION AND REMOVAL OF MISFOLDED PROTEINS/PEPTIDES
DÉTECTION ET RETRAIT DE PROTÉINES/PEPTIDES À MAUVAIS REPLIEMENT

(30) Priorität: 10.02.2011 DE 102011003944
(43) Veröffentlichungstag der Anmeldung: 18.12.2013
(73) Patentinhaber: Oxprotect GmbH, 48149 Münster (DE)
(72) Erfinder: KEHREL, Beate, 43431 Rheine (DE); BRODDE, Martin, 48329 Havixbeck (DE)
(74) Vertreter: Weickmann & Weickmann PartmbB
(86) Internationale Anmeldenummer: PCT/EP2012/052330
(87) Internationale Veröffentlichungsnummer: WO 2012/107567

(56) Entgegenhaltungen:
- EP-A1- 1 380 290
- WO-A2-2008/047370
- WO-A2-2008/047370
- US-A1- 2005 053 993
- K. ETO: "RGD-independent Binding of Integrin alpha 9beta 1 to the ADAM-12 and -15 Disintegrin Domains Mediates Cell-Cell Interaction", JOURNAL OF BIOLOGICAL CHEMISTRY, Bd. 275, Nr. 45, 3. November 2000 (2000-11-03), Seiten 34922-34930, XP055028844, ISSN: 0021-9258, DOI: 10.1074/jbc.M001953200
- MAYS CHARLES E ET AL: "Plasminogen stimulates propagation of protease-resistant prion protein in vitro", THE FASEB JOURNAL, FEDERATION OF AMERICAN SOCIETIES FOR EXPERIMENTAL BIOLOGY, UNITED STATES, Bd. 24, Nr. 12, 23. August 2010 (2010-08-23), Seiten 5102-20101201, XP009153686, ISSN: 1530-6860, DOI: 10.1096/FJ.10-163600
- HATCHER K ET AL: "Cryptic Peptides of the Kringle Domains Preferentially Bind to Disease-Associated Prion Protein", JOURNAL OF ALZHEIMER'S DISEASE, IOS PRESS, AMSTERDAM, NL, Bd. 16, Nr. 2, 1. Februar 2009 (2009-02-01), Seiten 421-431, XP008107267, ISSN: 1387-2877, DOI: 10.3233/JAD-2009-0980
- E. Herczenik ET AL: "Molecular and cellular aspects of protein misfolding and disease", The FASEB Journal, vol. 22, no. 7, 1 July 2008 (2008-07-01), pages 2115-2133, XP55277926, US ISSN: 0892-6638, DOI: 10.1096/fj.07-099671

## Beschreibung

Die Erfindung betrifft die Verwendung von Peptiden, Polypeptiden und Proteinen zum Nachweis, zur Quantifizierung und zur Entfernung von missgefalteten Proteinen/Peptiden.

Die Erfindung bezieht sich auf das Gebiet der Detektion und Messung von missgefalteten Proteinen/Peptiden. Insbesondere auf die Detektion und Messung von missgefalteten Proteinen/Peptiden in Körperflüssigkeiten, auf Zelloberflächen und Geweben von Mensch und Säugetieren, auf die Detektion von missgefalteten Proteinen/Peptiden in Testreagenzien für Forschungs- und oder diagnostische Zwecke und in Medikamenten oder Medikamentzusatzstoffen, sowie die Entfernung von missgefalteten Proteinen/Peptiden aus Testreagenzien für Forschungs- und oder diagnostische Zwecke und aus Medikamenten oder Medikamentzusatzstoffen. Des Weiteren enthält die Erfindung Nachweissubstanzen und Methoden zur Detektion von Biofilmen, eine Methode zur Prüfung der Hämokompatibilität von Materialien und zur Optimierung von Medizinprodukten, Reagenzien zum Anreichern von Mikroorganismen zur Erleichterung der Diagnostik und Qualitätskontrolle von Biopharmaka und Nachweissubstanzen für das Screening nach Vorstufen von Amyloiden, welche für technische Zwecke genutzt werden können.

### Hintergrund der Erfindung:

Die Proteinfaltung ist der Prozess, durch den Proteine ihre dreidimensionale Struktur erhalten.

Proteine müssen, um ihre spezifischen biologischen Aufgaben erfüllen zu können, in einer dreidimensionalen Struktur gefaltet werden. Diese Struktur ist aber in vielen Fällen nicht stabil. Proteine können partiell oder komplett entfaltet und dabei partiell oder komplett denaturiert werden. Bei Missfaltung wird ein Protein in eine dreidimensionale Struktur überführt, die nicht dem nativen Zustand entspricht. Die Neigung zur partiellen oder kompletten Entfaltung oder Umfaltung der Struktur wohnt allen Proteinen inne.

Während die Mechanismen zur Kontrolle von missgefalteten Proteinen/Peptiden, speziell nach Neusynthese von Polypeptiden und bei Mutationen, innerhalb der Zelle weitestgehend bekannt sind, werden extrazellulär befindliche missgefaltete Proteine und ihre Bedeutung erst seit kurzem erforscht.

Zellen verfügen über eine ausgeklügelte Qualitätskontrolle für Proteinfaltungen und korrigieren oder recyceln im Normalfall über Chaperone, Ubiquitin Proteasome System falsch gefaltete Proteine innerhalb der Zelle.

Die Auslöser für Missfaltungen an extrazellulären Proteinen sind vielfältig. So kann zum Beispiel Kontakt von Proteinen/Peptiden an Glas, Lipide, Zellmembranen ¹ Metalle und Metallverbindungen und Zuckerpolymeren solche Missfaltungen hervorrufen ². Selbst Medikamente können missgefaltet werden ³.

Das Phänomen der Missfaltung von Proteinen ist besonders für die Untersuchung der Biokompatibilität von Oberflächen von blutführenden Transplantaten wie Herzunterstützungssysteme und künstliche Herzklappen von besonderer Bedeutung. Missfaltung von Proteinen/Peptiden kann aber insbesondere auch durch posttranslationale Proteinmodifikationen, wie z. B. Glykierung ^{4,5}, welche zu "advanced glycation" Endprodukten führt, oder durch Modifikation mit ROS ⁶, HOCI, Aldehyden, wie Acrolein (Propenal), 4-Hydroxy-2-nonenal, und Malondialdehyd ⁷, Einfluss von Peptidyl-Prolylcis/trans-Isomerasen und Proteindisulfidisomerasen ⁸, enzymatische Spaltung ⁹, Veränderungen durch Glykosilierungen oder Glykosidasen hervorgerufen werden. Die Zusammensetzung der Umgebungsmilieus, Detergenzien, Vorkommen von Zink ¹⁰, Cu, Pb oder Ethanol, pH-Wert, Konzentrationsdichte von Biomolekülen, Kontakt zu Membranen und auch der Gehalt von Cholesterin und Sphingolipiden in der Membran ^{11,12}, Druck, Scherkräfte und Temperaturveränderungen, selbst der Kontakt zu anderen Biomolekülen spielen bei der Missfaltung von Proteinen eine Rolle.

Mikroorganismen produzieren gezielt missgefaltete Proteine, um sich damit an Wirtszellen anzuheften ¹³. So gelangen bei Infektionen mit Bakterien, Viren und pathogenen Pilzen missgefaltete Proteine in den Blutkreislauf des Wirtes. Dieses Phänomen könnte auch für das Krankheitsgeschehen der Sepsis von Bedeutung sein. Interessanterweise scheinen auch Säugetiere missgefaltete Proteine /Peptide gezielt zu nutzen. So verhalten sich die α-Defensine humaner Granulozyten in vielerlei Hinsicht wie missgefaltete Proteine. Sie lagern sich im Kontakt mit Membranen zusammen, binden an das generelle Bindeprotein für missgefaltete Proteine t-PA und binden, wie wir kürzlich zeigen konnten, an das Chaperon GRP78/BIP und können zudem Thrombozyten aktivieren¹⁴.

Levinthal stellte 1968 fest ¹⁵, dass die korrekte Faltung eines neu synthetisierten Polypeptides länger dauern würde als die Lebensdauer des Universums, wenn alle möglichen Konformationen in einer nach dem Zufallsprinzip ausgewählten Suche ausprobiert würden. Da die Proteinfaltung aber nur Sekunden oder gar Millisekunden dauert, nimmt man an, dass stabile Zwischenzustände existieren. Die native Konformation eines Proteins entspricht dem Zustand der niedrigsten freien (Gibbs) Enthalpie ¹⁶. Kleine Veränderungen im Umgebungsmilieu können, wie oben beschrieben, Oligopeptide/Polypeptide/Proteine zur partiellen Entfaltung bringen, sodass diese wieder stabile Zwischenzustände in ihrer Konformation einnehmen. Partiell entfaltete /missgefaltete Proteine neigen zur Selbstassoziation, was zu Oligomerisierung, Aggregatenbildung und Ausbildung von Fibrillen führen kann.

In den 1980er Jahren wurden Enzyme entdeckt, welche missgefaltete Proteine erkennen und ihre hydrophoben Oberflächen abdecken können, die sogenannten Chaperone. Diese können auch in begrenztem Umfang Fehlfaltungen unter Energiezufuhr reparieren. Probleme dabei können aber auftreten und die Zahl der Erkrankungen, welche auf Missfaltungen von Proteinen/Peptiden zurückgeführt werden können, steigt stetig. Diese zeigen, wie wichtig eine korrekte Proteinfaltung ist und dass die Proteinmissfaltung ein Schlüsselmechanismus für Erkrankungen ist, welche starke Einschränkungen und Behinderungen mit sich bringen. Bekannt ist das Auftreten von missgefalteten Proteinen insbesondere bei neurodegenerativen Erkrankungen wie Alzheimer, BSE, CJD (Creutzfeldt-Jakob-Krankheit), ALS (Amyotrophe Lateralsklerose) oder Parkinson ¹⁷. Missfaltung von Proteinen und Proteinaggregation wird immer mehr auch als ein Mechanismus angesehen, welcher andere massive Gesundheitsprobleme auslöst wie die Arteriosklerose, welche zu periphären Gefäßverschlüssen, Herzinfarkt und Schlaganfall führt, Amyloidose in Zusammenhang mit Dialyse, Präeklampsie, einer hypertensiven Schwangerschaftserkrankung ¹⁸ und die Immunogenität von Protein/Peptid haltigen Medikamenten ¹⁹.

Bei einer großen Zahl der Fehlfaltungen, die als Krankheitsursache nachgewiesen wurden, werden die Proteine in hoch-geordnete Aggregate und Fibrillen ("Amyloide") überführt, die sich der zellulären Qualitätskontrolle und der Protein-Degradation entziehen. Heute weiß man aber, dass besonders die niedermolekularen Intermediate, Oligomere und Aggregate für die Krankheitsursachen verantwortlich sind.

Das Risiko an einer Erkrankung aufgrund Missfaltung von Proteinen/Peptiden zu leiden steigt mit dem Alter. Die Forschung nimmt an, dass das Qualitätskontrollsystem für Proteinfaltungen mit dem Alter schlechter funktioniert und daher missgefaltete Proteine und die daraus entstehenden Erkrankungen häufiger vorkommen ²⁰. Der Stand des Wissens unterstützt die Ansicht, dass alle missgefalteten Proteine einen gemeinsamen Strukturmechanismus tragen, welcher in den meisten Fällen für die Zytotoxizität verantwortlich ist. So können Oligomere aus missgefalteten Proteinen mit Zellmembranen wechselwirken und Strukturen bilden, welche die selektive lonenpermeabilität zerstört. Dies kann zum Zelltod führen. Missgefaltete Proteine beeinflussen aber auch direkt die Fibrinolyse und das Kallikrein System, da eine Fibronektin type-I Domäne in den Serinproteasen Gewebsplasminogenaktivator (tPa) und FXII solche Proteine direkt erkennt ^{21,22}.

Die Aktivierung der Fibrinolyse und des Kontaktaktivierungssystems könnte als Mechanismen angesehen werden, gefährliche extrazelluläre missgefaltete Proteine/Peptide abzubauen, bevor sie durch Fibrillenbildung resistenter gegen Proteasen werden ^{23,24}. In den letzten Jahren wurden Chaperone, wie das GRP78, auch BIP genannt, und andere auch auf Zelloberflächen gefunden. Nahm man zuerst an, dass GRP78 nur auf Tumorzellen vorkommt, so weiß man doch heute, dass GRP78 auch auf gestressten anderen Zellen detektiert werden kann. Autoantikörper gegen zellmembranständiges GRP78 findet man bei vielen Tumorpatienten insbesondere bei Prostata-, Ovar- oder Magenkrebs ²⁵. In Abhängigkeit von Stress, wie z.B. Hypoxie, Mangel oder Überfluss an Glucose und Einwirken von Scherkräften, findet man z.B. GRP78 auf Endothelzellen, Kardiomyozyten, Monozyten/Makrophagen und glatten Muskelzellen. GRP78 wird besonders in fortgeschrittenen arteriosklerotischen Läsionen und auf der Oberfläche der fibrösen Kappe in Apolipoprotein-defizienten Mäusen und im Menschen gefunden. Insbesondere ist GRP78 dort an den Gefäßstellen zu finden, welche aufgrund von rheologischen Bedingungen besonders anfällig für arteriosklerotische Veränderungen sind ²⁶⁻²⁸. Chen et al fanden, dass Statine (HMG-CoA Reduktaseinhibitoren) die Expression von GRP78 erhöhen²⁹.

Überexpression von GRP78 inhibiert die prokoagulante Aktivität von Tissue Faktor³⁰. GRP78 kann als Regulator der Tissue Faktor abhängigen Gerinnung angesehen werden ³¹. Unabhängig voneinander entdeckten die Arbeitsgruppen um Lina Badimon und Beate Kehrel GRP78 auch auf der Plasmamembran von Thrombozyten ^{32,33}. Herczenik und andere konnten zeigen, dass Thrombozyten durch missgefaltete Proteine aktiviert werden ³⁴. Die Gruppe um Beate Kehrel konnte zeigen, dass die Thrombozytenaktivierung durch die unterschiedlichen missgefalteten Proteine HOCI-modifiziertes Albumin, EAP aus S. aureus ³⁵, α-Defensin auf humanen neutrophilen Granulozyten ³⁶ und das amyloidogene TSP-1 Peptid RFYVVMWK durch GRP78 vermittelt wird. GRP78 scheint somit ein Rezeptor auf Thrombozyten für veränderte Strukturen/Missfaltungen in Proteinen/Peptiden zu sein. Die Funktion von GRP78 ist abhängig von ATP ³⁷. Deng et al konnten zeigen, dass Aspirin die Aktivität von GRP78 in Fibroblasten hemmt, indem es seine ATPase-Aktivität hemmt ³⁸. Wissenschaftler der Firma OxProtect GmbH konnten zeigen, dass Aspirin/ASS über Hemmung der ATPase-Aktivität des GRP78 die Aktivierung vom Thrombozyten durch missgefaltete Proteine hemmt. So können missgefaltete Protein spezifisch eingesetzt werden, um das Ansprechen von Patienten auf eine Therapie mit ASS/Aspirin zu monitoren HOCI-modifizierte missgefaltete Proteine interagieren zudem direkt mit den aktiven Metaboliten der Thienopyridine. Zwei Möglichkeiten, warum HOCI-modifizierte Proteine die Wirkung von Thienopyridinen auf die ADP induzierte Thrombozytenaktivierung negativ beeinflussen können, sind denkbar. Erstens können HOCI-modifizierte Proteine, aktive Metaboliten der Thienopyridine, wegfangen werden, sodass weniger aktiver Metabolit zur Hemmung des ADP-Rezeptors P2Y12 verbleibt. Immobilisierte HOCI-modifizierte Proteine wurden in arteriosklerotischen Gefäßwänden gefunden. Sugiyama et al. fanden HOCI-modifizierte Proteine bei Untersuchungen von arteriosklerotischen Plaques, welche einen tödlichen Schlaganfall ausgelöst hatten, direkt unter dem Thrombus auf der Plaque-Erosionsstelle ³⁹. Wissenschaftler der Firma OxProtect GmbH konnten auf HOCI-modifizierten Proteinen reaktive Gruppen nachweisen, welche mit hoher Affinität an freie Thiolgruppen binden. Diese reagieren mit den freien Thiolgruppen der aktiven Metabolite der Thienopyridine, wie Clopidogrel und Prasugrel. Da die Thiolgruppen der aktiven Metabolite der Thienopyridine für die Hemmung von P1Y12 essentiell sind, stehen freie Metabolite zur Inhibition von P2Y12 in geringerer Konzentration zur Verfügung. Zweitens haben wahrscheinlich besonders die Patienten, welche ein hohes Risiko für vaskuläre Ereignisse (Herzinfarkt, Schlaganfall, peripherer Gefäßverschluss) haben, eine erhöhte Konzentration an HOCI-oxidierten Proteinen im Blut, denn bei diesen Patienten wurden hohe Konzentrationen von freier Myeloperoxidase im Plasma oder Serum gemessen, dem Enzym, welches für die Produktion von HOCl verantwortlich ist, wie viele Arbeiten zeigen ⁴⁰⁻⁵².

Da HOCI-modifizierte Proteine als missgefaltete Proteine selbst starke Thrombozytenaktivatoren sind, wie EP 1 328 289 zeigt, und missgefaltete Proteine mit GRP78 auf der Oberfläche von arteriosklerotischen Plaques reagieren können, ist es wahrscheinlich, dass sie selbst maßgeblich an dem Krankheitsgeschehen beteiligt sind und dass wenigstens ein Teil des Schutzmechanismus von Thienopyridinen und ASS/Aspirin auf der Hemmung der pathogenen Wirkung von missgefalteten Proteinen beruht.

Im Zusammenhang mit der vorliegenden Erfindung wurde von den Erfindern außerdem gefunden, dass HOCI-modifizierte Proteine in Labormethoden zum Monitoring einer Therapie mit Thienopyridinen eingesetzt werden können.

Ein Rezeptor für missgefaltete Proteine auf Endothelzellen, glatten Muskelzellen und Monozyten/Makrophagen ist das GRP78. Bindung von Polypeptiden, wie Kringel 5 aus Plasminogen oder ADAM15, entscheiden über Apotose oder Proliferation, über Leben oder Tod der Endothelzelle ⁵³⁻⁵⁵. Auf Makrophagen ist GRP78 mit G alpha q11 assoziiert. Aktiviertes alpha 2 Makroglobulin löst über GRP78 ein Signalkette in Makrophagen und Tumorzellen aus ⁵⁶. Autoantikörper, welche an GRP78 auf Monozyten binden, induzieren die Produktion von TNF alpha ⁵⁷.

Missgefaltete Proteine aktivieren die Fibrinolyse. Dies kann, wie das Beispiel für Endostatin, ein missgefaltetes Fragment von Kollagen XVIII, zeigt, bis zum Ablösen der Endothelzellen von der subendothelialen Matrix führen. ⁵⁸. Es ist wahrscheinlich, dass die beschriebenen wichtigen Zellfunktionen des GRP78 von seinen missgefalteten Proteinliganden beeinflusst werden und damit das Vorhandensein und die Konzentration von missgefalteten Proteinen über so wichtige Fragen wie Zellproliferation oder Apoptose, Angiogenese, Wundheilung und Entstehung einer Thrombose mit entscheidet.

GRP78 ist an der Internalisierung von Mikroorganismen und Proteinen in Zellen hinein beteiligt. Auch an GRP78 auf Zelloberflächen gebundene Peptide können internalisiert werden ⁵⁹.

Dies könnte zu Stress im retikuloendothelialen System führen. ER-Stress wurde kürzlich als eine der wichtigsten Triebfedern für die Arteriosklerose, Typ II Diabetes, Obesitas und ihre Folgeerkrankungen erkannt ⁶⁰⁻⁶³. Ein vollständiges Fehlen von GRP78 ist mit dem Leben nicht vereinbar. *Grp78*^{+/-} heterozygote Mäuse sind resistent gegen Diät induzierte Hyperinsulinämie, Entstehung einer Fettleber, Entzündungserscheinungen im weißen Fettgewebe und Hyperglykämien. Interessanterweise nahmen diese Mäuse trotz hochkalorischer, fettreicher Diät nicht an Gewicht zu ^{64,65}. GRP 78 Heterozygotie führte im weißen Fettgewebe bei fettreicher Diät zu einer Steigerung der adaptiven Antwort auf ungefaltete Proteine (unfolded protein response (UPR)) und verbesserte die Qualitätskontrolle des Endoplasmatischen Retikulums. Dadurch wurde in den *Grp78*^{+/-}Mäusen Obesitas und Typ 2 Diabetes verbessert. Das Ergebnis macht klar, dass die Qualitätskontrolle gegen missgefaltete Proteine für die Homeostase der Energybalance und den Glucosemetabolismus wichtig ist.

ER-Stress kann zu Apoptose von glatten Muskelzellen führen. Daher ist es wahrscheinlich, dass ER-Stress zur Plaqueruptur beiträgt ⁶⁶. Es liegt auch nahe, dass durch Bildung seines Liganden OxLDL, welches auch ein missgefaltetes Protein ist, die Schaumzellbildung, einer der ersten Schritte der Arteriosklerose, moduliert oder gar reguliert wird.

Neben zellständigen Chaperonen gibt es extrazelluläre lösliche Proteine, welche chaperonähnliche Funktionen ausüben können ⁶⁷. Zu diesen Proteinen gehören z.B. das alpha 2 Makroglobulin, Clusterin (Apolipoprotein J), serum amyloid P und Haptoglobin ⁶⁸⁻⁷⁰. Diese Proteine binden an missgefaltete Proteine und vermitteln die Aufnahme der Komplexe in die Zellen hinein durch Scavenger Rezeptoren wie "low density lipoprotein receptor related protein" (LRP, CD68, CD91), CD36, Scavenger Rezeptor A, Scavenger Rezeptor B-I, und RAGE. Komplexe auf missgefalteten Proteinen und Chaperon-ähnlichen löslichen Proteinen können im Blut, Plasma, Serum detektiert werden.

Auch Apolipoproteine, insbesondere Apolipoprotein E (ApoE), Komplement Faktoren, wie C1q und Heparansulfat-Proteoglykane können missgefaltete Proteine binden und damit dazu beitragen, dass sie ihre schädlichen Wirkungen auf den Organismus zeigen. Dies wurde für das Amyloid Beta, welches für Morbus Alzheimer verantwortlich ist, beschrieben ⁷¹⁻⁷³. Isoformen und Differenzen zwischen Spezies beeinflussen die Rolle von Apo E für die Selbstassoziation/aggregation und die Entfernung von Amyloid beta Protein.

Komplexe aus Apolipoproteinen oder Komplementfaktoren und missgefalteten Proteinen könnten daher gute Marker für Erkrankungen sein, bei denen extrazelluläre missgefaltete Proteine eine Rolle spielen, und Heparansulfat-Proteoglykane könnten zur Erkennung von missgefalteten Proteinen beitragen. Ob ApoE über Reaktion mit extrazellulären missgefalteten Proteinen zur Entstehung von Arteriosklerose beiträgt, kann zurzeit nur spekuliert werden.

Arteriosklerose und die daraus entstehenden vaskulären Erkrankungen, wie Herzinfarkt, Schlaganfall, peripherer Gefäßverschluss und Morbus Alzheimer sind konvergente Erkrankungen. Beiden Krankheiten gemeinsam sind eine Basis auf dem Boden einer Entzündungsreaktion, die Bedeutung von Cholesterin und Sphingolipiden in der Zellmembran und das Auftreten von missgefalteten Proteinen ⁷⁴.

Im Zusammenhang mit Arteriosklerose und ihren Folgeerkrankungen, wie z.B. Angina pectoris, Herzinfarkt, TIA, Schlaganfall, periphere Gefäßverschlüsse, sind insbesondere Missfaltungen an Proteinen interessant, welche durch eine Reaktion mit einem Produkt der Myeloperoxidase oder durch sPLA2 verursacht werden, sind die beiden Enzyme selbst doch gute Biomarker für ein erhöhtes vaskuläres Risiko ⁷⁵⁻⁷⁷. Inhibitoren gegen sPLA2 befinden sich als Therapeutika in klinischen Prüfungen. Eine Bestimmung von durch sPLA2 Aktivität missgefaltetem LDL wäre daher auch zur Überwachung der Therapie mit PLA2-Inhibitoren wünschenswert. Amyloide Insulinablagerungen wurden bei Typ 2 Diabetikern und anderen älteren Patienten beobachtet.

Hyperinsulinämie und Hyperglykämie, verursacht durch Insulin Resistenz, führen zu verstärkter Plaquebildung um Neurite bei Alzheimer Patienten ^{78,79}. Eine kritische Eigenschaft des Insulins ist seine Neigung zur Bildung amyloidaler Fibrillen. Missgefaltetes Insulin wurde in Form von fibrillären Insulinablagerungen bei Diabetikern gefunden ^{80,81}. Unlösliche Insulinfibrillen können zum Verstopfen von Injektionskanülen bei der Applikation der pharmazeutischen Insulinpräparate führen und werden auch für immunologische Unverträglichkeitsreaktionen verantwortlich gemacht, die gelegentlich während der Therapie mit Insulinpräparaten auftreten können ^{82,83}. Vorstufen der Fibrillen von missgefalteten Proteinen dienen als eine Art Kristallisationskeim für die Bildung größerer Aggregate und Fibrillen. Neben Ablagerungen bei Typ II-Diabetes-Patienten ist die Fibrillenbildung jedoch vor allem ein großes Problem bei der Herstellung, Lagerung und der Verabreichung von gelöstem Insulin in Diabetespräparaten. Vorstufen der Fibrillen von missgefalteten Proteinen dienen als eine Art Kristallisationskeim für die Bildung größerer Aggregate und Fibrillen. Daher ist es für die Produktion von Insulin und für die Applikation im Patienten wichtig, schon partiell ungefaltetes/missgefaltetes Insulin erkennen und aus dem Medikament und seinen Präparationsvorstufen eliminieren zu können. WO/2004/013176 beschreibt ein Verfahren zur Reinigung von Preproinsulin, welches das Auftreten von Fibrillenbildung des Insulins verringert.

Da Missfaltung von extrazellulären Proteinen ein so wichtiges Phänomen in der Pathogenese von Erkrankungen ist, aber missgefaltete Proteine auch als Nanomaterialien nützlich sind, wurden Methoden zur Detektion von missgefalteten Proteinen/Peptiden beschrieben.

Missgefaltete Proteine neigen zur Selbstassoziation ⁸⁴. Sie können dabei Oligomere, amorphe Aggregate von unterschiedlicher Größe oder regulär angeordnete Fibrillen bilden. Solche Fibrillen werden als Amyloide bezeichnet. Andere Autoren beschreiben heute schon die missgefalteten Protein/Peptidmonomere, -oligomere und -aggregate als Amyloid. Auch in vitro hergestellte Fibrillen aus missgefalteten Proteinen werden von manchen Autoren als Amyloide bezeichnet ⁸⁵. Fibrillen haben meistens einen mittleren Durchmesser von ca. 10 nm und eine unterschiedliche Länge. Extrazelluläre amyloide Ablagerungen, wie sie bei der klassischen Amyloidose auftreten, lassen sich mit Kongorot-Färbung unter polarisiertem Licht, welches zu einer grünliche Doppelbrechung ("apple-green bi-refringence" so genannter Dichroismus) führt, nachweisen ^{86, 87}. Andere Nachweismethoden für amyloide Fibrillen sind die Thioflavin S-Fluoreszenz oder die Analyse der Fibrillen mittels Röntgenstrahlbrechung. Beide Nachweisverfahren erfassen missgefaltete Protein/Peptidmonomere, -oligomere und -aggregate nicht oder äußerst schlecht.

Anfänglich wurden nur die fibrillären Strukturen als pathogen erkannt, welche die Grundlage für die klassischen Amyloidosen ^{88, 89} bilden, wie zum Beispiel Immunglobulin-Leichtketten ⁹⁰, Amyloid A ⁹¹, Transthyretin ⁹², Cystatin C, Apolipoprotein A-I ⁹³, Gelsolin, Fibrinogen Aa-Kette, Lysozym, Apolipoprotein A-II (2001), Islet Amyloid Polypeptid (IAPP) ^{94, 95}, Leukozyten Chemotaktischer Faktor 2, LECT2 ⁹⁶, Alzheimer amyloid beta-peptide ⁹⁷, Prion Protein ^{98- 100}, Beta2 Microglobulin ¹⁰¹.

Nachdem sich aber nun gezeigt hat, dass die Missfaltung von Proteinen nicht auf wenige Proteine beschränkt ist, sondern ein generelles Phänomen ist, besteht die Notwendigkeit, auch nichtfibrilläre Konformationen von missgefalteten Proteinen zu erkennen. Dabei gilt es Mechanismen zu finden, welche für alle missgefalteten Proteine/Peptide, oder wenigstens für einen größeren Teil dieser Proteine/Peptide zur Identifikation, Bindung, Anreicherung und Eliminierung genutzt werden können.

Missgefaltete Proteine sind sehr heterogen. Sie umfassen monomere missgefaltete Proteine/Peptide, kleine bis größere Oligomere von missgefalteten Proteinen/Peptiden, große, amorph aussehende Aggregate von missgefalteten Proteinen/Peptiden, bis hin zu großen geordneten fibrillären Strukturen. Allen gemeinsam sind, unabhängig von der Aminosäuresequenz, bestimmte Eigenschaften, welche für die Forschung, Heilkunst und kommerzielle Anwendung genutzt werden können. EP 1 380 290 beschreibt die Bindung von missgefalteten Proteinen an den Gewebsplasminogenaktivator (tPa), die Aktivierung der Fibrinolyse durch missgefaltete Proteine und die Nutzung der Fibrinolyse zur Bekämpfung von missgefalteten Proteinen.

EP 2 007 800 beschreibt Methoden missgefaltete Proteine mit einer Crossbeta-Struktur durch Binding an Chaperone, insbesondere an GRP78/BiP, Clusterin, HSP72 oder Haptoglobin zu erkennen. Es werden auch HSP60, HSP90, DNAK, HGFA, tPA, Plasminogen, Faktor XII, IVIg, und die Zellrezeptoren low density lipoprotein receptor related protein (LRP, CD91), CD36, scavenger receptor A, scavenger receptor B-I, RAGE, als Bindproteine genannt.

EP 1 820 806 beschreibt einen Antikörper, welcher missgefaltete Proteine erkennt.

EP 2 058 000 beschreibt die Verstärkung von Immunreaktionen durch missgefaltete Proteine und die Nutzung von missgefalteten Proteinen zur Unterstützung der Immunantwort. WO 2010/059046 beschreibt missgefaltete Proteine als Carrier in Impfstoffen.

WO 2007/008073 beschreibt die Sinnhaftigkeit missgefaltete Proteine zu detektieren und zu messen indem man den Gehalt an missgefalteten Proteinen in einer Probe mit dem in einer Referenzprobe vergleicht, wobei die Testprobe so verändert worden ist, dass eine Änderung im Gehalt an missgefalteten Proteinen zu erwarten ist. Als Nachweismethode wird die Aktivierung der Fibrinolyse über tPa und die Aktivierung des Kalikreinsystems über FXIIa, welche mit tPa homologe Bereich teilt, beschrieben. In der Arbeit sind als weitere Bindesubstanzen für missgefaltete Proteine Thioflavin T (ThT), Congo Red, ThT, rekombinante Fingerdomänen von tPA, FXII, HGFA und Fibronectin, die vollständigen Proteine tPA, FXII, HGFA, Fibronectin; serum amyloid P component (SAP), Antikörper gegen missgefaltete Proteine und ein lösliches Fragment des Rezeptors für "advanced glycation end- products" (sRAGE) beschrieben.

Eto et al., J. Biol. Chemistry, Vol. 275, No. 45, November 10, (2000), Seiten 34922-34930 beschreiben die spezifische Interaktion von Integrin α₉β₁ mit den rekombinanten Disintegrin-Domänen von ADAM12 und ADAM15 und die mögliche biologische oder pathologische Signifikanz dieser Interaktion.

WO 2008/047370 A2 beschreibt Zusammensetzungen und Verfahren zur Induktion der Angiogenese unter Verwendung eines Peptids, welches eine Aminosäuresequenz HWRR umfasst.

US 2005/005399 A1 beschreibt die Verwendung eines als GPR78 bezeichneten Rezeptors und anderer Endothelzellrezeptoren.

Herczenik et al., FASEB J., 2008, Vol. 22, 2115-2133 beschreiben molekulare und zelluläre Aspekte der Proteinmissfaltung und damit verbundener Erkrankungen.

### Probleme, welche durch die Erfindung gelöst werden.

Für Verfahren zur Detektion, Messung, Imaging, Anreicherung von Proteinen/Peptiden, Depletion/Abreicherung von Proteinen/Peptiden, Überwachung von Therapien, welche auf der Bindung eines Testreagenzes mit einem Protein/Peptid beruht, welches missgefaltet ist, ist es günstiger, wenn das Testreagenz nicht selbst ein Protein oder Polypeptid ist. Als Testreagenz fungierende Proteine, wie sie in den oben genannten Patentanmeldungen beschrieben werden, können selbst durch vielerlei Umstände, wie z. B. Lagerung, Oxidation, Temperatur, Einfluss von Lipiden, insbesondere freien Fettsäuren und Cholesterin, Glykierungen, Einfluss von Membranen, Einfluss von Verfahren zur Virusinaktivierung wie Solvent-Detergent-(S/D)-Verfahren, Pasteurisierung, Trockenerhitzung, pH4-Verfahren, Nanofiltration, lonenstärke, Bestrahlung, Aufkonzentration, Einfrieren und Auftauen, Kontakt zu ungünstigen Materialien, missgefaltet werden. Bei Einsatz eines Proteins oder Polypeptides als Bindepartner für missgefaltete Proteine führt die Missbildung der Testbindesubstanz (Analyt, Indikator) zu inkonstanten, nicht reproduzierbaren Ergebnissen. Ein ähnliches Problem wurde in der Patentanmeldung DE60202008 für eine Methode zur Detektion und Messung von "advanced glycation endproducts" beschrieben. Auch darf keines in einem Verfahren zur Detektion, Messung, Imaging, Anreicherung von Proteinen/Peptiden, Depletion/Abreicherung von Proteinen/Peptiden, Überwachung von Therapien genutztes Begleitreagenz missgefaltete Proteine/Peptide enthalten. Komplette Proteine oder größere Proteinteile davon, wie sie in den oben genannten Patentanmeldungen genutzt werden, sind zudem teuer, da sie entweder selbst rekombinant hergestellt werden müssen, oder aus biologischem Material gereinigt werden müssen. Für Verfahren zur Reinigung von Medikamenten von missgefalteten Proteinen/Peptiden, zur Ligandenadsorption von missgefalteten Proteinen aus Körperflüssigkeiten und für den Einsatz im Körper z.B. zum Imaging, ist es wichtig, dass die Testsubstanz keine Abwehrreaktion/Antikörperbildung auslöst. Bei Verfahren zum Imaging innerhalb des Körpers eines Säugetieres oder Menschen, ist es von Vorteil, wenn das Testreagenz klein ist.

Die Bedeutung von extrazellulären missgefalteten Proteinen/Peptiden für die Biochemie und Pathobiochemie in Organismen, insbesondere in Säugetieren und dem Mensch, wird zunehmend erkannt. Leider befinden sich in vielen Medikamenten und Medikamentzusatzstoffen missgefaltete Proteine. Diese müssen detektiert und das Pharmakon und/oder der Zusatzstoff davon depletiert werden. Auch bei der Herstellung, Reinigung, Aufbewahrung, Virusdekontamination kann es durch eine Vielzahl an Gründen in Medikamenten und ihren Produktionsvorstufen zu einer Missfaltung von Proteinen/Peptiden kommen. Daher ist eine Methode wünschenswert, welche missgefaltete Proteine erkennt, damit alle Verfahren, die zur Herstellung eines Arzneimittels führen, auf das Vorhandensein von missgefalteten Proteinen/Peptiden geprüft und deren Konzentration gemessen werden kann. Insbesondere bei Blutprodukten oder rekombinant hergestellten Proteinen/Peptiden kann schon das Ausgangsmaterial für die Herstellung von Medikamenten missgefaltete Proteine enthalten. Die Bedeutung von missgefalteten Proteinen für die unterschiedlichsten physiologischen und pathophysiologischen Prozesse wird intensiv beforscht. Damit die Versuche nicht zu Fehlschlüssen verleiten, muss der Gehalt an missgefalteten Proteinen in allen Reagenzien bekannt sein. Optimalerweise sollten alle Hilfsreagenzien keine missgefalteten Proteine enthalten. Reagenzien wie Medienzusätze in der Zellkultur, Proteinzusätze zur Stabilisierung oder zur Inhibierung unerwünschter Interaktionen, wie, aber nicht beschränkt auf, Serum- oder Wachstumsfaktorzusätze für die Zellkultur, Albumine oder Magermilchpulver zum Blocken, sind davon in ganz besonderem Maße betroffen. Das Bindungsreagenz für missgefaltete Proteine muss neben der Detektion und Messung von missgefalteten Proteinen auch für Methoden zur Depletion/Abreicherung von missgefalteten Proteinen, aber auch zu deren Anreicherung geeignet sein, wenn solche Proteine/Peptide gezielt genutzt werden sollen.

### Offenlegung der Erfindung:

Die Gegenstände der vorliegenden Erfindung ergeben sich aus den Patentansprüchen.

Insbesondere ist Gegenstand der Erfindung die Verwendung einer Bindesubstanz ausgewählt aus der Gruppe, bestehend aus Peptiden, welche die Sequenz His, Trp, Arg, Arg (HWRR), die Sequenz His, Trp, Arg, Arg, Pro (HWRRP), die Sequenz Ala, Val, Thr, Leu, Glu, Asn, Phe, Leu, His, Trp, Arg, Arg entsprechend den Aminosäuren 278-289 der ADAM15 Sequenz, die Sequenz Glu, Asn, Phe, Leu, His, Trp, Arg, Arg, Ala, His, Leu, Leu (ENFLHWRRAHLL) entsprechend den Aminosäuren 282-293 der ADAM 15 Sequenz oder die Sequenz His, Trp, Arg, Arg, Ala, His, Leu, Leu, Pro, Arg, Leu, Pro (HWRRAHLLPRLP) entsprechend den Aminosäuren 286-297 der ADAM15 Sequenz umfassen, der Metalloproteasedomäne von ADAM15 und ADAM15 (Metargidin), *in vitro* zur Anbindung missgefalteter Proteine oder Peptide, die bei Erkrankungen eine Rolle in der Pathogenese spielen.

Ferner ist Gegenstand der Erfindung ein Verfahren zur Bestimmung von missgefalteten Proteinen oder Peptiden in einer Probe *in vitro,* umfassend
a) Inkontaktbringen der Probe mit einer oder mehreren Bindesubstanzen, ausgewählt aus der Gruppe, bestehend aus Peptiden, welche die Sequenz His, Trp, Arg, Arg (HWRR), die Sequenz His, Trp, Arg, Arg, Pro (HWRRP), die Sequenz Ala, Val, Thr, Leu, Glu, Asn, Phe, Leu, His, Trp, Arg, Arg entsprechend den Aminosäuren 278-289 der ADAM15 Sequenz, die Sequenz Glu, Asn, Phe, Leu, His, Trp, Arg, Arg, Ala, His, Leu, Leu (ENFLHWRRAHLL) entsprechend den Aminosäuren 282-293 der ADAM 15 Sequenz oder die Sequenz His, Trp, Arg, Arg, Ala, His, Leu, Leu, Pro, Arg, Leu, Pro (HWRRAHLLPRLP) entsprechend den Aminosäuren 286-297 der ADAM15 Sequenz umfassen, der Metalloproteasedomäne von ADAM15 und ADAM15 (Metargidin),
b) qualitative oder/und quantitative Bestimmung der gebundenen missgefalteten Proteine oder Peptide.

Ein wiederum weiterer Gegenstand der Erfindung ist ein Verfahren zur Entfernung von missgefalteten Proteinen oder Peptiden aus einer Probe oder einem Produkt in vitro, umfassend
a) Inkontaktbringen der Probe oder des Produkts mit einer oder mehreren Bindesubstanzen, ausgewählt aus der Gruppe, bestehend aus Peptiden, welche die Sequenz His, Trp, Arg, Arg (HWRR), die Sequenz His, Trp, Arg, Arg, Pro (HWRRP), die Sequenz Ala, Val, Thr, Leu, Glu, Asn, Phe, Leu, His, Trp, Arg, Arg entsprechend den Aminosäuren 278-289 der ADAM15 Sequenz, die Sequenz Glu, Asn, Phe, Leu, His, Trp, Arg, Arg, Ala, His, Leu, Leu (ENFLHWRRAHLL) entsprechend den Aminosäuren 282-293 der ADAM 15 Sequenz oder die Sequenz His, Trp, Arg, Arg, Ala, His, Leu, Leu, Pro, Arg, Leu, Pro (HWRRAHLLPRLP) entsprechend den Aminosäuren 286-297 der ADAM15 Sequenz umfassen, der Metalloproteasedomäne von ADAM15 und ADAM15 (Metargidin),
b) Abtrennen der gebundenen missgefalteten Proteine oder Peptide.

Ferner ist ein Gegenstand der Erfindung ein Verfahren zur Bereitstellung von missgefalteten Proteinen oder Peptiden aus einer Probe in vitro, umfassend
a) Inkontaktbringen der Probe mit einer oder mehreren Bindesubstanzen, ausgewählt aus der Gruppe, bestehend aus Peptiden, welche die Sequenz His, Trp, Arg, Arg (HWRR), die Sequenz His, Trp, Arg, Arg, Pro (HWRRP), die Sequenz Ala, Val, Thr, Leu, Glu, Asn, Phe, Leu, His, Trp, Arg, Arg entsprechend den Aminosäuren 278-289 der ADAM15 Sequenz, die Sequenz Glu, Asn, Phe, Leu, His, Trp, Arg, Arg, Ala, His, Leu, Leu (ENFLHWRRAHLL) entsprechend den Aminosäuren 282-293 der ADAM 15 Sequenz oder die Sequenz His, Trp, Arg, Arg, Ala, His, Leu, Leu, Pro, Arg, Leu, Pro (HWRRAHLLPRLP) entsprechend den Aminosäuren 286-297 der ADAM15 Sequenz umfassen, der Metalloproteasedomäne von ADAM15 und ADAM15 (Metargidin),
b) Abtrennen der gebundenen missgefalteten Proteine oder Peptide, und
c) Isolieren der missgefalteten Proteine oder Peptide,
   wobei vorzugsweise die Probe ausgewählt ist aus Körperflüssigkeiten oder Gewebeauszügen wie beispielsweise Blut, Serum Blutplasma, Lymphe, Sperma, Vaginalflüssigkeit, Fruchtwasser, Cerebrospinalflüssigkeit, Synovialflüssigkeit, Urin, Sputum, Flüssigkeit aus Lavagen, wie z.B. bronchioalveoläre Lavageflüssigkeit und Peritoneallavageflüssigkeit, oder
   wobei die Probe ausgewählt ist aus Medizinprodukten wie Arzneimitteln, Arzneizusatzstoffen, pharmazeutischen Zusammensetzungen, Reagenzien und Hilfsreagenzien für Diagnostische Tests, Forschungsreagenzien, Lebensmitteln, Genussmitteln, Nahrungsergänzungsmitteln, Trink- und Brauchwasser und Biofilmen.

Schließlich ist Gegenstand der Erfindung ein in vitro Verfahren zur Diagnose, Stratifizierung und Überwachung von Erkrankungen gemäß der Ansprüche. Die Erfindung umfasst des Weiteren einen diagnostischen Kit für die Bestimmung von missgefalteten Proteinen oder Peptiden, umfassend
a) eine erste Bindesubstanz, ausgewählt aus der Gruppe, bestehend aus Peptiden, welche die Sequenz His, Trp, Arg, Arg (HWRR), die Sequenz His, Trp, Arg, Arg, Pro (HWRRP), die Sequenz Ala, Val, Thr, Leu, Glu, Asn, Phe, Leu, His, Trp, Arg, Arg entsprechend den Aminosäuren 278-289 der ADAM15 Se-quenz, die Sequenz Glu, Asn, Phe, Leu, His, Trp, Arg, Arg, Ala, His, Leu, Leu (ENFLHWRRAHLL) entsprechend den Aminosäuren 282-293 der ADAM 15 Sequenz oder die Sequenz His, Trp, Arg, Arg, Ala, His, Leu, Leu, Pro, Arg, Leu, Pro (HWRRAHLLPRLP) entsprechend den Aminosäuren 286-297 der ADAM15 Sequenz umfassen, der Metalloproteasedomäne von ADAM15 und ADAM15 (Metargidin), und
b) ein spezifisches Detektionsreagenz für das missgefaltete Protein oder Peptid, wobei das Detektionsreagenz vorzugsweise eine weitere Bindesubstanz, ausgewählt aus der Gruppe, bestehend aus Peptiden, welche die Sequenz His, Trp, Arg, Arg (HWRR), die Sequenz His, Trp, Arg, Arg, Pro (HWRRP), die Sequenz Ala, Val, Thr, Leu, Glu, Asn, Phe, Leu, His, Trp, Arg, Arg entsprechend den Aminosäuren 278-289 der ADAM15 Sequenz, die Sequenz Glu, Asn, Phe, Leu, His, Trp, Arg, Arg, Ala, His, Leu, Leu (ENFLHWRRAHLL) entsprechend den Aminosäuren 282-293 der ADAM 15 Sequenz oder die Sequenz His, Trp, Arg, Arg, Ala, His, Leu, Leu, Pro, Arg, Leu, Pro (HWRRAHLLPRLP) entsprechend den Aminosäuren 286-297 der ADAM15 Sequenz umfassen, der Metalloproteasedomäne von ADAM15 und ADAM15 (Metargidin), oder ausgewählt aus Chaperonen, Scavenger Rezeptoren, t-Pa, FXII, HGFA, Kongorot oder Thioflavin, oder einen spezifischen Antikörper gegen das Protein oder Peptid umfasst,
   wobei eine der Komponenten a) und b) eine nachweisbare Markierung trägt und die andere der Komponenten a) und b) an einer Festphase immobilisiert ist.

Ferner umfasst die Erfindung ein Verfahren zur Bestimmung der Biokompatibilität eines Materials *in vitro,* umfassend die Schritte:
a) Inkontaktbringen des Materials mit einer Testsubstanz, die Proteine oder Pep-tide umfasst,
b) qualitative oder/und quantitative Bestimmung von missgefalteten Proteinen oder Peptiden in der Testsubstanz, nach einem Verfahren wie in einem der Ansprüche 4 und 5 definiert,
c) Vergleich der Menge der in der Testsubstanz enthaltenen missgefalteten Pro-teine oder Peptide vor und nach dem Inkontaktbringen mit dem Material und
d) Bestimmung, ob das Material in Proteinen oder Peptiden der Testsubstanz Missfaltungen erzeugt.

Und schließlich umfasst die Erfindung ein Verfahren zur Aufkonzentrierung von Mikroorganismen, welche missgefaltete Proteine auf ihrer Oberfläche tragen, aus einer Probe *in vitro,* umfassend die Schritte:
a) Inkontaktbringen der Probe mit einer oder mehreren Bindesubstanzen, ausgewählt aus der Gruppe, bestehend aus Peptiden, welche die Sequenz His, Trp, Arg, Arg (HWRR), die Sequenz His, Trp, Arg, Arg, Pro (HWRRP), die Sequenz Ala, Val, Thr, Leu, Glu, Asn, Phe, Leu, His, Trp, Arg, Arg entsprechend den Aminosäuren 278-289 der ADAM15 Sequenz, die Sequenz Glu, Asn, Phe, Leu, His, Trp, Arg, Arg, Ala, His, Leu, Leu (ENFLHWRRAHLL) entsprechend den Aminosäuren 282-293 der ADAM 15 Sequenz oder die Sequenz His, Trp, Arg, Arg, Ala, His, Leu, Leu, Pro, Arg, Leu, Pro (HWRRAHLLPRLP) entsprechend den Aminosäuren 286-297 der ADAM15 Sequenz umfassen, der Metalloproteasedomäne von ADAM15 und ADAM15 (Metargidin),
b) Abtrennen der gebundenen Mikroorganismen aus der Probe.

In den Patentanmeldungen WO 2005/039616 und WO 2008/047370 werden die oben genannten ADAM15 Peptide aufgrund ihrer Fähigkeit, direkt an GRP78/BIP zu binden, als Medikamente zur Steigerung der Angiogenese beschrieben. In WO 2010/052715 werden ADAM15, seine Metalloproteasedomäne und die oben genannten ADAM15 Sequenzen als Mittel zur lokalen Behandlung von kardiovaskulären Erkrankungen genannt. Die Patentanmeldung WO 2010/052715 beschreibt die Nutzung der Peptide in implantierbaren Vorrichtungen, wie z.B. Stents, zum Anlocken von endothelialen Progenitorzellen, zur lokalen Steigerung der Angiogenese und zur Inhibierung der Endothelzellapoptose an krankhaft veränderten Orten des Herz-Kreislaufsystems. Die Patentanmeldung lehrt, dass ADAM15 und seine oben genannten Peptide direkt an GRP78/BIP auf Endothelzellen binden und es so schützen. Diese Peptide können zum Rekrutieren von endothelialen Vorläuferzellen z.B. aus der Nabelschnur und aus dem periphären Blut genutzt werden. Sie wirken chemotaktisch für endotheliale Vorläuferzellen und steigern die Endothelzellvermehrung. Da die Erfinder der drei letztgenannten Patentanmeldungen das Protein GRP78/Bip als direkten Bindungspartner und als verantwortliches Protein für die beobachtete Steigerung der Angiogenese angesehen haben, wurde nicht nach weiteren oder alternativen Bindungspartnern gesucht und die Verwendung der Peptide zur Diagnostik nicht in Erwägung gezogen.

Überraschenderweise fanden wir, dass ADAM15 (Metargidin), die Metalloproteasedomäne von ADAM15 und insbesondere Peptide, welche die Sequenz His, Trp, Arg, Arg, Ala, His, Leu, Leu, Pro, Arg, Leu, Pro (HWRRAHLLPRLP) entsprechend der ADAM15 Sequenz 286-297, die Sequenz Glu, Asn, Phe, Leu, His, Trp, Arg, Arg, Ala, His, Leu, Leu (ENFLHWRRAHLL) ADAM 15 Sequenz 282-293, oder die Sequenz Ala, Val, Thr, Leu, Glu, Asn, Phe, Leu, His, Trp, Arg, Arg ADAM15 Sequenz 279-290, oder die Sequenz His,Trp,Arg, Arg, Pro oder His,Trp,Arg, Arg (HWRR) enthalten, generelle Bindungspartner mit hoher Affinität für missgefaltete Proteine sind.

Beschrieben werden Bindungspartner mit einer hohen natürlichen, generellen Affinität für missgefaltete Proteine/Peptide mittels derer missgefaltete Proteine/Peptide detektiert und gemessen werden können. Gegenstand der vorliegenden Erfindung sind auch Bindungspartner für missgefaltete Proteine/Peptide für die Überwachung und das Monitoring von therapeutischen Maßnahmen. Auch sind die Substanzen geeignet für das Depletieren/Abreichern von missgefalteten Proteinen/Peptiden aus Körperflüssigkeiten, Medikamenten oder Zusatzstoffen in Medikamenten, aus Reagenzien für diagnostische Zwecke, aus Forschungsreagenzien sowie aus Zusatzstoffen in Forschungsreagenzien sowie aus Lebensmitteln, Genussmittel, Nahrungsergänzungsmitteln und Trink- oder Brauchwasser, sofern diese missgefaltete Proteine/Peptide beinhalten. Daneben sind beschrieben Substanzen und Methoden zur Überwachung, Optimierung und Qualitätskontrolle von Biopharmazeutika, zur Untersuchung der Biokompatibilität von Materialien und zur Untersuchung der Einflussnahme von Unterstützungs- oder Messsystemen für die Anwendung im oder am Körper von Säugetieren oder Menschen, insbesondere von implantierbare Unterstützungssystemen, auf Zellen, Gewebe und Körperflüssigkeiten, mit derer Hilfe solche Systeme optimiert werden können. Eine bevorzugte Ausführungsform der vorliegenden Erfindung ist auch die Nutzung eines oder mehrerer der Bindungspartner gemäß Erfindung und Verfahren zum Finden, zur Anreicherung und zur Immobilisation von missgefalteten Proteinen/Peptiden für technische Anwendungen.

Spezielle Ausführungsformen der Erfindung sollen jetzt ausführlicher dargestellt werden.

### 1. Verwendung der Bindesubstanzen zur Detektion und Quantifizierung von missgefalteten Proteinen/Peptiden (Diagnostik von Erkrankungen, Methoden und Kits)

Gemäß einer besonders bevorzugten Ausführungsform der vorliegenden Erfindung binden ADAM15 (Metargidin), die Metalloproteasedomäne von ADAM15 und insbesondere Peptide, welche die Sequenz His, Trp, Arg, Arg, Ala, His, Leu, Leu, Pro, Arg, Leu, Pro (HWRRAHLLPRLP) entsprechend der ADAM15 Sequenz 286-297, die Sequenz Glu, Asn, Phe, Leu, His, Trp, Arg, Arg, Ala, His, Leu, Leu (ENFLHWRRAHLL) ADAM 15 Sequenz 282-293, oder die Sequenz Ala, Val, Thr, Leu, Glu, Asn, Phe, Leu, His, Trp, Arg, Arg ADAM15 Sequenz 279-290, His, Trp, Arg, Arg, Pro oder His, Trp, Arg, Arg (HWRR) haben, missgefaltete Proteine generell mit einer hohen natürlichen Affinität. Sie sind daher als Bindesubstanzen in Methoden und Kits zur Detektion und Messung von missgefalteten Proteinen in Körperflüssigkeiten oder Gewebeauszügen von Säugetieren und Menschen, zur Detektion und Quantifizierung im Gewebe von Säugetieren und Menschen und zum Bioimaging von an den Wänden von Systemen, welche mit Blut, Lymphe oder Liquor durchströmt werden, befindlichen missgefalteten Proteinen, z.B. zum Bioimaging von arteriosklerotischen Plaques und von Ablagerungen im Gehirn bei neurodegenerativen Erkrankungen, nutzbar.

In einer ersten Ausführungsform sind die Bindesubstanzen gemäß Erfindung Teil eines neuen diagnostischen Testsystems gemäß der Ansprüche. In einer besonders bevorzugten Ausführungsform der Erfindung betrifft die Erfindung die Verwendung der oben offengelegten Proteine, Proteinfragmente und Peptide gemäß der Ansprüche als Diagnostika, wobei mindestens eines der offengelegten Proteine, Proteinfragmente oder Peptide ausgewählt ist. Die Kenntnis solcher Systeme gehört für den Fachmann zum Allgemeinwissen.

Die Bestimmung der missgefalteten Proteine/Peptide außerhalb des menschlichen oder tierischen Körpers und die Bestimmung erfolgt in einer ex vivo / in vitro Diagnose. In einer weiteren Ausführungsform der Erfindung betrifft die Erfindung die Verwendung gemäß der Ansprüche deroben offengelegten Proteine, Proteinfragmente und Peptide als Diagnostika, wobei mindestens eines der offengelegten Proteine, Proteinfragmente oder Peptide ausgewählt wird.

Eine ganz bevorzugte Ausführungsform der Erfindung ist die direkte oder indirekte Bindung oder Fixierung der Bindesubstanzen an eine solide Oberfläche. Nach Waschschritten und Blockade möglicher unspezifischen Bindungen wird die zu untersuchende Lösung (bevorzugt Körperflüssigkeit) wie, aber nicht beschränkt auf, Blut, Serum Blutplasma, Lymphe, Sperma, Vaginalflüssigkeit, Fruchtwasser, Cerebrospinalflüssigkeit, Synovialflüssigkeit, Urin, Sputum, Flüssigkeit aus Lavagen, wie z.B. bronchioalveoläre Lavageflüssigkeit, Peritoneallavageflüssigkeit, oder einem Gewebsauszug mit der soliden Oberfläche, welche eine oder mehrere der Bindesubstanzen trägt, in Verbindung gebracht, sodass eventuell vorhandene missgefaltete Proteine/Peptide immobilisiert werden. Diese können dann, nach weiteren Waschschritten, z.B. als allgemeine missgefaltete Proteine/Peptide detektiert oder gemessen werden. Dies kann gemäß einer bevorzugten Ausführungsform der Erfindung z. B. über ein Plasmonresonanzverfahren erfolgen oder durch Nutzung einer anderen generellen Bindesubstanz, wobei diese eine Substanz oder eine schon bekannte Bindesubstanz, wie z.B. ein Chaperon, ein Scavenger Rezeptor, t-Pa, FXII, HGFA oder Kongorot oder Thioflavin, sein kann, in einem Sandwich-Assay. Es können aber auch gezielt spezifische missgefaltete Proteine detektiert und gemessen werden, wie z.B. eines der vielen Proteine, für welche eine Neigung zur Missfaltung gefunden wurde oder für die die Neigung zu einer Missfaltung vermutet wird. Die Detektion erfolgt dann mit einem spezifischen Detektionsreagenz für das gesuchte individuelle Protein/Peptid. Besonders geeignet sind als Detektionsreagentien spezifische markierte Antikörper gegen die individuellen Proteine/Peptide. Die Auswahl der spezifischen Detektionsreagenzien richtet sich nach der Erkrankung, welche prognostiziert oder detektiert werden soll. In einer weiteren Ausführungsform, lassen sich auch missgefaltete Proteine/Peptide messen, welche bestimmte mit einer Indikatorsubstanzen reagible chemische Gruppen tragen, wie aber nicht begrenzt auf Proteinmodifikationen durch Einwirkung von Acrolein und anderen elektrophilen Substanzen, Glykierungen, proteolytische Spaltung, Phosphorylierung, Dephosphorylierung, Glykosylierung, Acetylierung, S-Nitrosylierung, Citrullinierung oder Sulfatierung. Reagenzien zur Detektion solcher reaktiver Gruppen sind bekannt und im Handel erhältlich.

In einer Ausführungsform werden die Bindungssubstanzen an eine feste Oberfläche gebunden oder fixiert, welche zu einem Assay oder einer diagnostischen Einrichtung gehört.

Diese kann in einer besonders bevorzugten Ausführungsform eine Mikrotiterplatte, ein Chip für eine Oberflächen Plasmon Resonanz Untersuchung, ein Mikroarray-Chip, ein Filter wie Nitrocellulose, Nylon oder PVDF, eine Membran, ein magnetisches oder Fluorophor-markiertes Kügelchen, ein Silizium-Wafer, Glas, Metall, Kunststoff, ein Chip, ein massenspektrometrisches Target oder eine Matrix, sowie Kügelchen (Beads) für Untersuchungen in Durchflusszytometern sein.

Im Sinne dieser Erfindung sind zum Beispiel geeignete Ausführungsformen Assays und Vorrichtungen, wie ELISA, Bead-based Assay, Western Blot, affinitätschromatographische Verfahren (z.B. so genannte Lateral Flow Affinitätsligandenassays) oder ähnliche Single- oder Multiplex-Nachweisverfahren und Plasmon Resonanz. Ein Mikroarraychip im Sinne dieser Erfindung ist die systematische Anordnung von Bindesubstanzen für missgefaltete Proteine und andere nachzuweisende Substanzen, welche über eine Erkrankung, das Risiko einer Erkrankung oder die Wahrscheinlichkeit eine bestimmte Erkrankung zu bekommen Aufschluss geben oder welche für das Monitoring und die Überwachung von therapeutischen Maßnahmen gedacht sind, auf einem festen Träger. Die hier beschriebenen Bindesubstanzen werden auf einen modifizierten Objektträger an eine genau definierte Stelle gedruckt. Der Nachweis möglicher missgefalteter Proteine/Peptide auf dem Biochip erfolgt in zwei Stufen. In einem ersten Inkubationsschritt wird der mit einer Bindesubstanz gemäß Erfindung bedeckte Biochip mit einer Körperflüssigkeit des Menschen, bevorzugt Blut, Plasma oder Cerebrospinalflüssigkeit, inkubiert, sodass vorhandene missgefaltete Proteine/Peptide auf dem Biochip binden. In einem zweiten Inkubationsschritt werden gebundene missgefaltete Proteine/Peptide mittels eines zweiten Bindereagenzes für missgefaltete Proteine/Peptide erkannt, welches mit einem Nachweissystem verbunden ist, oder mittels eines spezifischen Antikörpers gegen ein natives Epitop des missgefalteten Proteins detektiert, welcher mit einem Nachweissystem verbunden ist, oder mit spezifischen Detektionsreagenzien gegen Proteinmodifikationen, welche mit einem Nachweissystem verbunden sind, detektiert und quantifiziert. Die Ratio aus einem in der Körperflüssigkeit vorhandenem spezifischen Protein/Peptid und den mit einem Verfahren gemäß Erfindung bestimmten missgefalteten Protein/Peptid, gibt den prozentualen Anteil an Missfaltung für ein spezifisches Protein/Peptid.

Die Visualisierung der erfindungsgemäßen Wechselwirkung zwischen den hier offengelegten Bindesubstanzen und missgefalteten Proteinen/Peptiden kann mit den gängigen Mitteln zum Nachweis des "Bindungserfolges" beispielsweise durch Fluoresenzmarkierung, Biotinylierung, Radio-Isotopen-Markierung oder durch kolloidale Gold- oder Latex-Partikel- Markierung sowie durch Enzymreaktionen mit signalgebenden Substraten oder Chemilumineszenz in üblicher Weise erfolgen.

In einer Ausführungsform der Erfindung werden die Bindungssubstanzen mit einem Erkennungslabel markiert. Markierungen oder Teile von Markierungen, beispielsweise eine Komponente eines spezifischen Bindungspaares, können kovalent an das nachzuweisende Molekül gebunden werden. Dazu sind dem Fachmann verschiedene Verfahren bekannt und vielfach auch geeignete Reagenzien im Handel erhältlich. Geeignete Erkennungslabel können z.B., aber nicht beschränkt auf, Fluoreszenzmarker sein, wie z.B. Dil, FITC, PE, PerCp Cy-, Alexa-, Dyomics, oder ähnliche Fluoreszenzfarbstoffe, Biotin, ein HIS-Tag, ein GST-Tag, ein SEAP-Tag, ein Maltose binding protein-Tag (MBP-Tag), ein FLAG-Tag. Digoxigenin, ein paramagnetisches Atom, ein radioaktives Atom, wie z.B. Kohlenstoff-11, Jod-125/123, ^{99m}Tc, Cu-64 oder 111In, sowie Reporter-Enzyme, wie alkalischer Phosphatase, Meerrettichperoxidase, β-Galactosidase, Glukoseoxidase, Luciferase, β-Lactamase, Urease oder Lysozym usw. zusammen mit den entsprechenden colorimetrischen, fluoreszenten oder chemolumineszenten Substraten. Die daran gebundenen Nachweisreaktionen, wie die Umsetzung von o-Phenylendiamin, 4-Chlornaphthol oder Tetramethylbenzidin durch Meerrettich-Perioxidasen, sind dem Fachmann ebenfalls hinlänglich bekannt.

Die Detektion und Quantifizierung oder Semiquantifizierung in vitro in und auf Geweben und Zellen des Körpers eines Säugetieres, bevorzugt des Menschen. Hierfür werden markierte Bindungspartner gemäß dieser Erfindung mittels der gängigen, dem Fachmann gut bekannten Methoden der Histochemie oder Durchflusszytometrie genutzt, um missgefaltete Proteine/Peptide nachzuweisen und zu quantifizieren.

In einer weiteren Ausführungsform erfolgt der Nachweis von missgefalteten Proteinen /Peptiden mittels DIP-Stick (Teststreifen)-Methode durch Immobilisation von einer der Bindesubstanzen bevorzugt auf einem Filter oder einer Membran. Der Teststreifen wird mit der zu testenden Probe in Verbindung gebracht, so dass durch Kapillarkräfte die Flüssigkeit der Probe bis über die Stelle gezogen wird, an welcher einer der Bindesubstanzen fest immobilisiert worden ist. Der Nachweis erfogt wie oben für andere Verfahren beschrieben durch ein weiteres Detektionsreagenz, welches mit einem Visualisierungssystem verbunden ist.

Verfahren gemäß der Erfindung können verwendet werden zur in vitro Diagnose, Stratifizierung und /oder Überwachung einer Erkrankung, bei der missgefaltete Proteine eine pathogenetische Rolle spielen. Dies sind insbesondere Erkrankungen, welche mit der Bildung von Amyloiden verbunden sind, wie die Gruppe der klassischen Amyloidosen und neurodegenerativen Erkrankungen, aber auch Diabetes, metabolisches Syndrom, Adipositas, Arteriosklerose und seine Folgeerkrankungen, Blutungen, Thrombosen, DIC, Nierenversagen, Hämodialyse, Kataract, multiples Myelom, Lymphom, Sepsis, schweres Gewebstrauma, Zustand nach Einsatz von Herzlungenmaschine, Organtransplantation, Zustand nach Implantation von Herzunterstützungssystemen, Pankreatitis, Zustand nach Reanimation, Ischämie-Reperfusionsschaden, Präeklampsie.

Der Begriff der in vitro Diagnose umfasst die medizinische in vitro Diagnostik und diesbezügliche Untersuchungen, insbesondere die Labordiagnostik. Risikostratifizierung oder Therapiesteuerung im Sinne dieser Erfindung bedeutet, dass das erfindungsgemäße Verfahren Entscheidungen zur Behandlung und Therapie des Patienten erlaubt, sei es Hospitalisierung des Patienten, Einsatz, Wirkung und / oder Dosierung eines oder mehrerer Arzneimittel, eine therapeutische Maßnahme oder die Überwachung eines Krankheitsverlaufes sowie Therapieverlauf bzw. Ätiologie oder Klassifizierung einer Erkrankung, z.B. in einen neuen oder bestehenden Subtyp oder die Differenzierung von Krankheiten und dessen Patienten. In einer weiteren Ausführungsform der Erfindung umfasst der Begriff "Stratifizierung" insbesondere die Risikostratifizierung mit der Prognose eines "outcome" eines nachteiligen gesundheitlichen Ereignisses. Im Rahmen dieser Erfindung wird unter "Patient" ein beliebiger Proband - Mensch oder Säugetier - verstanden, mit der Maßgabe, dass der Proband auf eine Krankheit oder eine Krankheitsvorstufe untersucht wird, bei der missgefaltete Proteine/Peptide eine Rolle in der Pathogenese spielen.

In einer bevorzugten Ausführung der Erfindung umfasst das Verfahren auch folgende Schritte:
1) Vergleich des in einer diagnostischen Methode gemäß Erfindung ermittelten Wertes mit einem Referenzwert und/oder einem in Referenzproben ermittelten Mittelwerten. Eine Referenzprobe kann von Gesunden oder von Patienten stammen, welche die infrage befindliche Erkrankung sicher haben oder nicht haben. Anstatt des Einsatzes von Referenzproben können auch festgelegte Referenzwerte verwendet werden
2) Quantifizierung der missgefalteten Proteine
3) Vergleich der Konzentration an missgefaltetem Protein oder einem spezifischen missgefalteten Protein, oder von missgefalteten Proteinen, welche bestimmte chemische Reaktionsgruppen tragen mit den Konzentrationen, welche zu einem anderen Zeitpunkt beim gleichen Probanden gemessen wurden
4) In einigen Fällen ist es hilfreich durch Bildung des Quotienten aus der Konzentration eines spezifischen untersuchten Proteins und der Konzentration desgleichen Proteins, welches missgefaltet ist, den Prozentsatz an Missfaltung in einem bestimmten Protein zu berechnen.

Die hier beschriebene Erfindung erlaubt u.a. die gezielte Suche nach geeigneten Kandidaten unter den missgefalteten Proteinen/Peptiden für die Diagnostik von Erkrankungen unter Verwendung der hier offengelegten Bindesubstanzen.

Hierfür wird mindestens eine der hier offengelegten Bindungsubstanzen für missgefaltete Proteine an eine feste Oberfläche, wie oben beschrieben, immobilisiert. Das beschichtete Material wird dann mit einer Körperflüssigkeit (siehe oben) oder einem Gewebeauszug von Patienten mit der Krankheit, für die eine Methode zum Nachweis eines spezifischen missgefalteten Proteins/Peptids gesucht werden soll, und in Vergleichsproben von gesunden Probanden inkubiert und spezifisch gebundene Proteine/Peptide werden mit den gängigen Methoden der Proteomik analysiert und identifiziert. Hierzu gehören z.B. 2D-Gelelektrophorese, Western Blotting, Immunpräzipitation, partielle Proteolyse und Massenspektrometrie der Spaltprodukte. Geeignete missgefaltete Kandidatenproteine/peptide für den Aufbau spezifischer Testmethoden für bestimmte Erkrankungen oder Erkrankungsausformungen/-schweregrade sind solche, deren Konzentration in der Körperflüssigkeit der Patienten signifikant höher ist, als in der Körperflüssigkeit der Kontrollgruppen.

Das Verfahren zur Detektion und Messung von missgefalteten Proteinen kann gemäß Erfindung auch verwendet werden um in vitro in Körperflüssigkeiten oder Geweben von scheinbar gesunden Probanden nach Indikatoren für eine frühe noch symptomfreie oder symptomarme Verlaufsstufe einer Erkrankung zu suchen. So ist zum Beispiel eine bevorzugte Ausführung der Erfindung die Detektion und Quantifizierung von Amyloid beta Oligomeren, bevor der Gedächtnisverlust deutlich zutage tritt oder die Detektion und Quantifizierung von HOCI- modifizierten Proteinen, missgefaltetem Fibrinogen, missgefaltetem Albumin, missgefaltetem Ceruloplasmin oder von elektronegativem LDL (LDL (-)), bevor Folgen der Arteriosklerose durch Angina Pectoris, TIA, Schlaganfall, Herzinfarkt oder periphärem Gefäßverschluss offenkundig werden, von missgefalteten Serpinen, insbesondere alpha1 Antitrypsin, bevor z.B. die klinischen Zeichen eine Präeklampsie diagnostiziert werden, oder von missgefalteten Proteinen aus der Gruppe der Proteine, welche eine klassische Amyloidose verursachen, bevor es zu Amyloidablagerungen kommt.

Die Aufgabe der vorliegenden Erfindung wird weiterhin gelöst durch ein diagnostisches Kit nach Anspruch 13.

An einen solchen Kit müssen erfindungsgemäß besondere Anforderungen gestellt werden. Alle in den Verfahren genutzten Reagenzien müssen selber frei von missgefalteten Proteinen/Peptiden sein.

Ein Reagenz ist ein in einem Assay zur Detektion von missgefalteten Proteinen verwendetes Biomolekül, das Prozessen unterliegen kann, welche zur Missfaltung von Proteinen/Peptiden führen, z.B. einer Pufferkomponente, einem Stabilisierungsprotein, einer Komponente zur Beschichtung oder zum Blockieren von nicht-spezifischen Bindern der Festphase wie aber nicht beschränkt auf Rinderserumalbumin, Humanalbumin, Magermilchpulver, während der Herstellung der Festphase verwendete Reagenzien, ein Nachweisreagenz, insbesondere, wenn es sich dabei um einen Antikörper oder ein andere Protein, insbesondere wenn es sich um ein rekombinantes Protein handelt. Von missgefalteten Proteinen/Peptiden freie oder zumindest im Wesentlichen von missgefalteten Proteinen/Peptiden freie Reagenzien werden ausgewählt und verwendet.

Der Begriff Festphasenmaterialien /-komponente wird verwendet, um Festphasen oder Trägermaterialien zu beschreiben, welche routinemäßig in Bindeassays verwendet werden. In der vorliegenden Erfindung wird ein Verfahren zur Herstellung einer Festphasenkomponente, und Hilfsreagentien, wie Blockierungsreagenz und Nachweisreagenz beschrieben, welche frei von missgefalteten Proteinen/Peptiden ist. Beschichten der Festphasenkomponente mit einem spezifischen Bindungspartner, der frei von missgefalteten Proteinen/Peptiden ist. Waschen der Festkörperkomponente und Zugeben einer Blockier- oder Stabilisierlösung, welche frei von missgefalteten Proteinen/Peptiden ist. Nutzung eines Nachweisreagenzens/Bindepaarmitgliedes, welches frei von missgefalteten Proteinen/Peptiden ist. Ein besonderes Augenmerk gilt hier der Verwendung von Avidin, Streptavidin, Rinderserumalbulin, Humanalbulin, Magermilchpulver, jeder Art von Antikörpern und Nachweisenzymen.

In einer besonderen Ausführungsform der Erfindung wird mindestens eine der hier offengelegten Bindungssubstanzen für missgefaltete Proteine/Peptide an einen Sensorchip zu Messung der Plasmon Resonanz in einer Miniaturflusszelle immobilisiert. Korperflüssigkeiten oder verdünnte Körperflüssigkeiten oder Gewebsauszüge, welche potentiell missgefaltete Proteine/Peptide und damit Interaktionspartner für die Bindesubstanzen enthalten, werden über die Oberfläche dieses Sensorchips gespült und Wechselwirkungen mit den immobilisierten Molekülen werden über eine Änderung des Brechungsindexes detektierbar. Zeitabhängige Änderungen des Brechungsindexes werden registriert und dienen zur Ermittlung kinetischer Parameter.

Missgefaltete extrazelluläre Proteine/peptide gehen im Körper Komplexe mit so genannten extrazellulären Chaperonen ein. Hierzu gehören unter anderen, alpha2 Makroglobulin, Haptoglobin, Clusterin ¹⁰².

Gemäß der Erfindung können solche Komplexe durch einen Sandwich-Assay detektiert und gemessen werden, bei dem einer der beiden Bindungspartner eine der in dieser Patentanmeldung offengelegten Bindesubstanz für missgefaltete Proteine/Peptide ist, oder eine bisher bekannte Bindesubstanz, wie z.B. t-PA, FXII, HGFa, Fibronektin und der andere Bindungspartner eine spezifische Bindesubstanz für das so genannte extrazelluläre Chaperon, z.B. ein monoklonaler Antikörper gegen alpha2 Makroglobulin, ein monoklonaler Antikörper gegen Clusterin oder ein monoklonaler Antikörper gegen Haptoglobin.

Die vorliegende Erfindung erlaubt die Detektion und Messung von missgefalteten Makroglobulin-Komplexen mit anderen Proteinen mit den offengelegten Werkzeugen/Bindesubstanzen und Methoden. Diese ist besonders zur Diagnostik von Tumorerkrankungen geeignet.

### 2. Therapiemonitoring und -überwachung

Eine besonders bevorzugte Ausführungsform der Erfindung ist die Nutzung von ADAM15 (Metargidin), der Metalloproteasedomäne von ADAM15 und insbesondere von Peptiden, welche die Sequenz His, Trp, Arg, Arg, Ala, His, Leu, Leu, Pro, Arg, Leu, Pro (HWRRAHLLPRLP), die Sequenz Glu, Asn, Phe, Leu, His, Trp, Arg, Arg, Ala, His, Leu, Leu (ENFLHWRRAHLL) oder die Sequenz Ala, Val, Thr, Leu, Glu, Asn, Phe, Leu, His, Trp, Arg, Arg oder , His,Trp,Arg, Arg, Pro oder His, Trp, Arg, Arg (HWRR) haben oder beinhalten , zur Detektion und Messung von missgefalteten Proteinen gemäß der Ansprüche zum Monitoring, Überwachen von medikamentösen Therapien, sowie die Einteilung von Patienten in "Responder und Nicht-Responder" bezüglich einer Therapie oder dessen Therapieverlauf.

Missgefaltete Proteine können Thrombozyten über die Rezeptoren CD36 und GPIb aktivieren ¹⁰³. Die Thrombozytenaktivierung durch missgefaltete Proteine ist unabhängig von der Bildung von Thromboxan A2 und der Freisetzung von ADP. Beide Sekundäragonisten verstärken aber die Thrombozytenaktivierung durch missgefaltete Proteine als Feedback-Reaktion. Brodde und Kehrel konnten zeigen, dass Aspirin/ASS über Hemmung der ATPase-Aktivität des GRP78 die Aktivierung vom Thrombozyten durch missgefaltete Proteine/Peptide hemmt. So können missgefaltete Proteine/Peptide spezifisch eingesetzt werden, um das Ansprechen von Patienten auf eine Therapie mit ASS/Aspirin zu monitoren. Aspirin (Synonyme: Acetylsalicylsäure (ASS)) wird zur Prophylaxe von thrombotischen, thromboembolischen und insbesondere kardio-vaskulären Ereignissen, inklusive Schlaganfall und periphärem Gefäßverschluss eingesetzt. Nicht alle Patienten sprechen in gleicher Weise auf die Medikation an. Das Phänomen der " Aspirin-Non-Responder" wurde immer wieder beschrieben und eingehend untersucht. Seine Ursache ist bis heute nicht sicher geklärt ¹⁰⁴. Ein schlechtes Ansprechen auf Aspirin nach 24 Stunden ist statistisch mit Entzündungsmarkern, Rauchen und Diabetes verbunden ¹⁰⁵. Dies sind alles Konditionen, welche im Körper zu Missfaltung von Proteinen/Peptiden führen.

Interessanterweise bietet die langfristige Therapie mit Aspirin auch einen gewissen Schutz vor soliden Tumoren ¹⁰⁶. Einer der wichtigsten Zellrezeptoren für extrazelluläre missgefaltete Proteine/Peptide ist das GRP78/Bip. Es reguliert so wichtige Prozesse wie die Angiogenese, Autophagie und Zellproliferation.

Missgefaltete Proteine können zum Aspirin "Non-Response" oder zur Unterdosierung in einigen Patienten beitragen, indem sie die Präsentation von GRP78/BIP auf der Thrombozytenoberfläche induzieren. Das Medikament ist dann mit der Hemmung der ATPase-Aktivität des GRP78 beschäftigt. Es ist daher sinnvoll, den Gehalt an missgefalteten Proteinen/Peptiden im Blut von Patienten unter Therapie mit Aspirin zu detektieren und zu messen.

Hierfür eignen sich insbesondere oben beschriebene Verfahren zur Bestimmung aller missgefalteten Proteine im Blut oder Plasma mittels z.B. Plasmon Resonanz-Messung oder eine Methode zur Quantifizierung in einem Sandwichverfahren, wobei beide genutzten Bindesubstanzen generelle Bindungspartner für missgefaltete Proteine/Peptide sind und diese mit hoher Affinität binden.

Auch für Medikamente aus der Gruppe der Thienopyridine wurde ein individuell stark unterschiedliches Ansprechen beobachtet, was dem Therapieerfolg entgegenstehen kann ¹⁰⁷.

HOCI-modifizierte missgefaltete Proteine/Peptide interagieren zudem durch eine reaktive Gruppe, welche mit freien SH-Gruppen reagiert, direkt mit den aktiven Metaboliten der Thienopyridine und beeinflussen so die Therapie mit Thienopyridinen, wie Clopidogrel und Prasugrel. Da die Thiolgruppen der aktiven Metabolite der Thienopyridine für die Hemmung von P1Y12 essentiell sind, stehen freie Metabolite zur Inhibition von P2Y12 in geringerer Konzentration zur Verfügung. HOCI-modifizierte missgefaltete Proteine können aktive Metabolite der Thienopyridine wegfangen und so die geplante therapeutische Hemmung der ADP induzierten Thrombozytenaktivierung negativ beeinflussen (Brodde, Heptinstall, Kehrel, Manuskript in Vorbereitung).

Die Aktivität der Paraoxonase-1, einem Enzym im HDL, wurde mit dem unterschiedlichen Ansprechen zwischen Individuen auf Clopidogrel kausal in Verbindung gebracht ¹⁰⁸. Patienten, welche *PON1* QQ192 homozygot waren, zeigten ein höheres Risikos für Stent-thrombosen als PON1 RR192 homozygote Patienten. *PON1* QQ192 Homozygotie ist mit geringeren Paraoxonase-1 Aktivitäten im Plasma, einer niedrigeren Konzentration von aktiven Metaboliten des Clopidogrels und einer schlechteren Inhibition der ADP induzierten Plättchenaktivierung verbunden. Die Paraoxonase-1 ist an der Bioaktivierung von Clopidogrel beteiligt. Das Enzym wird durch HOCI, Acrolein und reaktive Gruppen an Proteinen, welche durch Acrolein verändert wurden, inaktiviert ¹⁰⁹. HOCI-modifizierte, missgefaltete Proteine sind daher mit hoher Wahrscheinlichkeit ursächlich an den nicht genetischen Gründen für ein schlechtes Ansprechen auf Medikamente aus der Gruppe der Thienopyridine (Clopidogrel, Prasugrel, Ticlopidin) beteiligt.

Eine Quantifizierung von HOCI-modifizierten missgefalteten Proteine/Peptide ist aber auch schon deshalb sinnvoll, weil solche Substanzen starke Thrombozytenaktivatoren sind und eine Thrombenbildung auslösen können. Als eine der reaktiven Gruppen auf HOCI-modifizierten missgefalteten Proteinen/Peptiden, welche mit freien Thiolgruppen reagieren, wurde von uns das FDP-Lysin (N (epsilon)-(3-formyl-3,4-dehydropiperidino)lysine) auf missgefalteten Proteinen gefunden. Durch spezifische im Handel erhältliche monoklonale Antikörper gegen diese Gruppe, sind missgefaltete Proteine/Peptide, welche die Therapie mit Thienopyridinen beeinflussen können, in Kombination mit Bindesubstanzen für missgefaltete Proteine/Peptide, detektier- und messbar. Auch durch die Kombination einer Bindungssubstanz für missgefaltete Proteine/Peptide mit Detektoren, welche selbst freie SH-Gruppen tragen, sind missgefaltete Proteine/Peptide, welche die Therapie mit Thienopyridinen beeinflussen können, messbar.

Als eine Ausführungsform wird ein Reagenz, welches missgefaltete Proteine/Peptide bindet, nämlich eine Bindesubstanz gemäß dieser Erfindung, an eine solide Oberfläche immobilisiert und nachfolgend mit einer Blut, Plasma oder Serumprobe eines Patienten in Verbindung gebracht. Gruppen, welche an Thiol binden, können dann auf unterschiedliche Weise detektiert werden, wie z.B. durch Antikörper gegen FDP-Lysin aber auch durch Detektionsreagenzien, welche selbst freie SH-Gruppen tragen, wie z.B. aber nicht beschränkt auf red.-Glutathion-Gold, red.-Glutathion-Biotin, zusammen mit einem geeigneten Nachweissystem (siehe oben).

Unter Verwendung von einer Festphase, die freie SH-Gruppen trägt, wie, aber nicht beschränkt auf, Mikrotiterplatten oder Beads, an welche Glutathion immobilisiert worden ist, wie z.B. Thermo Scientific Pierce Glutathione Coated Plate, Thermo Scientific Pierce Magnetic Glutathione Beads, kann der Sandwich Assay auch vice versa aufgebaut werden.

Derartige Reagenzien sind zahlreich im Handel erhältlich, da sie für die Detektion und/oder Reinigung von GST-markierten rekombinanten Proteinen entwickelt wurden. Somit sind sie dem Fachmann geläufig.

Die Aktivierung von Thrombozyten durch missgefaltete Proteine/Peptide kann, wie die Patenanmeldung DE 10 2010 043 733.6 zeigt, auch durch unbeladene PEGylierte Liposomen inhibiert werden. Solche PEGylierten Liposomen sind schon als Hilfsmittel für den Transport von Medikamenten in klinischen Studien eingesetzt worden. Daher ist zu erwarten, dass PEGylierte Liposomen selbst direkt in Säugetieren und Menschen eingesetzt werden können. Zum Monitoring einer Therapie mit PEGylierten Liposomen in Säugetieren, besonders bevorzugt in Tieren, welche für vorklinische Studien eingesetzt werden, und im Menschen kann die in vitro Detektion und Messung von missgefalteten Proteinen/Peptiden erfindungsgemäß eingesetzt werden.

Hierzu eignen sich die gleichen erfindungsgemäßen Verfahren, die für das Monitoring von Aspirin in dieser Patentanmeldung beschrieben wurden.

Die Aktivität der sekretorische Phospholipase A2 (sPLA2) im Blut/Plasma von Patienten ist ein Biomarker für kardiovaskulären Erkrankungen ¹¹⁰⁻¹¹⁴ metabolisches Syndrom ¹¹⁵, Multiorganversagen nach schwerem Trauma ¹¹⁶, ¹¹⁷. Männliches Geschlecht, Obesitas und eine hohe Konzentration an LDL Cholesterol sind statistisch mit hohen Aktivitäten von sPLA2 verbunden, wie viele Studien gezeigt haben.

Inhibitoren der sPLA2, wie Varespladib methyl, werden in Studien zur Therapie von Herzkreislauf-Erkrankungen auf dem Boden einer Arteriosklerose erfolgreich eingesetzt ^{118, 119}.

Unter anderen Funktionen kann sPLA2 direkt LDL so modifizieren, dass dieses atherogener wird. Das Apolipoprotein B-100 im LDL wird verändert und zeigt eine höhere Affinität zu Glycosaminoglykanen und Proteoglykanen. Die hohe Affinität zu Heparin, Glycosaminoglykanen und Proteoglykanen ist ein häufig beobachtetes, aber recht unspezifisches Phänomen von missgefalteten Proteinen. Tatsächlich modifiziert die Calzium-abhängige sPLA2 das LDL zu elektronegativem LDL (LDL(-)), welches, wie physikochemische Methoden wie Zirkulardichroismus gezeigt haben, eindeutig missgefaltete Proteine enthält ^{120, 121}

Erfindungsgemäß kann die Aufgabe der Therapieüberwachung bei Therapie mit sPLA2 Inhibitoren dadurch gelöst werden, dass in einem in vitro Assay mit zwei Bindesubstanzen missgefaltetes Apolipoprotein B100 detekiert und gemessen wird. Eine der Bindesubstanzen ist eine Substanz, welche mit hoher Affinität Apolipoprotein B100 erkennt, die andere Bindesubstanz ist eine der in dieser Patentanmeldung offengelegten Bindesubstanzen für missgefaltete Proteine/Peptide und ggf. eine der anderen bisher bekannten Bindesubstanzen für missgefaltete Proteine, wie, aber nicht beschränkt auf, Chaperone wie GRP78, tPA, HGFA, Fibronektin und FXII.

Ein Protein, Polypeptid oder Peptid wird i eine solide Oberfläche immobilisiert. Blut, Plasma oder Serum des Patienten oder Probanden, oder Säugetieres wird in Kontakt mit der vorbereiteten Oberfläche gebracht, sodass missgefaltete Proteine/Peptide an die Bindungssubstanzen andocken können. Der Nachweis von Apolipoprotein B100 unter den gebundenen Proteinen erfolgt mittels eines spezifischen Nachweisreagenzes für Apolipoprotein B100 und einem geeignetem Nachweissystem, analog zu den im Abschnitt Diagnostik beschriebenen Verfahren.

Extrazelluläre missgefaltete Proteine können an Chaperon-Proteine auf der Oberfläche von Zellen andocken. Auf gestressten Zellen, insbesondere auf Tumorzellen, Endothelzellen, Monozyten, glatten Muskelzellen, Kardiomyozyten und vielen anderen, wurde GRP78/Bip auf der Zelloberfläche gefunden.

Es wird in Verbindung gebracht mit dem Ansprechen von Tumorzellen auf Chemotherapeutika ¹²².

Für das Prostatakarzinom wurde kürzlich festgestellt, dass das extrazelluläre Prostata-spezifisches Antigen (PSA) einen Komplex mit alpha2 Makroglubulin eingeht und dass dieser Komplex an GRP78 auf der Prostatakarzinomzelle bindet. Missgefaltetes alpha2 Makroglobulin bindet an GRP78 und löst ein Signal aus. Dieses Signal über GRP78 steigert die Zellproliferation und Migration der Tumorzelle und unterdrückt die Autophagie ¹²³. GRP78 Rezeptoren wurden auf vielen Tumorzellen, wie Brustkrebs, Ovarkrebs, Leber- und Darmkrebs, Lungenkrebs und Melanomzellen gefunden. Autoantikörper gegen GRP78 auf Melanomzellen fördern in der Maus das Tumorwachstum ¹²⁴. Es ist wahrscheinlich, dass Komplexe aus anderen Proteinen mit missgefaltetem alpha2 Makroglobulin auch für die Pathogenese anderer Tumoren wichtig sind.

GRP78 auf der Zelloberfläche ist beteiligt an der Regulation von Angiogenese, Endothelproliferation, Endothelzellmigration, Anlocken von endothelialen Progenitorzellen ¹²⁵ an Gewebsreparatur und Wundheilung z.B. nach Herzinfarkt ¹²⁶, aber auch an der Tumorneoangiogenese ¹²⁷. Viele Substanzen, welche als Inhibitoren der Angiogenese in klinischen Prüfungen waren oder sind, sind selbst missgefaltete Proteine/Proteinfragmente/Peptide 128

Die erfindungsgemäße Detektion und Messung von missgefalteten Proteinen/Peptiden gemäß den Ansprüchen der vorliegenden Erfindung und die damit ermöglichte Auswahl eines für eine spezifische Erkrankung beteiligten spezifisch missgefalteten Proteins/Peptids ist somit auch für Diagnostik und Therapiemonitoring von Erkrankungen, die heute noch nicht mit der Missfaltung von Proteinen/Peptiden in Verbindung gebracht werden, wie die Therapie zur Förderung oder Inhibierung der Angiogenese, die Chemotherapie zur Tumorbekämpfung oder die Therapie zur Verbesserung der Wundheilung von Geweben sinnvoll und mit den offengelegten Bindungssubstanzen für missgefaltete Proteine/Peptideanwendbar.

**3. Abtrennung und Isolierung von missgefalteten Proteinen/Peptiden** Eine andere Ausführungsform der Erfindung nutzt die in dieser Patentanmeldung offengelegten Bindesubstanzen für missgefaltete Proteine/Peptide nämlich ADAM15 (Metargidin), die Metalloproteasedomäne von ADAM15 und insbesondere Peptide, welche die Sequenz His, Trp, Arg, Arg, Ala, His, Leu, Leu, Pro, Arg, Leu, Pro (HWRRAHLLPRLP) entsprechend der ADAM15 Sequenz 286-297, die Sequenz Glu, Asn, Phe, Leu, His, Trp, Arg, Arg, Ala, His, Leu, Leu (ENFLHWRRAHLL) ADAM 15 Sequenz 282-293, oder die Sequenz Ala, Val, Thr, Leu, Glu, Asn, Phe, Leu, His, Trp, Arg, Arg ADAM15 Sequenz 279-290, His, Trp, Arg, Arg, Pro oder His, Trp, Arg, Arg (HWRR) haben, um missgefaltete Proteine/Peptide aus Materialien/Objekten oder in vitro aus Körperflüssigkeiten zu entfernen oder den Gehalt an missgefalteten Proteinen/Peptiden zu reduzieren.

### 4. Detektion, Abtrennung und Isolierung von missgefalteten Proteinen/Peptiden in/aus Arzneimitteln, Arzneizusatzstoffen und pharmazeutischen Zusammensetzungen, in/aus Reagenzien und Hilfsreagenzien für diagnostische Tests, in/aus Forschungsreagenzien und in/aus Lebensmitteln und Flüssigkeiten, sowie Kontrolle des Herstellungsprozesses und/oder Lagerungsprozesses von Arzneimitteln, Arzneimittelzusatzstoffen, Reagenzien und Hilfsreagenzien für diagnostische Tests, Forschungsreagenzien und Biofilmen.

### a) Arzneimittel, Arzneizusatzstoffe und pharmazeutische Zusammensetzungen

Der Markt an Medikamenten, welche Proteine oder Peptide enthalten, insbesondere Blutprodukte oder Antikörper-basierte Therapeutika und andere rekombinant hergestellte Medikamente, wächst gewaltig.

Ein großes Problem bei der Herstellung von rekombinanten Proteinen/Peptiden, aber auch bei der Nutzung von Proteinen/Peptiden aus biologischen Material, wie Blut, Plasma, Serum, ist die chemische und physikalische Destabilisierung des Proteins/Peptids, welche zu Missfaltungen führt. Diese kann auf allen Stufen des Herstellungsprozesses passieren, wie in der Zellkultur, im Ausgangsmaterial, während Reinigung und Virusinaktivierung sowie der Formulierung, bei der Lagerung, im Handel und beim Gebrauch des Produktes. Ein Grund dafür ist die Tatsache, dass die Proteine/Peptide in wirtsfremden Organismen exprimiert werden, die Konzentration der exprimierten Proteine/Peptide recht hoch ist und die zur Synthese genutzten Zellen unter Stress stehen. Bei der Reinigung der Proteine/Peptide fehlen natürliche Begleitstoffe wie Lipide, Lipoproteine und andere Proteine, welche in natürlicher Umgebung die hergestellten Proteine/Peptide stabilisieren. Einige rekombinante Proteine/Peptide werden gar aus Einschlusskörperchen, welche sich in den exprimierenden Organismen aus dem missgefalteten Protein/Peptid gebildet haben, aufgereinigt, da die Einschlusskörperchen gut vom Zellmaterial abtrennbar sind. Solche Proteine/Peptide müssen dann wieder in eine halbwegs native Konformation zurück gebracht werden.

Bei der Reinigung eines Proteins bedingen weitere Konditionen Missfaltungen. So beeinflussen zum Beispiel Scherstress, Lösungsmittel, Detergenzien, pH-Wert, Temperatur, Einfrieren und Auftauprozess, Material der Oberflächen mit denen das Zielprotein in Berührung kommt, lonenstärke, Übergänge von Grenzschichten, wie die Luft-Flüssigkeitsgrenzschicht, Lyophilisierung, Kälte, Hitze, Druck, Bestrahlung (UV,IR, X-rays), "Vortexen", "Ultraschall", Rühren, Schütteln, hohe Proteinkonzentrationen und chemische Modifikationen die Stabilität der nativen Protein-/Peptidkonformation. Chemische Modifikationen sind zum Beispiel proteolytische Spaltungen, Oxidation, Carbamylierung, beta-Eliminierung, Racemisierung, Deamidierung, und Acylierung des hergestellten oder gereinigten Zielproteins. Cystein enthaltende Proteine können intermolekulare Disulfidbrücken bilden. Insbesondere globuläre Proteine sind in wässrigen Lösungen recht instabil und neigen zu Missfaltungen und anschließender Aggregation. Solche Proteine können präzipitieren und Chromatographiesäulen und Filter verstopfen. Missfaltung von Zielproteinen führt in vielen Fällen zum Verlust der Aktivität und damit zur Verringerung der spezifischen Aktivität eines Präparates. Die genannten Mechanismen können die Zielproteine/-peptide, wie auch Begleitproteine/Peptide betreffen. Die Affinitätschromatographie nutzt spezifische Interaktionen zwischen dem Protein/Peptid, welches gereinigt werden soll, und seinem immobilisierten Liganden. Oft sind die Elutionsbedingungen ziemlich harsch. Ionenaustauschchromatographie oder hydrophobe Interaktions-Chromatographie verursachen auch Stress für die Proteinkonformation .

Auch bei der Lagerung von Arzneimitteln, welche Proteine oder Peptide als Wirk-oder Zusatzstoff enthalten, kann es durch physikalische und chemische Prozesse zu Missfaltung dieser Inhaltsstoffe kommen.

Arzneimittel, welche aus Material stammen, welches einem tierischen oder menschlichen Körper entnommen wurden, können schon durch das Ausgangsmaterial missgefaltete Proteine/Peptide enthalten. Insbesondere dann, wenn der Spender des Ausgangsmaterials an einer der vielen Erkrankungen leidet, welche mit Proteinmissfaltung einhergeht, insbesondere dann, wenn die Erkrankung noch nicht offensichtlich ist, und so weder dem Patienten noch einem Arzt bisher aufgefallen ist.

Zu den Medikamenten, welche Proteine/Peptide als Wirkstoffe oder Zusatzstoffe enthalten, gehören z.B. Antikörper, Impfstoffe, aus Blut gereinigte Gerinnungsfaktorpräparate, Hormone, Zytokine und Wachstumsfaktoren, Blutbankprodukte wie Plasma und Zellkonzentrate, rekombinante Proteine als Immunsuppressiva , rekombinante Proteine als Antirheumatika und antiinflammatorische Wirkstoffe, rekombinante Proteine gegen Osteoporose, rekombinante Proteine als Antirheumatika und antiinflammatorische Wirkstoffe, rekombinante Proteine bei Krebs, rekombinante Proteine zur Beeinflussung der Blutgerinnung, rekombinante Proteine bei Diabetes, rekombinante und synthetische Hypophysen- und Hypothalamushormone, rekombinante Proteine zur Beeinflussung der Blutzellbildung, rekombinante Proteine zur Wundbehandlung und Psoriasis. Viele Produkte beinhalten als Stabilisator ein anderes Protein, wie z.B. Humanalbumin.

Lagerungsbedingte Veränderungen im Arzneimittel können eine deutliche Beeinträchtigung der Wirkung mit sich bringen. Als Beispiel sei hier ein Blutbankpräparat genannt. So beschrieben Koch et al. dass Erythrozytenkonzentrate mit der Lagerungsdauer schlechter verträglich werden können ¹²⁹ Einfach nach kurzer Lagerung alle Erythrozytenkonzentrate zu vernichten ist in Anbetracht der Knappheit dieses wichtigen Medikaments keine Lösung des Problems. Eine Methode, welche es erlauben würde, diejenigen Erythrozytenpräparate herauszufischen, welche eine schlechtere Wirksamkeit haben und welche die in der zitierten Arbeit beschriebenen Nebenwirkungen verursachen, wäre ein großer Fortschritt für die Medizin.

Medikamente, welche Proteine/Peptide enthalten, können Gesundheitsprobleme verursachen. So sind als Nebenwirkungen z.B. anaphylaktische Reaktionen, Fieber, Störung der Hämostase, Fibrinolyse, Sepsis, disseminierte intravasale Gerinnung (DIC), Thrombosen, und (Auto)antikörperbildungen beschrieben worden. Die beschriebenen Nebenwirkungen sind oft Chargenabhängig und abhängig vom Gesundheitszustand des Patienten, der diese Arzneimittel erhält. Da Missfaltungen von Proteinen/Peptiden mit Veränderungen der Immunabwehr, Antikörperbildung und Störungen der Hämostase/Fibrinolyse direkt in Verbindung stehen, ist es wahrscheinlich, dass missgefaltete Proteine/Peptide in den Medikamenten oder Medikamentzusatzstoffen direkt am Nachlassen der Produktqualität und am Auftreten der Nebenwirkungen beteiligt sind.

Hermeling et al. beschreiben den Zusammenhang zwischen der Faltung eines Proteins/Peptides und seiner Immunogenizität ¹³⁰. Rosenberg berichtet, dass Medikamente, welche oxidierte oder aggregierte Proteine enthalten, immunogener sind ¹³¹. Joubert et al beschreiben eine Aktivierung der innaten Immunreaktion durch aggregierte Proteine in Medikamenten ¹³².

Missfaltung von Proteinen/Peptiden in Medikamenten kann die Qualität und Effizienz des Produktes ungünstig verändern ¹³³.
Daher sind Methoden notwendig, welche solche Veränderungen anzeigen ¹³⁴⁻¹³⁸

Die Detektion von Aggregaten, eine Ausführungsform von missgefalteten Proteinen/Peptiden, gehört in der Pharmazeutischen Biotechnologie zum Standard. Mit Verfahren, welche die Aggregatbildung von Proteinen monitoren, werden Produktionsprozesse modifiziert und optimiert. Zu diesen Verfahren gehören langwierige physikalische Methoden wie Analytische Techniken: "Dynamic Light Scattering (DLS)", "Multi-Angle Light Laser Scattering (MALLS)", UV Spektroskopie, "Light Obscuration", "Micro-Flow Imaging (MFI)", "Nanoparticle Tracking Analysis (NTA)" und Separationstechniken wie: "Size Exclusion Chromatography (SEC)", "Field Flow Fractionation (FFF)", "Capillary Electrophoresis. (CE)" oder analytische Ultrazentifugation ¹³⁹.

Eine erfindungsgemäße Ausführung der Erfindung ist es, missgefaltete Proteine/Peptide schon unabhängig von der Aggregatbildung gemäß den Ansprüchen zu detektieren, und damit den gesamten Herstellungsprozess (siehe oben) zu optimieren und die Ausbeute an Medikament für den Hersteller und die Qualität des Medikaments für den Patienten zu erhöhen.
Ziel ist es, so wenig wie möglich missgefaltete Proteine/Peptide im gesamten Herstellungsprozess zu erzeugen und/oder im Medikament zu belassen.

Mit Hilfe der Bindungsubstanzen können missgefaltete Proteine/Peptide in Herstellungsstufen und im Medikament einfach und schnell detektiert werden. Das Vorgehen ist analog zu den im Abschnitt Diagnostik beschriebenen Verfahren. Statt einer Körperflüssigkeit als Testsubstanz wird jedoch eine Substanzprobe aus einem beliebigen Herstellungsschritt, wie, aber nicht beschränkt auf, das Ausgangsmaterial, Zellkultur, eines der Reinigungsverfahren, Virusinaktivierungsverfahren, Formulierung, Lagerung, Transport, Handel, Handling durch den Verbraucher, auf das Vorhandensein von missgefalteten Proteinen/Peptiden getestet und dies dokumentiert. Als Testverfahren eignen sich z.B. Mikrotiterplattenassay mit Nutzung der erfindungsgemäßen Bindesubstanz, aber auch Plasmon Resonanz, Bead-assay, Turbimetrie und viele andere, welche dem Fachmann geläufig sind.

Eine Eliminierung oder Abreicherung von missgebildeten Proteinen/Peptiden aus Produktionschritten der Herstellung des Medikamentes ist erfindungsgemäß durch ein Verfahren durchführbar, bei dem ADAM15 (Metargidin), die Metalloproteasedomäne von ADAM15 und insbesondere Peptide, welche die Sequenz His, Trp, Arg, Arg, Ala, His, Leu, Leu, Pro, Arg, Leu, Pro (HWRRAHLLPRLP) entsprechend der ADAM15 Sequenz 286-297, die Sequenz Glu, Asn, Phe, Leu, His, Trp, Arg, Arg, Ala, His, Leu, Leu (ENFLHWRRAHLL) ADAM 15 Sequenz 282-293, oder die Sequenz Ala, Val, Thr, Leu, Glu, Asn, Phe, Leu, His, Trp, Arg, Arg ADAM15 Sequenz 279-290, His, Trp, Arg, Arg, Pro oder His, Trp, Arg, Arg (HWRR) haben, an eine feste Oberfläche immobilisiert wird. Diese kann bevorzugt ein Filtersystem, eine Membran, ein Schlauchsystem oder eine Matrix sein, wie sie üblicherweise für Affinitätschromatographien verwendet wird. Die Abtrennung der missgefalteten Proteine/Peptide erfolgt, indem man die missgefalteten Proteine/Peptide aus den Materialien der Herstellungsschritte des Medikamentes oder aus dem fertigen Medikament an die beschichtete Matrix binden lässt und dann das in Lösung befindliche Medikament, welches dann geringere Konzentrationen an missgefalteten Proteinen/Peptiden oder keine mehr enthält, von der festen Matrix trennt.

Bei der erfindungsgemäß bevorzugten Nutzung der oben genannten Peptide, kann das entfernte missgefaltete Protein/Peptidmaterial in einem folgenden Schritt einfach mittels eines Überschusses an freiem Peptid eluiert werden. Dies ist sinnvoll für spätere Analysen des missgefalteten Materials, welche zur Modifizierung und Optimierung des Herstellungsprozess genutzt werden können.

### b) Reagenzien und Hilfsreagenzien für diagnostische Tests

Die Erfindung bezieht sich auch auf Verfahren der Selektion und/ oder Qualitätskontrolle von Reagenzien oder beschichteten Festphasenkomponenten, die für alle Analysen und Assay zur Untersuchung von missgefalteten Proteinen geeignet sind.

An eine Methode oder einen Kit zur Detektion und Messung von missgefalteten Proteinen/Peptiden müssen erfindungsgemäß besondere Anforderungen gestellt werden.

Ein kommerziell und/oder wissenschaftlich brauchbarer Assay erfordert, neben anderen Dingen, dass unter Verwendung solcher Assays erzeugter Ergebnisse von Labor zu Labor, wie auch von Ansatz zu Ansatz reproduzierbar sind. Reagens- und Kit-stabilität während des Versands und/oder Lagerung sind ebenfalls sehr wichtige Kriterien für eine sichere Routineuntersuchung von Assays für missgefaltete Proteine/Peptide.

Die Verwendung von Reagenzien, welche selbst missgefaltete Proteine enthalten, führt zu hohen Variabilitäten zwischen Chargen, hoher, unkalkulierbarer Hintergrundreaktion und schlechter Kit-Stabilität. Insbesonder die Stabilität der Festphasenkomponente, die in den Assays für missgefaltete Proteine verwendet werden, ist äußerst komplex und kritisch. Eine besonders bevorzugte Ausführungsform der vorliegenden Erfindung sind Methoden unter Verwendung der Bindesubstanzen für missgefaltete Proteine/Peptide, welche verwendet werden, um Reagenzien oder insbesondere Festphasenkomponenten für Bindeassays zur Identifikation von missgefalteten Proteinen/Peptiden auszuwählen, die für Bindeassays zur Identifikation von missgefalteten Proteinen/Peptiden geeignet sind, die frei von missgefalteten Proteinen/Peptiden sind, oder in denen missgefaltete Proteine/Peptide nur in geringen Spuren vorkommen, und damit in den Assays zur Detektion und Quantifizierung von missgefalteten Proteinen/Peptiden verwendet werden können.

Ein Reagenz im Sinne der vorliegenden Offenbarung ist ein in einem Assay zur Detektion von missgefalteten Proteinen verwendetes Biomolekül, das Prozessen unterliegen kann, welche zur Missfaltung von Proteinen/Peptiden führen, z.B. einer Pufferkomponente, einem Stabilisierungsprotein, einer Komponente zur Beschichtung oder zum Blockieren von nicht-spezifischen Bindern der Festphase, wie, aber nicht beschränkt auf, Rinderserumalbumin, Humanalbumin, Magermilchpulver, während der Herstellung der Festphase verwendete Reagenzien, ein Nachweisreagenz, insbesondere, wenn es sich dabei um einen Antikörper oder ein andere Protein, insbesondere wenn es sich um ein rekombinantes Protein handelt. Von missgefalteten Proteinen/Peptiden freie oder zumindest im Wesentlichen von missgefalteten Proteinen/Peptiden freie Reagenzien werden ausgewählt und verwendet. Der Begriff Festphasenmaterialien /-komponente wird verwendet um Festphasen oder Trägermaterialien zu beschreiben, welche routinemäßig in Bindeassays verwendet werden. In der vorliegenden Erfindung wird ein Verfahren zur Herstellung einer Festphasenkomponente, und Hilfsreagenzien, wie Blockierungsreagenz und Nachweisreagenz, beschrieben, welches frei von missgefalteten Proteinen/Peptiden ist. Beschichten der Festphasenkomponente mit einem spezifischen Bindungspartner, der frei von missgefalteten Proteinen/Peptiden ist. Waschen der Festkörperkomponente und zugeben einer Blockier- oder Stabilisierlösung, welche frei von missgefalteten Proteinen/Peptiden ist. Nutzung eines Nachweisreagenzes/Bindepaarmitgliedes, welches frei von missgefalteten Proteinen/Peptiden ist. Ein besonderes Augenmerk gilt hier der Verwendung von Avidin, Streptavidin, Rinderserumalbulin, Humanalbulin, Magermilchpulver, jeder Art von Antikörpern und Nachweisenzymen.

Die Nutzung der vorliegenden Erfindung beschränkt sich nicht auf Reagenzien, welche in diagnostischen Tests zur Detektion und Messung von missgefalteten Proteinen/Peptiden genutzt werden. Allgemein ist es in diagnostischen Tests , in denen Proteine oder Peptide verwendet werden von Vorteil, dass keine missgefaltetenProteine/Peptide in den Reagenzien vorkommen, da diese unspezifische Reaktionen eingehen können, welche das Testergebniss negativ beeinflussen.

Mit Hilfe der Bindungsubstanzen können missgefaltete Proteine/Peptide in Herstellungsstufen und im diagnostischen Reagenz einfach und schnell detektiert werden. Das Vorgehen ist analog zu den im Abschnitt Diagnostik beschriebenen Verfahren. Statt einer Körperflüssigkeit als Testsubstanz wird jedoch eine Substanzprobe aus einem beliebigen Herstellungsschritt, wie, aber nicht beschränkt auf, das Ausgangsmaterial, Zellkultur, eines der Reinigungsverfahren, Virusinaktivierungsverfahren, Formulierung, Lagerung, Transport, Handel, Handling durch den Verbraucher oder dem diagnostischen Reagens selbst auf das Vorhandensein von missgefalteten Proteinen/Peptiden getestet und dies dokumentiert. Als Testverfahren eignen sich z.B. Mikrotiterplattenassay mit Nutzung der erfindungsgemäßen Bindesubstanz, aber auch Plasmon Resonanz, Bead-assay, Turbimetry und viele andere, welche dem Fachmann geläufig sind. Eine Eliminierung oder Abreicherung von missgebildeten Proteinen/Peptiden aus Produktionschritten der Herstellung des Reagenzes für ein diagnostisches Verfahren oder einen diagnostischen Kit ist erfindungsgemäß durch ein Verfahren durchführbar, bei dem ADAM15 (Metargidin), die Metalloproteasedomäne von ADAM15 und insbesondere Peptide, welche die Sequenz His, Trp, Arg, Arg, Ala, His, Leu, Leu, Pro, Arg, Leu, Pro (HWRRAHLLPRLP) entsprechend der ADAM15 Sequenz 286-297, die Sequenz Glu, Asn, Phe, Leu, His, Trp, Arg, Arg, Ala, His, Leu, Leu (ENFLHWRRAHLL) ADAM 15 Sequenz 282-293, oder die Sequenz Ala, Val, Thr, Leu, Glu, Asn, Phe, Leu, His, Trp, Arg, Arg ADAM15 Sequenz 279-290, His, Trp, Arg, Arg, Pro oder His, Trp, Arg, Arg (HWRR) haben, an eine feste Oberfläche immobilisiert wird. Diese kann bevorzugt ein Filtersystem, eine Membran, ein Schlauchsystem oder eine Matrix sein, wie sie üblicherweise für Affinitätschromatographien verwendet wird.

Die Abtrennung der missgefalteten Proteine/Peptide erfolgt analog zu dem unter Bindung von missgefalteten Proteinen/Peptiden aus Körperflüssigkeiten beschriebenem Verfahren, indem man die missgefalteten Proteine/Petide aus den Material der Herstellungsschritte des Medikamentes oder aus dem fertigen Medikament an die beschichtete Matrix binden lässt und dann das in Lösung befindliche Medikament, welches dann geringere Konzentrationen an missgefalteten Proteinen/Peptiden oder keine mehr enthält, von der festen Matrix trennt.

### c) Forschungsreagenzien

Bei allen wissenschaftlichen Untersuchungen im Bereich Forschung und allen Untersuchungen zur Findung oder Testung von Therapeutika und Diagnostika, bei denen andere Proteine wie z.B. Rezeptoren, an welche missgefaltete Proteine/Peptide binden können oder bei denen die Testreagenzien selbst missgefaltete Proteine/Peptide sind, eine Rolle spielen, ist es für die richtige Deutung der erzielten Ergebnisse unabdingbar, dass die Begleitreagenzien, damit sind Reagenzien oder Zusatzstoffe gemeint, welche selbst nicht im Mittelpunkt der Untersuchung stehen, wie insbesondere Zellkulturmedien, Zellkulturmedienzusätze, ganz besonders Seren aus Säugetieren wie fetales Kälberserum, Reagenzien zur Stabilisierung von Proteinen, hier insbesondere Zusätze wie Albumin, Rinderserumalbumin, Magermilchpulver, keine missgefalteten Proteine enthalten. Dies ist besonders dann wichtig, wenn für das Experiment eine Bindung oder die Auswirkung einer Bindung von einer Testsubstanz an ein anderes Protein getestet werden soll, welches missgefaltete Proteine/Peptide erkennen kann. Zur richtigen Deutung von Versuchsergebnissen ist es für den Untersucher zudem wichtig zu wissen, ob einer der Reaktionspartner ein missgefaltetes Protein/Peptid ist.

Eine solche Auswahl von geeigneten Reagenzien oder Festphasenkomponenten ist erfindungsgemäß insbesondere wichtig für Untersuchungen oder Medikamentenscreening und -testung in den Bereichen Fibrinolyse, tPA, Plasminogen, Kontaktphasenaktivierung, FXII, Chaperone, Phagozytose, Apoptose, Zellproliferation, Wundheilung, Amyloidosen, Wirkung von ADAMs, Wachstumsfaktoren, extrazelluläre Matrix, Infektion, Entzündung, Ischämie, Ischämie-Reperfusionschaden, Rheuma, Autoantikörpermessung, Autoantikörperbildung, Vaccine, Gerinnung, Antikörperbildung, innate und adaptive Abwehr, Arteriosklerose, neurodegenerative Erkrankungen, Sepsis, Multiorganversagen, Biomarker, Biokompatibilität von Materialien, ansprechen von Tumoren auf Chemotherapeutika, Angiogenese, Neoangiogenese, Shedding von Oberflächenproteinen, Quantifizierung von abgespaltenen Proteinen/Peptiden, grundsätzlich allen Arbeiten mit rekombinanten Proteinen und mit Mikroorganismen.

Die vorliegende Erfindung ist daher in einer weiteren Ausführungsform auch ein Verfahren zur Selektion und/oder Qualitätskontrolle eines Forschungsreagenzes und zur Entfernung von missgefalteten Proteinen aus Forschungsreagenzien.

Mit Hilfe der Bindungssubstanzen können missgefaltete Proteine/Peptide in Forschungsreagenzien einfach und schnell detektiert werden. Das Vorgehen ist analog zu den im Abschnitt Diagnostik beschriebenen Verfahren. Statt einer Körperflüssigkeit als Testsubstanz wird jedoch eine Substanzprobe aus einem beliebigen Herstellungsschritt wie, aber nicht beschränkt auf, das Ausgangsmaterial, Zellkultur, eines der Reinigungsverfahren, Virusinaktivierungsverfahren, Formulierung, Lagerung, Transport, Handel, Handling durch den Verbraucher) eines Forschungsreagenzes oder dem Forschungsreagenz selbst auf das Vorhandensein von missgefalteten Proteinen/Peptiden getestet und dies dokumentiert. Als Testverfahren eignen sich z.B. Mikrotiterplattenassay mit Nutzung der erfindungsgemäßen Bindesubstanz, aber auch Plasmon Resonanz, Bead-assay, Turbimetry und viele andere, welche dem Fachmann geläufig sind. Das Verfahren eignet sich auch zur Otimierung der Stabilität eines Forschungsreagenzes und zur Testung des Einflusses von Lagerungsbedingungen.

Eine Eliminierung oder Abreicherung von missgebildeten Proteinen/Peptiden aus Produktionschritten der Herstellung des Forschungsreagenzes oder eines seiner Herstellungsvorstufen ist erfindungsgemäß durch ein Verfahren durchführbar, bei dem ADAM15 (Metargidin), die Metalloproteasedomäne von ADAM15 und insbesondere Peptide, welche die Sequenz His, Trp, Arg, Arg, Ala, His, Leu, Leu, Pro, Arg, Leu, Pro (HWRRAHLLPRLP) entsprechend der ADAM15 Sequenz 286-297, die Sequenz Glu, Asn, Phe, Leu, His, Trp, Arg, Arg, Ala, His, Leu, Leu (ENFLHWRRAHLL) ADAM 15 Sequenz 282-293, oder die Sequenz Ala, Val, Thr, Leu, Glu, Asn, Phe, Leu, His, Trp, Arg, Arg ADAM15 Sequenz 279-290, His, Trp, Arg, Arg, Pro oder His, Trp, Arg, Arg (HWRR) haben, an eine feste Oberfläche immobilisiert wird. Diese kann bevorzugt ein Filtersystem, eine Membran, ein Schlauchsystem oder eine Matrix sein, wie sie üblicherweise für Affinitätschromatographien verwendet wird.

Die Abtrennung der missgefalteten Proteine/Peptide erfolgt analog zu dem unter Bindung von missgefalteten Proteinen/Peptiden aus Körperflüssigkeiten beschriebenem Verfahren, indem man die missgefalteten Proteine/Petide aus den Material der Herstellungsschritte des Medikamentes oder aus dem fertigen Medikament an die beschichtete Matrix binden lässt und dann das in Lösung befindliche Medikament, welches dann geringere Konzentrationen an missgefalteten Proteinen/Peptiden oder keine mehr enthält, von der festen Matrix trennt.

### d) Biofilme

Biofilme entstehen, wenn Mikroorganismen wie Bakterien, Algen, Einzeller, Pilze, sich an Grenzflächen ansiedeln. Sie bestehen aus einer dünnen schleimigen, extrazellulären Grundsubstanz aus Polymeren, in der Mikroorganismen eingebettet sind und bilden sich überwiegend in wässrigen Systemen, entweder auf der Wasseroberfläche oder auf einer Grenzfläche zu einer festen Phase ¹⁴⁰. Man findet sie quasi überall, wo ein Leben für Mikroorganismen möglich ist, zum Beispiel auf nährstoffreichem, fließendem oder stehendem Wasser, in Flüssen, Bächen, Seen, auf der Oberfläche von Zähnen, Zahnersatz, künstlichen Linsen und Kontaktlinsen, auf Lebensmitteln, auf Materialien zum Einsatz im Körper, wie Kathetern, Implantaten, auf chirurgischen Instrumenten, in Hausinstallationen und auf Lebens- und Genussmitteln. Sie können erheblich zur Beeinträchtigung des menschlichen oder tierischen Lebens beitragen, zum Beispiel, aber nicht begrenzt auf, durch Infektionen und Beeinträchtigung der Haltbarkeit von Lebensmitteln ¹⁴¹. Wenn z.B. auf Lebensmitteln sichtbare Beläge oder Gerüche durch Biofilme festgestellt werden können, ist es zu spät durch geeignete Maßnahmen, wie z.B. Überwachung der Kühlung, die Vermehrung der Mikroorganismen effektiv eindämmen zu können.

Es ist daher wichtig, Sensoren und Methoden zur zeitnahen Detektion und Messung von Biofilmen zu entwickeln. Missgefaltete Proteine können sich wie oben beschrieben zu Amyloiden zusammenlagern. Amyloide Adhäsionsproteine wurden in Hülle und Fülle in natürlichen Biofilmen gefunden ¹⁴² Somit enthalten Biofilme missgefaltete Proteine/Proteinfragmente/Peptide.

Die Bindesubstanzen für missgefaltete Proteine/Peptide können an ein Nachweissystem gekoppelt und als Sensoren für Biofilme genutzt werden.

Eine weitere Ausführungsform der Erfindung ist daher die Detektion und Messung von Biofilmen mit Hilfe einer Methode gemäß der Ansprüche, bei der eine Bindesubstanz für missgefaltete Proteine/Peptide genutzt wird. Bevorzugt ist diese Bindesubstanz ADAM15 (Metargidin), die Metalloproteasedomäne von ADAM15 und insbesondere Peptide, welche die Sequenz His, Trp, Arg, Arg, Ala, His, Leu, Leu, Pro, Arg, Leu, Pro (HWRRAHLLPRLP) entsprechend der ADAM15 Sequenz 286-297, die Sequenz Glu, Asn, Phe, Leu, His, Trp, Arg, Arg, Ala, His, Leu, Leu (ENFLHWRRAHLL) ADAM 15 Sequenz 282-293, oder die Sequenz Ala, Val, Thr, Leu, Glu, Asn, Phe, Leu, His, Trp, Arg, Arg ADAM15 Sequenz 279-290, His, Trp, Arg, Arg, Pro oder His, Trp, Arg, Arg (HWRR) haben.

Eine zusätzliche Ausführungsform der Erfindung ist die Nutzung der oben beschriebenen Nachweismethode zur Erfassung von gesundheitlichen Risiken sowie zur Charakterisierung von Hygiene- und Qualitätsmängeln, besonders bevorzugt im Bereich Gesundheitswesen, Lebensmittelgewinnung, -lagerung und -handel.

### 5. Detektion und Messung von missgefalteten Proteinen/Peptiden zur Untersuchung der biologischen Verträglichkeit und Bio-/Hämokompatibilität von Materialien und zur Untersuchung des Einflusses eines Medizinproduktes auf die Erzeugung von missgefalteten Proteinen/Peptiden. Verwendung dieser Methode zur Optimierung von Medizinprodukten.

An Materialien, welche in Kontakt mit Blut, Plasma oder Gewebe kommen, müssen besondere Anforderungen gestellt werden. Sie müssen biologisch verträglich und biokompatibel sein. Sind sie in kurzem, langzeitigem oder gar permanentem Kontakt mit Blut, so müssen sie hämokompatibel sein, stehen sie in Kontakt mit Zellen und Geweben, so müssen sie zytokompatibel sein. Der Begriff Biokompatibilitat bedeutet im Sinne der Erfindung die Verträglichkeit von Werkstoffen mit lebendem Gewebe (Knochen, Weichgewebe, Blut) von Menschen und Tieren. Das Material sollte sich möglichst inert verhalten und nicht zu Abbau- oder Abstoßungsreaktionen führen und den Stoffwechsel so wenig wie möglich beeinflussen. Williams definierte dies 2003 ins Deutsche übersetzt so: Die Biokompatibilität eines Medizinproduktes für die Langzeitanwendung bezieht sich auf die Fähigkeit des Produktes die vorgesehene Funktion mit dem erwünschten Grad der Integration in den Organismus auszuführen, ohne in diesem jegliche unerwünschten lokalen oder systemischen Effekte auszulösen¹⁴³.

An Materialen, welche kurzfristig, lang oder permanent mit Blut in Berührung kommen, sind zusätzlich ganz spezifische Anforderungen zu stellen. So sollte die poröse Wand von kleinlumigen, mikroporösen Gefäßprothesen zwar abdichtbar sein, die Innenflächen der Gefäßprothese dürfen aber nicht thrombogen sein, da dies sonst zu einem thrombotischen Verschluss der Prothese oder eines Blutgefäßes kommen kann, in welche ein thrombotischer Embolus getragen worden ist. Herzklappen und Herzunterstützungssysteme dürfen das Blut weder so verändern, dass es im Patienten zu Blutungen, noch dass es zu Thrombosen kommen kann.

Zu den Materialien, welche in Kontakt mit Blut kommen gehören zum Beispiel ein weites Spektrum an Kunststoffen wie Polyethylen, Polypropylen, Polyvinylchlorid, Polyester, Polystyren, Polyurethan, Silikon, Polysulphon, Polyamid, Polytetrafluorethylen etc. sowie deren Derivate, aber auch auch keramische Bauteile, sowie viele metallische Werkstoffe wie Edelstahl, Titan und deren Legierungen.

Beispiel für Medizinprodukte, welche kurzzeitig mit Blut in Berührung kommen, sind Katheter, Atherektomiegeräte, Blutmonitore, Führungsdrähte, intravaskulare Endoskope, intravaskularer Ultraschall, intravaskulare Lasersysteme, retrograde koronare Perfusionskatheter, Herz-Lungen-Maschinen-Kreislaufzubehör, extrakorporale Membranoxygenatoren, Blutdialyse-/Hämofiltrationsausrüstung, Spender und therapeutische Aphereseausrüstung, Geräte für die Absorption spezifischer Substanzen aus dem Blut, interventionelle Kardiologie- und vaskuläre Geräte, perkutane Kreislaufunterstützungssysteme und so weiter. Langfristig bis auf Lebenszeit sind zum Beispiel Annuloplastieringe, prothetische oder biologische Gewebe-/Gefäßimplantate, Kreislaufunterstützungssysteme (ventrikulare Assist Devices, künstliche Herzen, intraaortale Ballonpumpen), Vena-cavainferior-Filter, Embolisierungsgeräte, endovaskulare Implantate, implantierbare Defibrillatoren und Cardioverter, Stents, Gefäßprothesen, arteriovenöser Nebenschluss (Shunt), Blutmonitore, interne Katheter zur Medikamentenverabreichung, Schrittmacherkabel, intravaskulare Membranoxygenatoren (künstliche Lungen), Leukozyten-Removal-Filter, und Herzklappen mit Blut in Berührung.

Gemäß der ISO 10993-4 (DIN EN ISO 10993: Biologische Beurteilung von Medizinprodukten, Deutsches Institut für Normung, 2003) müssen für Medizinprodukte, welche in Kontakt mit Blut stehen mindestens ein, häufig aber auch mehrere Tests aus nachfolgender Liste durchgeführt werden: Thrombose, Gerinnung, Thrombozyten, Hämatologie, Komplementsystem.

Meistens wurden und werden folgende einfache Tests durchgeführt:
Thrombose:
   - Thrombozytenadhäsion und -aggregation,
   - Anhaftende Thrombozyten, Fibrin, Morphologie der Thrombozyten (Lichtmikroskopie, Rasterelektronenmikroskopie)
      Koagulation:
         - nicht aktivierte partielle Tromboplastinzeit, Thrombozyten
         - Thrombingeneration (TAT)
         - D-Dimer
      Thrombozyten: Thrombozytenzahl (Differenzblutbild)
         - Thrombozytenadhäsion
         - Plättchenfaktor 4 (PF4)
Hämatologie:
   - Leukozytenzahl (Differenzblutbild)
   - Hämolyse im direkten Blutkontakt
   - Hämolyse im indirekten Blutkontakt
      Komplementsystem:
      - Komplementfaktor C5a .

Diese Methoden messen zwar einige Auswirkungen, welche ein Material haben kann, liefern aber, da sie den Kern der Ursache für die Hämoinkompatibilität nicht erfassen, wenig Anhaltspunkte zur gezielten Auswahl und Optimierung von Biomaterialien.

Die geeigneten Materialien für Medizinprodukte zu finden, welche komplett hämokompatibel sind, stellt daher noch ein großes zu lösendes Problem dar 144

Es besteht zum Wohle der Patienten die dringende Notwendigkeit der Optimierung der präklinischen Evaluierungsstrategien von blutkontaktierenden "Medical Devices". Die Problematik bei der Entwicklung von besser biokompatiblen Materialien besteht darin, dass bisher nur unvollständige Kenntnisse über den Pathway der Aktivierungsmechanismen vorhanden sind.

Um das Problem zu lösen, müssen Methoden entwickelt und Sensoren gefunden werden, mit denen die Hämokompatibilität einfach, schnell und zuverlässig getestet werden kann, weil sie direkt an der Ursache der Aktivierungsmechanismen ansetzt.

Die in dieser Erfindung offen gelegten Bindesubstanzen für missgefaltete Proteine, sind solche Sensoren.

Bezüglich der Förderung oder Verhinderung der Veränderung des Blutes, sodass entweder eine Blutungsneigung oder eine Thromboseneigung im Patienten ausgelöst oder unterstützt wird, spielen Proteinadsorption bzw Wechselwirkungen der Proteine untereinander und mit der Werkstoffoberfläche eine ganz entscheidende Rolle. Das Maß der Proteinadsorption und die Art der Wechselwirkungen werden zusätzlich von rheologischen Eigenschaften, wie Fließgeschwindigkeit, Hämatokrit, Grenzflächenübergänge und dem Auftreten von Scherkräften beeinflusst.

Die komplexen Vorgänge bei der Proteinadsorption und die Wechselwirkung mit dem Material verursachen die Aktivierung des Blutgerinnungsaktivierung und führen zur Aktivierung von FXII und beobachteten morphologischen Veränderungen der Thrombozyten¹⁴⁵.

Adsorption und Denaturierung von Proteinen auf Werkstoffen gilt daher als Maß für deren Biokompatibilität. Diese wird bisher auf umständliche Weise gemessen. So beschrieben Merritt et al. eine Mehrstufenanalyse, mit deren Hilfe die an Oberflächen von Prüfkörpern aus dem zu untersuchenden Werkstoff anhaftenden Proteine mit Hilfe von Enzyme-linked immunosorbent Assay (ELISA) und Photometrie quantifiziert und identifiziert werden können 146

Spezifische Proteine, welche an der Oberfläche des Werkstoffes haften, können mittels spezifischer Antikörper, welche an Nachweissystem gekoppelt sind, visualisiert werden. Auch die Ellipsometrie wird zur Beurteilung einer Proteinschicht auf Werkstoffoberflächen genutzt. In dieser optischen Messmethode wird linear polarisiertes Licht von der zu messenden Materialoberfläche reflektiert und kann dann durch einen Photodetektor gemessen werden. Zur Identifizierung von adsorbierten Proteinen können diese abgelöst und mittels SDS-PAGE und Western-Blot identifiziert werden. Adsorbierte Proteine können aber auch elektronenmikroskopisch analysiert werden.

Mit all diesen recht zeit-, arbeits-, und geräteaufwendigen Methoden wird aber eine der wichtigsten Parameter für die Bio/Hämokompatibilität des Materials nicht gemessen, die Fähigkeit des Materials Missfaltungen in adsorbierten Proteinen auszulösen.

Missgefaltete Proteine aktivieren, wie oben beschrieben, direkt über FXII das Kallikrein-System, beeinflussen über t-PA und Plasminogen die Fibrinolyse und aktivieren die Thrombozyten. Aktivierte Thrombozyten wiederum aktivieren Leukozyten und Lymphozyten. Missgefaltete Proteine/Peptide sind Adhäsionsbrücken für Mikroorganismen und missgefaltete Proteine/Peptide sind über ihre Zellrezeptoren unter anderem in so wichtige Prozesse wie Zellproliferation, Apoptose, Autophagie, Zellmigration, Immunabwehr beteiligt.

Die Messung, ob ein Material in adsorbierten Proteinen oder Peptiden Missfaltungen erzeugt, ist daher eine wichtige Methode zur Bestimmung der Bio- insbesondere der Hämokompatibilität.

Erfindungsgemäß kann die Untersuchung der Bio-, insbesondere Hämokompatibilität mit Hilfsmitteln erfolgen, welche an missgefaltete Proteine/Peptide binden. Dies kann eines der Proteine sein, welche von anderen als generelle Bindeproteine für missgefaltete Proteine/Peptide beschrieben worden ist, wie z.B. ein Chaperon, bevorzugt GRP78/BIP, oder tPA, oder Fibronektin, oder HGFA, ganz bevorzugt ist es aber eine Bindesubstanz, gemäß der hier offengelegten Erfindung, ADAM15 (Metargidin), die Metalloproteasedomäne von ADAM15 und insbesondere Peptide, welche die Sequenz His, Trp, Arg, Arg, Ala, His, Leu, Leu, Pro, Arg, Leu, Pro (HWRRAHLLPRLP) entsprechend der ADAM15 Sequenz 286-297, die Sequenz Glu, Asn, Phe, Leu, His, Trp, Arg, Arg, Ala, His, Leu, Leu (ENFLHWRRAHLL) ADAM 15 Sequenz 282-293, oder die Sequenz Ala, Val, Thr, Leu, Glu, Asn, Phe, Leu, His, Trp, Arg, Arg ADAM15 Sequenz 279-290, His, Trp, Arg, Arg, Pro oder His, Trp, Arg, Arg (HWRR) haben.

Das zu untersuchende Material zum Beispiel in Form von Probekörpern, Probeplättchen, Probekügelchen oder das zu untersuchende Medizinprodukt, wird dabei mit einer Protein-/Peptidhaltigen Lösung, bevorzugt einer Körperflüssigkeit eines Tieres oder Menschen, besonders bevorzugt mit Blut oder Plasma in Kontakt gebracht. Erfindungsgemäß ist es sinnvoll dies sowohl unter statischen als im nächsten Versuch auch unter dynamischen Bedingungen zu tun.

Das nichtadsorbierte Material wird mit Hilfe von geeigneten Flüssigkeiten wie, aber nicht beschränkt auf, physiologische Kochsalzlösung, physiologische Pufferlösungen vorsichtig abgewaschen. Eine Bindesubstanz, welche missgefaltete Proteine/Peptide erkennt, direkt oder indirekt an ein Nachweissystem, bindet an das missgefaltete Protein/Peptid und detektiert und misst missgefaltete Proteine auf der zu untersuchenden Werkstoffoberfläche.

Missgefaltetes Protein kann dann zum Beispiel fluorimetrisch (Durchflusszytometrie auf Beads, Fluoreszenz in Plattensystemen), fluoreszenzmikroskopisch, elektronenmikroskopisch, enzymatisch unter Nutzung von colorimetrischen Substraten oder Chemilumineszenz, turbidometrisch oder einem anderen beliebigen den Fachmann geläufigen Verfahren detektiert und gemessen werden.

### Nachweisreagenz und Methode zur Untersuchung des Einflusses eines Medizinproduktes auf die Erzeugung von Missfaltungen in Proteinen/Peptiden

Beschrieben werden ein Nachweisreagenz und eine Methode zur Untersuchung des Einflusses eines Medizinproduktes, bevorzugt eines extrakorporalen Systems, welches mit zirkulierendem Blut in Kontakt kommt oder eines Implantates oder Implantatteils , ganz besonders bevorzugt eines extra- oder intrakorporalen Unterstützungssystems, Herzklappen, Gefäßprothesen oder Stents auf die Erzeugung von Protein/Peptid Missfaltungen in Proteinen/Peptiden in Flüssigkeiten. Bevorzugt sind diese Flüssigkeiten, Körperflüssigkeiten von Menschen oder Tieren, ganz besonders bevorzugt handelt es sich dabei um Blut oder Plasma von Menschen oder Tieren.

Da auch viele andere Eigenschaften wie, aber nicht beschränkt auf Strömungseigenschaften, Scherstress, Grenzflächenübergänge, Rauigkeit des Werkstoffes eines Medizinproduktes das Entstehen von Missfaltungen in Proteinen/Peptiden verursachen können (siehe unter "Biopharmazeutika"), ist es notwendig, den Einfluss solcher Eigenschaften auf die Entstehung von missgefalteten Proteinen/Peptiden zu messen.

Die vorliegende Erfindung beschreibt Bindesubstanzen für missgefaltete Proteine/Peptide, zur Verwendung in Methoden zur Untersuchung des Einflusses von Medizinprodukten auf Proteine/Peptide, und Methoden zur Untersuchung des Einflusses von Medizinprodukten auf Proteine/Peptide und zur Optimierung von Medizinprodukten.

Beschrieben ist eine Substanz zur Untersuchung des Einflusses eines Medizinproduktes auf die Erzeugung von Missfaltungen in Proteinen/Peptiden eine Bindesubstanz für missgefaltete Proteine/Peptide, bevorzugt ein Bindesubstanz wie ADAM15 (Metargidin), die Metalloproteasedomäne von ADAM15 und insbesondere Peptide, welche die Sequenz His, Trp, Arg, Arg, Ala, His, Leu, Leu, Pro, Arg, Leu, Pro (HWRRAHLLPRLP) entsprechend der ADAM15 Sequenz 286-297, die Sequenz Glu, Asn, Phe, Leu, His, Trp, Arg, Arg, Ala, His, Leu, Leu (ENFLHWRRAHLL) ADAM 15 Sequenz 282-293, oder die Sequenz Ala, Val, Thr, Leu, Glu, Asn, Phe, Leu, His, Trp, Arg, Arg ADAM15 Sequenz 279-290, His, Trp, Arg, Arg, Pro oder His, Trp, Arg, Arg (HWRR).

Flüssigkeiten, welche Proteine/Peptide enthalten, bevorzugt proteinhaltige Körperflüssigkeiten von Menschen oder Tieren, ganz besonders bevorzugt Blut oder Plasma, fließen durch ein Medizinprodukt, oder werden durch das Medizinprodukt gepumpt. Besonders bevorzugt sind dabei rheologische Bedingungen und Fließgeschwindingkeiten, wie sie beim Fließen von Blut durch einen menschlichen oder tierischen Körper vorkommen. Vor Einlass der protein-/peptidhaltigen Flüssigkeit und nach Zirkulation durch das Medizinprodukt werden Proben der Flüssigkeit entnommen. Analog wie oben unter Detektion von missgefalteten Proteinen in Körperflüssigkeiten zur Diagnostik beschrieben, wird in den Proben vor und nach Durchlauf durch das Medizinprodukt der Gehalt an missgefalteten Proteinen/Peptiden detektiert und gemessen.

Zur Optimierung eines Medizinproduktes werden Modelle des Medizinproduktes auf ihre Wirkung hinsichtlich Missfaltung von Proteinen/Peptiden charakterisiert und verglichen und dem Herstellungsvorgang bzw. Modell der Vorzug zur Weiterentwicklung gegeben, welcher ein Produkt erzeugt, das geringere Konzentrationen an missgefalteten Proteinen/Peptiden aus der gleichen Proteinlösung, bevorzugt Blut oder Plasma, erzeugt.

Eine ganz besonders bevorzugte Anwendung der vorliegenden Erfindung ist die oben beschriebene in vitro Untersuchung unter Verwendung von tierischem oder menschlichem Blut in Kombination mit einer oder mehrerer Untersuchungen zur Wirkung eines Medizinproduktes auf den Aktivierungszustand von Thrombozyten, die funktionelle Kapazität von Thrombozyten, Aktivierung der Blutgerinnung, Fibrinolyse, Kallikreinsystem, Komplementsystem aus den gleichen oder parallel gewonnenen Untersuchungsproben. Hierbei sind besonders bevorzugt Quantifizierungen von Chaperonen, bevorzugt GRP78/BIP, auf der Thrombozytenoberfläche, Präsentation von CD62 und/oder CD63 auf der Thrombozytenoberfläche, Aufnahme und Einlagerung von Mepacrin in Thrombozyten, Bindung eines Reagenz zur Erkennung von aktiviertem GPIIbIIIa an Thrombozyten, Messung und/oder Quantifizierung von Mikropartikeln aus Thrombozyten, Leukozyten und Lymphozyten, Messung und/oder Quantifizierung von Assoziaten aus Thrombozyten mit neutrophilen Granulozyten oder mit Monozyten, Messung lösliche Spaltprodukte von Thrombozyten, wir P-Selektin, GPIb-Fragment, GPV-Fragment, PAR-1 Spaltprodukt, PAR-4 Spaltprodukt, Quantifizierung von Oberflächenproteinen auf Thrombozyten, Messung von Aktivierungsmarker für Thrombozyten nach gezielter Aktivierung mit Agonisten, Messung eine Formveränderung der Thrombozyten, Messung der Fähigkeit der Thrombozyten zu adhärieren, Messung der Fähigkeit der Thrombozyten ohne und nach gezielter Aktivierung Fibrinogen zu binden, Abnahme der Zahl der Thrombozyten, Leukozyten, Erythrozyten, nach Passage durch das Medizinprodukt, Messung der Hämolyse, Messung der Fähigkeit der Thrombozyten ohne und/oder nach gezielter Aktivierung von Willebrand Faktor zu binden, Konzentration und des Multimerenmusters des vWF, Aktivierungsmarker für Leukozyten, Messung der Fähigkeit der Leukozyten durch eine Zellschicht zu transmigrieren, Messung von Substanzen, wie, aber nicht beschränkt auf, Cytokine, Wachstumsfaktoren, Proteine, ATP, auf Blutzellen, Messung von Aktivierungsmarkern der Gerinnung, wie, aber nicht beschränkt auf , Thrombin-Antithrombinkomplex, Fibrin-Monomere F1+2, Messung von Markern der Aktivierung der Fibrinolyse wie, aber nicht beschränkt auf, D-Dimer, Plasmin-Antiplasminkomplex, Messung der Aktivierung des FXII-Kallikrein-Pathways, Messung von Aktivierungsmarkern des Komplementsystems, wie aber nicht beschränkt auf C3a, C5a, Bb, iC3b, C4b, SC5b-9, CH50, C3-Konvertase, C5-Konvertase.

### 6. Nachweisreagenz und Methode zur Detektion von missgefalteten Proteinen/Peptiden zur Nutzung dieser Substanzen

Amyloide sind für viele technische Anwendungen ein attraktives Baumaterial, da sie zu den wichtigsten sich selbstorganisierenden Nanomaterialien gehören. Sie haben eine simple repetitive geordnete Struktur, ein robustes Design und sind ein dauerhaftes und preisgünstiges Material. Amyloide sind aussichtsreiche Kandidaten für die Herstellung molekularer Nanobiomaterialien, wie z. B. Drähte, Schichten, Gele, Gerüste, Matrizen und Flüssigkristalle^{147, 148}. Beispiele für Nanomaterial aus missgefalteten Proteinen/Peptiden sind zum Beispiel Nanoröhrenleiter^{149, 150}. Solche Leiter können z.B. aus missgefalteten n-terminalen und mittleren Abschnitten des Proteins SU36p der Bierhefe Saccharomyces cerevisiae hergestellt werden. Missgefaltete monomere oder oligomere Proteine /Peptide sind die Vorstufen der Amyloide. Eine andere Anwendung der vorliegenden Erfindung ist die Nutzung einer der beschriebenen Bindesubstanzen um missgefaltete Proteine/Peptide und damit mögliche geeignete Baustoffe für amyloide Fasern in Biomaterialien effizient und gezielt zu suchen und zu finden. Neben seinen technischen Anwendungen, können missgefaltete Proteine/Peptide auch anderweitig sinnvoll genutzt werden, wie, aber nicht beschränkt auf, als Adjuvans bei der Herstellung von Antikörpern und Impfstoffen (Patent: Amyloid proteins as vaccine scaffolds. Inventors: Amy S Rosenberg (FDA), James E Keller (FDA), Robert Tycko (NIDDK.))

Die Bindesubstanzen können daher auch genutzt werden, um missgefaltete Proteine/Peptide für nützliche Anwendungen gezielt und effizient in Biomaterialien zu suchen und zu finden.

### 7. Anreicherung von Mikroorganismem, welche missgefaltete Proteine auf ihrer Oberfläche tragen, zur Erleichterung der Diagnostik

Viele Mikroorganismen, möglicherweise sogar alle, welche an einen Wirt anhaften, tragen missgefaltete Proteine/Peptide auf ihrer Oberfläche¹⁵¹. Sind Mikroorganismen nur vereinzelt in Blutbeutel, Präparaten zur parenteralen Anwendung oder in Körperflüssigkeiten, wie Blut, Liquor, Synovialflüssigkeit und anderen von Mensch und Tier, so erschwert das die Diagnostik sehr. Die vorliegende Erfindung erlaubt es Mikroorganismen aus einer größeren Materialprobe aufzukonzentrieren und damit die Diagnostik zu erleichtern. Dazu wird eines der Bindesubstanzen für missgefaltete Proteine/Peptide auf einer festen Oberfläche , wie zum Beispiel Kügelchen (Beads), Trägermaterial wie Filter, Membran, immobilisiert und die Körperflüssigkeit damit in Kontakt gebracht. Die Mikroorganismen binden an die Festphase. Nach abtrennen von Festphase und Körperflüssigkeit, sind die Mikroorganismen auf der Festphase angereichert und können daher dort leichter nachgewiesen werden.

### Figuren

Figur 1 zeigt das Ergebnis der Messung der optischen Dichte bei 492 nm für die Anbindung von HOCI-modifiziertem Albumin und von HOCI-modifiziertem ATIII an das Peptid PRKLYDY aus Beispiel 3a).
Figur 2 zeigt das Ergebnis der Messung der optischen Dichte bei 492 nm für die Anbindung von EAP an das Peptid PRKLYDY aus Beispiel 3d).
Figur 3 zeigt das Ergebnis der Messung der optischen Dichte bei 492 nm für die Anbindung von SCN⁻-IgG bzw. von Urea-IgG an das Peptid PRKLYDY aus Beispiel 3e).
Figur 4 zeigt das Ergebnis der Messung der optischen Dichte bei 492 nm für die Anbindung von RFYVVMWK an das Peptid PRKLYDY aus Beispiel 3g).
Figur 5 zeigt das Ergebnis der Messung der optischen Dichte bei 492 nm für die Anbindung von solublem P-Selektin an das Peptid LYDY von Beispiel 3m).
Figur 6 zeigt das Ergebnis der Messung der optischen Dichte für die Anbindung von GPV-Fragment bzw. von GPIb aus Thrombozytenaktivierungsüberstand an das Peptid PRKLYDY von Beispiel 3m).
Figur 7 zeigt das Ergebnis der Messung der optischen Dichte bei 492 nm für die Anbindung von HOCI-modifiziertem ATIII an das Peptid PRKLYDY von Beispiel 3o).
Figur 8 zeigt das Ergebnis der Messung der optischen Dichte bei 492 nm für die Anbindung von HOCI-modifiziertem Albumin bzw. von HOCI-modifiziertem ATIII an das Peptid HWRRAHLLPRLP von Beispiel 4a).
Figur 9 zeigt das Ergebnis der Messung der optischen Dichte bei 492 nm für die Anbindung von EAP an das Peptid HWRRAHLLPRLP von Beispiel 4d).
Figur 10 zeigt das Ergebnis der Messung der optischen Dichte bei 492 nm für die Anbindung von Harnstoff-verändertem IgG bzw. SCN⁻-verändertem IgG an das Peptid HWRRAHLLPRLP von Beispiel 4e).
Figur 11 zeigt das Ergebnis der Messung der optischen Dichte für die Anbindung von RFYVVMWK an das Peptid HWRRAHLLPRLP von Beispiel 4g).
Figur 12 zeigt das Ergebnis der Messung der optischen Dichte bei 492 nm für die Anbindung von GVP-Fragment bzw. von GPIb aus Thrombozytenaktivierungsüberstand an das Peptid HWRRAHLLPRLP von Beispiel 4m).
Figur 13 zeigt das Ergebnis der Messung der optischen Dichte bei 492 nm für die Anbindung von HOCI-modifiziertem ATIII an das Peptid HWRRAHLLPRLP von Beispiel 4o).
Figur 14 zeigt das Ergebnis der Messung der optischen Dichte bei 492 nm für die Anbindung von anti-FDP-Lysin Antikörper an HOCI-modifiziertes ATIII gebunden an die Peptide PRKLYDY bzw. HWRRAHLLPRLP von Beispiel 5.
Figur 15 zeigt das Ergebnis der Messung der optischen Dichte bei 492 nm für verschiedene missgefaltete Proteine in Faktor VIII Produkten bei Anbindung an die Proteine PRKLYDY bzw. HWRRAHLLPRLP von Beispiel 10.

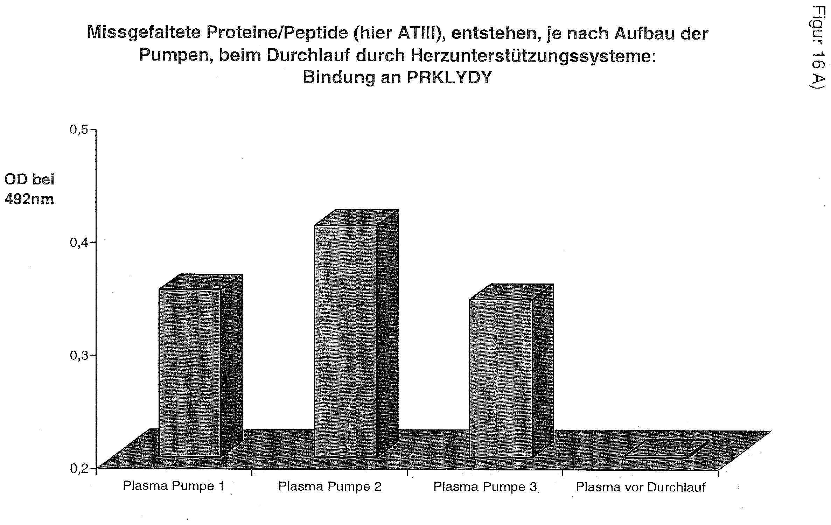

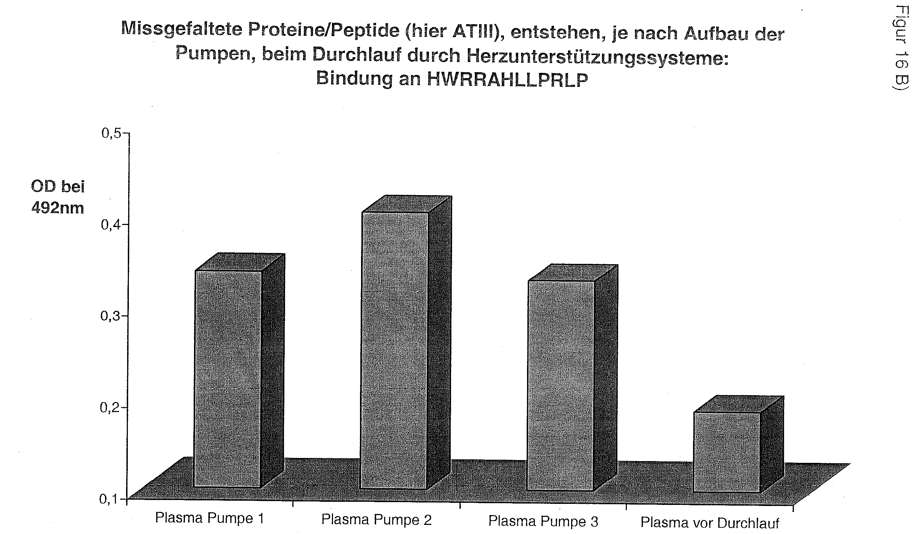

Figur 16 zeigt das Ergebnis der Messung der optischen Dichte bei 492 nm für verschiedene missgefaltete Proteine/Peptide, die je nach Aufbau der Pumpen beim Durchlauf durch Herzunterstützungssysteme entstehen. Die Messung wurde unter Anbindung an die Proteine PRKLYDY (nicht erfindungsgemäß) bzw. HWRRAHLLPRLP (erfindungsgemäß) durchgeführt.

### Beispiel

### 1. Synthese von Peptiden

Die Peptide PRKLYDY und HWRRAHLLPRLP wurden nach Standard-Festphasensynthese an einem automatischen Peptidsynthesizer synthetisiert. Es wurde hierbei die Strategie mit N-terminalen Fmoc-Schutzgruppen (Abspaltung der Schutzgruppe jeweils mit Piperidin nach jedem Kupplungsschritt) verwendet. Als Lösungsmittel wurde Dimethylformamid verwendet. Die Kupplungen erfolgten unter Zuhilfenahme von TBTU (Aktivator der Carboxylgruppen) und 4-Methyl-Morpholin (Deprotonierung der Aminogruppen).

Die Peptide wurden nach der letzten finalen Fmoc-Schutzgruppenabspaltung mit Trifluoressigsäure vom Harz entfernt und anschließend mittels präparativer HPLC gereinigt. Die Qualität wurde per MS kontrolliert.

### 2. Synthese von Biotin gekoppelten Peptiden gemäß Erfindung

a) biotinyliertes PRKLYDY: Das Peptid PRKLYDY wurde wie unter Beispiel 1 hergestellt. Anschließend wurde es nach der finalen Fmoc-Schutzgruppenabspaltung zunächst mit Fmoc-Aminohexansäure (als Spacer) umgesetzt. Nach der erneuten Fmoc-Abspaltung wurde es mit Biotin umgesetzt, danach ebenfalls mit Trifluoressigsäure vom Harz abgespalten und anschließend mittels präparativer HPLC gereinigt. Per MS wurde zuletzt die Qualität kontrolliert.
b) biotinyliertes HWRRAHLLPRLP: Das Peptid HWRRAHLLPRLP wurde wie unter Beispiel 1 hergestellt. Es wurde die N-terminale Fmoc-Schutzgruppe abgespalten und der N-terminus mit der Boc-Schutzgruppe geschützt. Dann wurde die DDE-Schutzgruppe am C-terminalen Lysin mit Hydrazin-Hydrat abgespalten. Anschließend wurde die epsilon-Aminogruppe des Lysins mit Biotin umgesetzt. Das Peptid wurde mit Trifluoressigsäure vom Harz abgespalten und mittels präparativer HPLC gereinigt. Per MS wurde zuletzt die Qualität kontrolliert.

### 3. Detektion und Messung von ganz unterschiedlichen missgefalteten Proteinen durch Biotin-PRKLYDY im Testplattenbindungsassay

### a) Detektion und Messung von missgefalteten HOCI-modifizierten Proteinen (hier als Unterbeispiele Albumin, Fibrinogen, Antithrombin, LDL)

Biotinyliertes PRKLYDY wurde für 1 h bei RT an eine vorgeblockte Strepatavidinplatte gecoatet. Nach einem Waschschritt wurde in unterschiedlichen Konzentrationen HOCI-modifiziertes Albumin, oder HOCImodifizierts Fibrinogen, oder HOCI-modifiziertes Antithrombin oder HOCI-modifiziertes LDL (inkl. Negativkontrolle) für 1 h bei RT zugegeben. Nach einem weiteren Waschschritt wurde bei HOCI-modifiziertem Albumin mouse anti HSA, bei HOCI-modifiziertem Fibrinogen rabbit anti Fibrinogen, bei HOCI-modifiziertem Antithrombin rabbit anti ATIII und bei HOCI-modifiziertem LDL mouse anti APO B Antikörper für 1h bei RT zugegeben. Nach einem erneuten Waschschritt wurde der entsprechende Sekundärantikörper, der mit Peroxidase gekoppelt war, für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen.

### b) Detektion und Messung von humanen alpha Defensinen (HNP1-4)

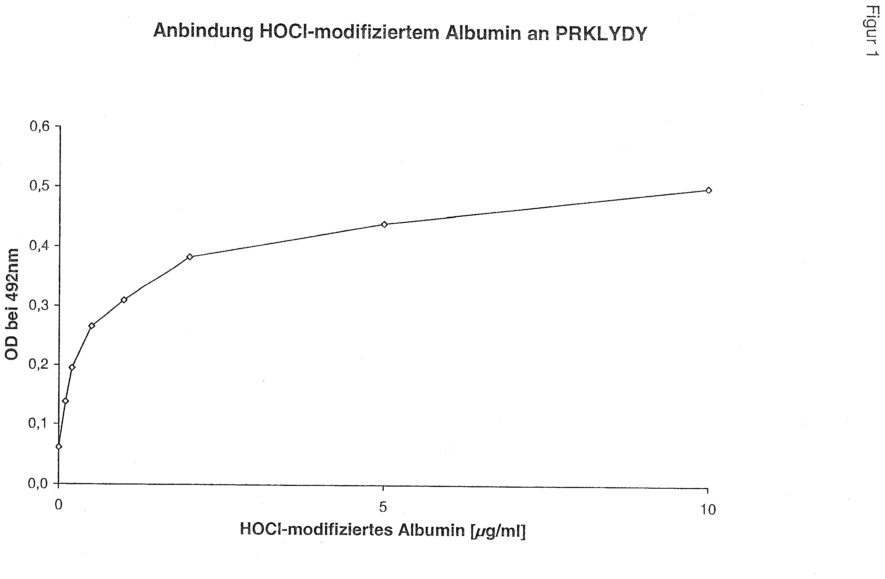

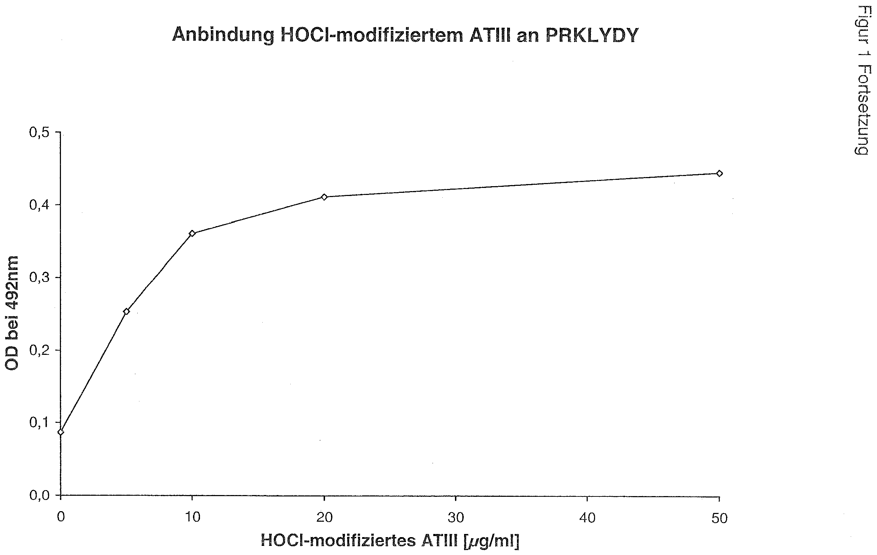

Biotinyliertes PRKLYDY wurde für 1 h bei RT an eine vorgeblockte Streptavidinplatte gecoatet. Nach einem Waschschritt wurde in unterschiedlichen Konzentrationen and Defensin (inkl. Negativkontrolle) für 1 h bei RT zugegeben. Nach einem weiteren Waschschritt wurde mouse anti Defensin für 1h bei RT zugegeben. Nach einem erneuten Waschschritt wurde der entsprechende Sekundärantikörper, anti Maus IgG POD für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen. Das Ergebnis ist in Figur 1 veranschaulicht.

### c) Detektion und Messung von AGE-Proteinen (hier als Unterbeispiel AGE-BSA, AGE-HB, AGE-HSA)

Biotinyliertes PRKLYDY wurde für 1 h bei RT an eine vorgeblockte Streptavidinplatte gecoatet. Nach einem Waschschritt wurde in unterschiedlichen Konzentrationen an AGE-BSA, oder AGE-HSA, oder AGE-HB (inkl. Negativkontrolle) für 1 h bei RT zugegeben. Nach einem weiteren Waschschritt wurde für AGE-BSA mouse anti BSA, für AGE-HSA mouse anti HSA und für AGE-HB chicken anti HB für 1h bei RT zugegeben. Nach einem erneuten Waschschritt wurde der entsprechende Sekundärantikörper, an den Peroxidase gekoppelt war, für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen.

### d) Detektion und Messung von missgefalteten Proteinen aus Mikroorganismen (hier als Unterbeispiel EAP von S.aureus)

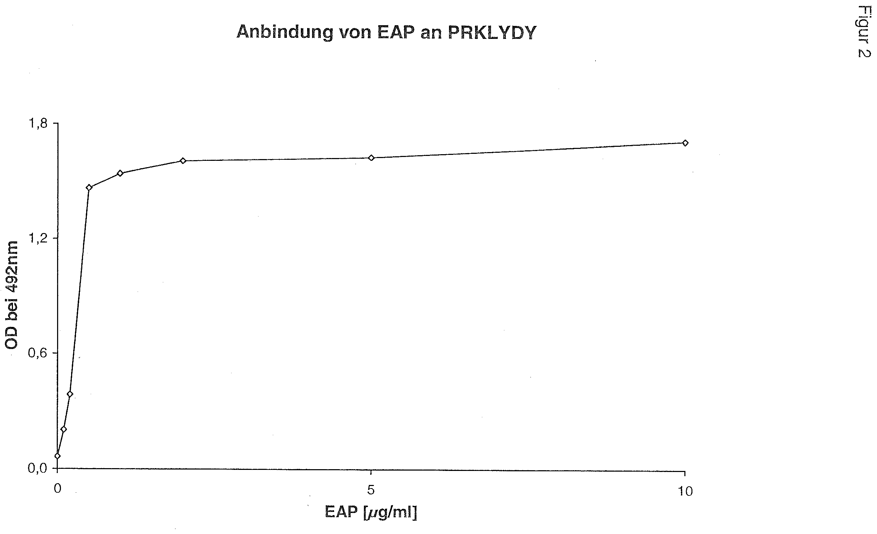

Biotinyliertes PRKLYDY wurde für 1 h bei RT an eine vorgeblockte Streptavidinplatte gecoatet. Nach einem Waschschritt wurde in unterschiedlichen Konzentrationen EAP (inkl. Negativkontrolle) für 1 h bei RT zugegeben. Nach einem weiteren Waschschritt wurde rabbit anti EAP für 1h bei RT zugegeben. Nach einem erneuten Waschschritt wurde der entsprechende Sekundärantikörper, anti rabbit IgG POD für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen (Fig. 2).

### e) Detektion und Messung von missgefalteten Immunglobulinen (hier als Unterbeispiel KSCN behandeltes IgG, Urea-behandeltes IgG, Hitze-Behandeltes IgG, durch mehrfaches Einfrieren und Auftauen behandeltes IgG, HOCI gehandeltes IgG)

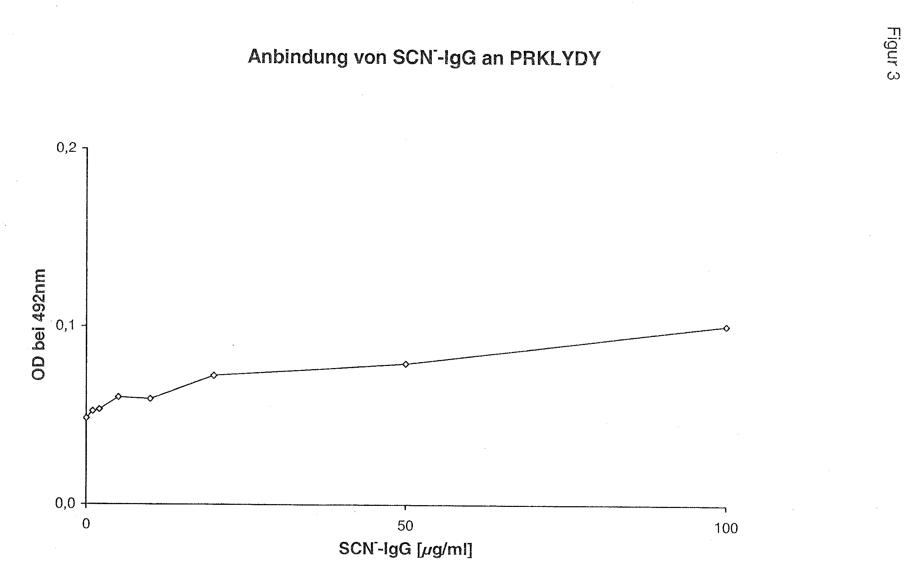

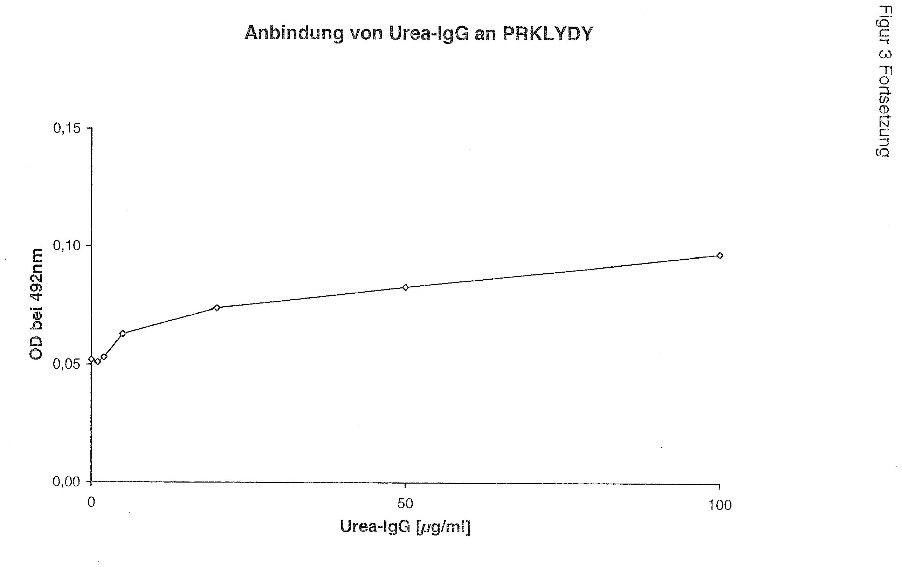

Biotinyliertes PRKLYDY wurde für 1 h bei RT an eine vorgeblockte Streptavidinplatte gecoatet. Nach einem Waschschritt wurde in unterschiedlichen Konzentrationen SCN⁻-behandeltes IgG, oder Harnstoffbehandeltes IgG, oder Hitze-behandeltes IgG, oder durch mehrfaches Einfrieren und Auftauen-behandeltes IgG, oder HOCI-behandeltes IgG (inkl. Negativkontrolle) für 1 h bei RT zugegeben. Nach einem weiteren Waschschritt wurde anti human IgG-POD Antikörper für 1h bei RT zugesetzt. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen (Figur 3).

### f) Detektion und Messung von Amyloid beta Peptid(1-42)

Biotinyliertes PRKLYDY wurde für 1 h bei RT an eine vorgeblockte Streptavidinplatte gecoatet. Nach einem Waschschritt wurde in unterschiedlichen Konzentrationen beta Amyloid Peptid (1-42) (inkl. Negativkontrolle) für 1 h bei RT zugegeben. Nach einem weiteren Waschschritt wurde mouse anti beta Amyloid Peptid Antikörper für 1h bei RT zugegeben. Nach einem erneuten Waschschritt wurde der entsprechende Sekundärantikörper, anti mouse IgG POD für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen.

### g) Detektion und Messung von missgefaltetem Peptid aus Thrombospondin-1

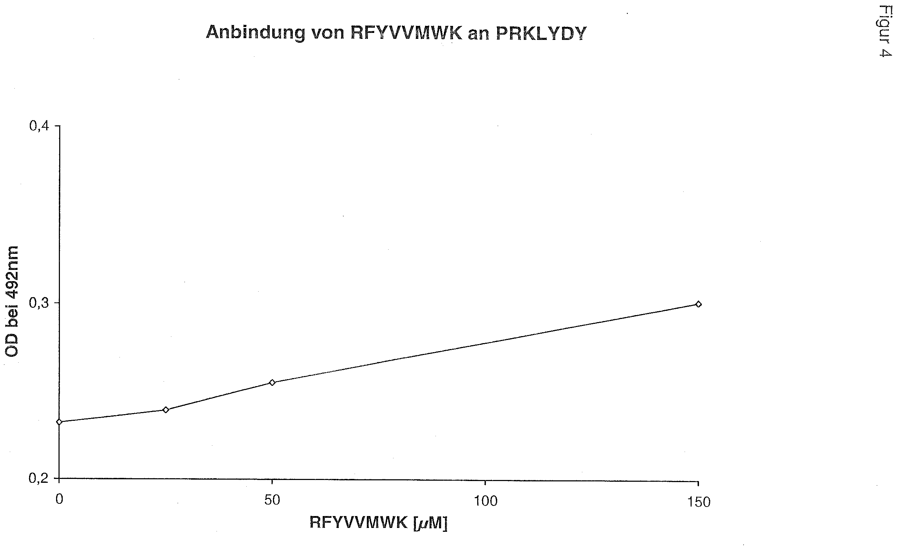

Biotinyliertes PRKLYDY wurde für 1 h bei RT an eine vorgeblockte Streptavidinplatte gecoatet. Nach einem Waschschritt wurde in unterschiedlichen Konzentrationen TSP-1-Peptid RFYVVMWK (inkl. Negativkontrolle) für 1 h bei RT zugegeben. Nach einem weiteren Waschschritt wurde rabbit anti TSP-1 Antikörper für 1h bei RT zugegeben. Nach einem erneuten Waschschritt wurde der entsprechende Sekundärantikörper, anti rabbit IgG POD für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen (Fig. 4).

### h) Detektion und Messung von Amylin

Biotinyliertes PRKLYDY wurde für 1 h bei RT an eine vorgeblockte Streptavidinplatte gecoatet. Nach einem Waschschritt wurde in unterschiedlichen Konzentrationen Amylin (inkl. Negativkontrolle) für 1 h bei RT zugegeben. Nach einem weiteren Waschschritt wurde human anti Amylin Antikörper für 1h bei RT zugegeben. Nach einem erneuten Waschschritt wurde der entsprechende Sekundärantikörper, anti human IgG POD für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen.

### i) Detektion und Messung von "scrambled" RNase

Biotinyliertes PRKLYDY wurde für 1 h bei RT an eine vorgeblockte Streptavidinplatte gecoatet. Nach einem Waschschritt wurde in unterschiedlichen Konzentrationen scrambled RNase (inkl. Negativkontrolle) für 1 h bei RT zugegeben. Nach einem weiteren Waschschritt wurde rabbit anti RNase Antikörper für 1h bei RT zugegeben. Nach einem erneuten Waschschritt wurde der entsprechende Sekundärantikörper, anti rabbit IgG POD für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen.

### j) Detektion und Messung von "aktiviertem" alpha2 Makroglobulin

alpha 2 Makroglobulin wurde auf zwei unterschiedliche Arten aktiviert.
A) Humanes alpha 2 Makroglobulin wurde 2 h bei 37°C mit 2µM Trypsin inkubiert und die Reaktion durch Zugabe 100µM (Endkonzentration) von p-Nitrophenyl-p'-guanidinobenzoat-HCl gestoppt.
B) Humanes alpha 2 Makroglobulin wurde über Nacht bei 37°C mit 200mM Ammoniumcarbonat inkubiert und anschließend gegen PBS-Puffer dialysiert. Die so "aktivierten" alpha 2 Makroglobuline wurden in unterschiedlichen Konzentrationen (inkl. Negativkontrolle) auf eine mit biotinyliertem PRKLYDY gecoateten, vorgeblockten Streptavidinplatte gegeben und für 1h bei RT inkubiert. Nach einem Waschschritt wurde rabbit anti alpha 2 Makroglobulin Antikörper für 1h bei RT zugegeben. Nach einem erneuten Waschschritt wurde der entsprechende Sekundärantikörper, anti rabbit IgG POD für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen.

### k) Detektion und Messung von sPla2 modifizierten LDL

Aus humanem Plasma isoliertes LDL wurde für 24 h bei 37°C mit sPLA2 inkubiert und die Reaktion durch Zugabe von 10mM EDTA. Auf diese Weise modifiziertes LDL wurde in unterschiedlichen Konzentrationen (inkl. Negativkontrolle) auf eine mit biotinyliertem PRKLYDY gecoateten, vorgeblockten Streptavidinplatte gegeben und für 1h bei RT inkubiert. Nach einem Waschschritt wurde mouse anti Apo B Antikörper für 1h bei RT zugegeben. Nach einem erneuten Waschschritt wurde der entsprechende Sekundärantikörper, anti mouse IgG POD für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen.

### l) Detektion und Messung von missgefaltetem Apo B100 Lipoprotein

Missgefaltetes Apo B 100 Lipoprotein wurde in unterschiedlichen Konzentrationen (inkl. Negativkontrolle) auf eine mit biotinyliertem PRKLYDY gecoateten, vorgeblockten Streptavidinplatte gegeben und für 1h bei RT inkubiert. Nach einem Waschschritt wurde mouse anti Apo B Antikörper für 1h bei RT zugegeben. Nach einem erneuten Waschschritt wurde der entsprechende Sekundärantikörper, anti mouse IgG POD für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen.

### m) Detektion und Messung von "gesheddeten" löslichen Membranproteinen (hier als Unterbeispiel sP-Selektin, sCD36, GPV-Fragment, GPVI-Fragment, GPIb-Fragment)

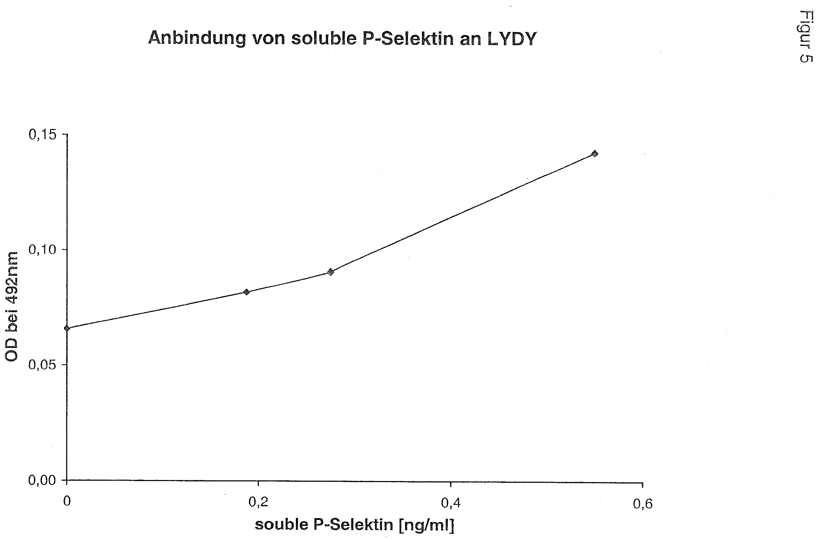

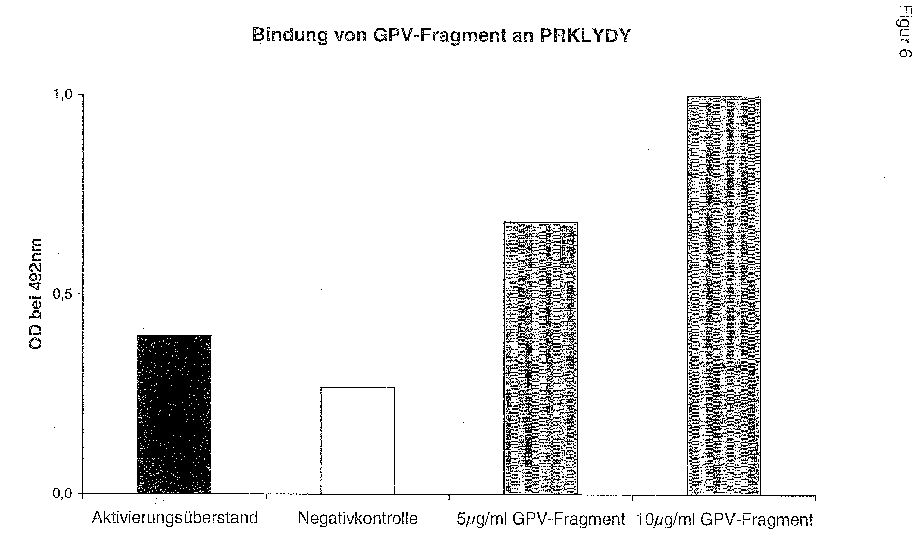

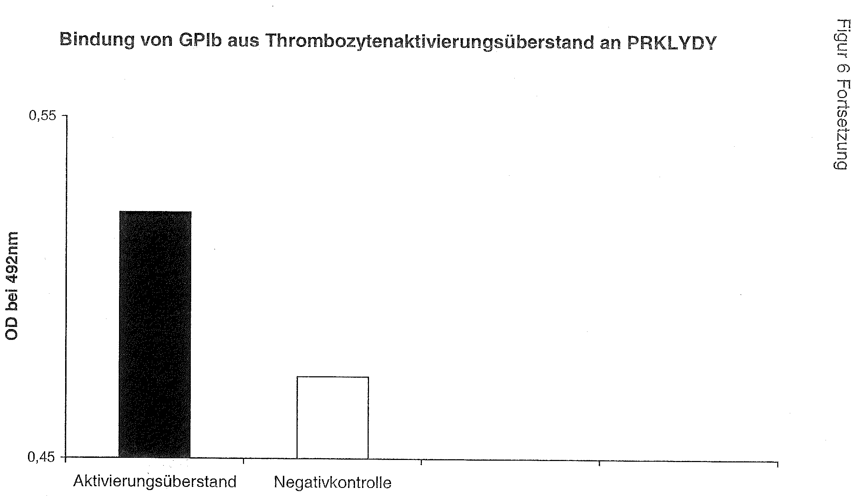

Lösliches P-Selektin, lösliches CD36, GPV-Fragment oder GPIb aus mit Kollagen und Thrombin aktivierten Thrombozyten (Aktivierungsüberstand) wurden in unterschiedlichen Konzentrationen (inkl. Negativkontrolle) auf eine mit biotinyliertem PRKLYDY gecoateten, vorgeblockten Streptavidinplatte gegeben und für 1h bei RT inkubiert. Nach einem Waschschritt wurde P-Selektin mit mouse anti CD62P, CD36 mit mouse anti CD36, GPV mit rabbit anti GPV, oder GPIb mit rabbit anti GPIb Antikörper für 1h bei RT inkubiert. Nach einem erneuten Waschschritt wurde der entsprechende Sekundärantikörper, an den Peroxidase gekoppelt war, für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492 nm gemessen (Fig. 5, 6).

### n) Detektion und Messung von missgefaltetem beta2-Mikroglobulin

Biotinyliertes PRKLYDY wurde für 1 h bei RT an eine vorgeblockte Streptavidinplatte gecoatet. Nach einem Waschschritt wurde in unterschiedlichen Konzentrationen missgefaltetes beta 2 Mikroglobulin (inkl. Negativkontrolle) für 1 h bei RT zugegeben. Nach einem weiteren Waschschritt wurde mouse anti beta 2 Mikroglobulin Antikörper für 1h bei RT zugegeben. Nach einem erneuten Waschschritt wurde der entsprechende Sekundärantikörper, anti mouse IgG POD für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen.

### o) Detektion und Messung von missgefalteten Serpinen (hier als Unterbeispiele Antithrombin III, alpha1 Antitrypsin)

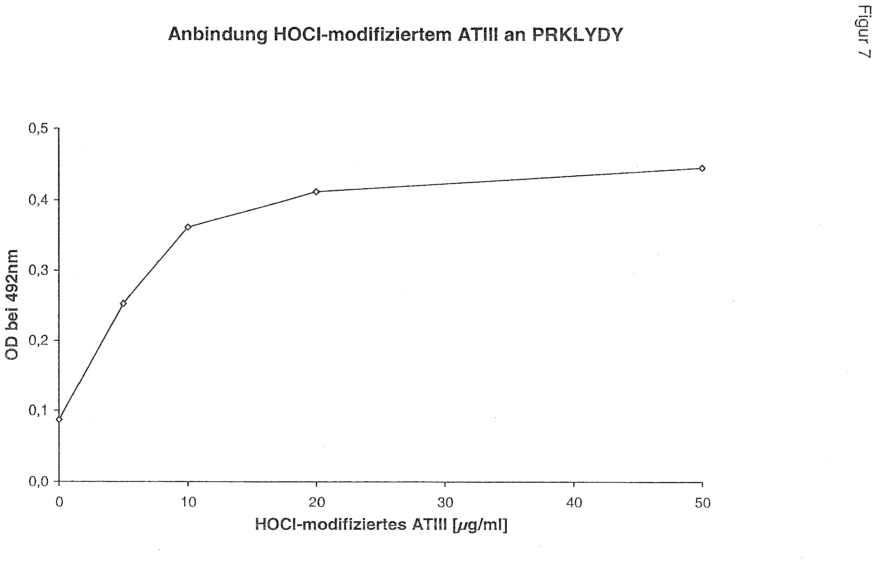

Biotinyliertes PRKLYDY wurde für 1 h bei RT an eine vorgeblockte Streptavidinplatte gecoatet. Nach einem Waschschritt wurde in unterschiedlichen Konzentrationen HOCI-modifiziertes ATIII, oder HOCI-modifiziertes alpha 1 Antitrypsin (inkl. Negativkontrolle) für 1 h bei RT zugegeben. Nach einem weiteren Waschschritt wurde missgefaltetes Antithrombin mit rabbit anti ATIII und missgefaltetes alpha 1 Antitrypsin mit rabbit anti alpha 1 Antitrypsin Antikörper für 1h bei RT zugegeben. Nach einem erneuten Waschschritt wurde der entsprechende Sekundärantikörper, anti rabbit IgG POD, für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492 nm gemessen (Fig. 7).

### 4. Detektion und Messung von ganz unterschiedlichen missgefalteten Proteinen durch HWRRAHLLPRLP- Biotin- im Testplattenbindungsassay

### a) Detektion und Messung von missgefalteten HOCI-modifizierten Proteinen (hier als Unterbeispiele Albumin, Fibrinogen, Antithrombin, LDL)

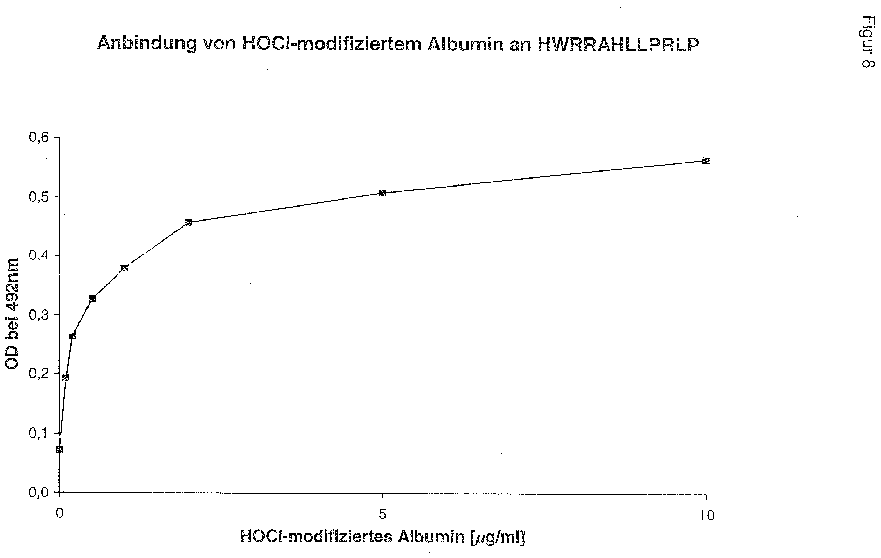

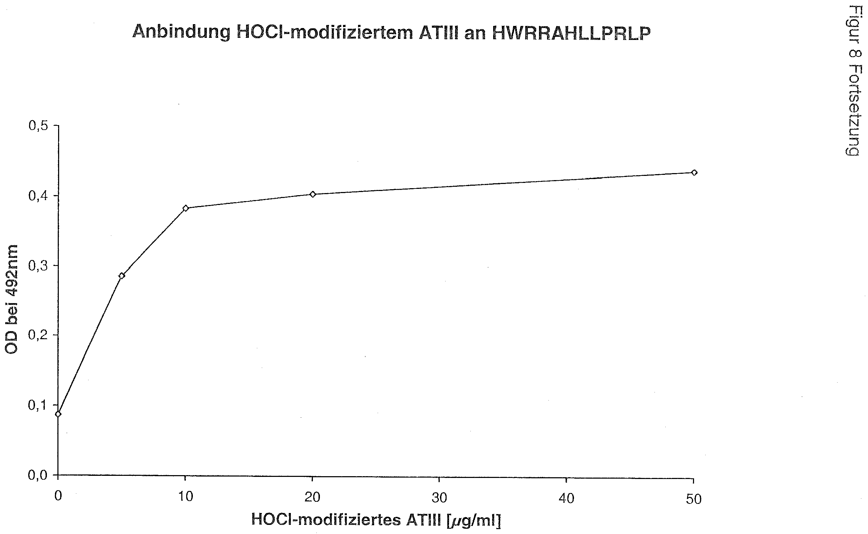

Biotinyliertes HWRRAHLLPRLP wurde für 1 h bei RT an eine vorgeblockte Streptavidinplatte gecoatet. Nach einem Waschschritt wurde in unterschiedlichen Konzentrationen HOCI-modifiziertes Albumin, oder HOCImodifizierts Fibrinogen, oder HOCI-modifiziertes Antithrombin oder HOCI-modifiziertes LDL (inkl. Negativkontrolle) für 1 h bei RT zugegeben. Nach einem weiteren Waschschritt wurde bei HOCI-modifiziertem Albumin mouse anti HSA, bei HOCI-modifiziertem Fibrinogen rabbit anti Fibrinogen, bei HOCI-modifiziertem Antithrombin rabbit anti ATIII und bei HOCI-modifiziertem LDL mouse anti APO B Antikörper für 1h bei RT zugegeben. Nach einem erneuten Waschschritt wurde der entsprechende Sekundärantikörper, der mit Peroxidase gekoppelt war, für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492 nm gemessen (Fig. 8).

### b) Detektion und Messung von humanen alpha Defensinen (HNP1-4)

Biotinyliertes HWRRAHLLPRLP wurde für 1 h bei RT an eine vorgeblockte Streptavidinplatte gecoatet. Nach einem Waschschritt wurde in unterschiedlichen Konzentrationen and Defensin (inkl. Negativkontrolle) für 1 h bei RT zugegeben. Nach einem weiteren Waschschritt wurde mouse anti Defensin für 1h bei RT zugegeben. Nach einem erneuten Waschschritt wurde der entsprechende Sekundärantikörper, anti Maus IgG POD für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen.

### c) Detektion und Messung von AGE-Proteinen (hier als Unterbeispiel AGE-BSA, AGE-HB, AGE-HSA)

Biotinyliertes HWRRAHLLPRLP wurde für 1 h bei RT an eine vorgeblockte Streptavidinplatte gecoatet. Nach einem Waschschritt wurde in unterschiedlichen Konzentrationen an AGE-BSA, oder AGE-HSA, oder AGE-HB (inkl. Negativkontrolle) für 1 h bei RT zugegeben. Nach einem weiteren Waschschritt wurde für AGE-BSA mouse anti BSA, für AGE-HSA mouse anti HSA und für AGE-HB chicken anti HB für 1h bei RT zugegeben. Nach einem erneuten Waschschritt wurde der entsprechende Sekundärantikörper, an den Peroxidase gekoppelt war, für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen.

### d) Detektion und Messung von missgefalteten Proteinen aus Mikroorganismen (hier als Unterbeispiel EAP von S.aureus)

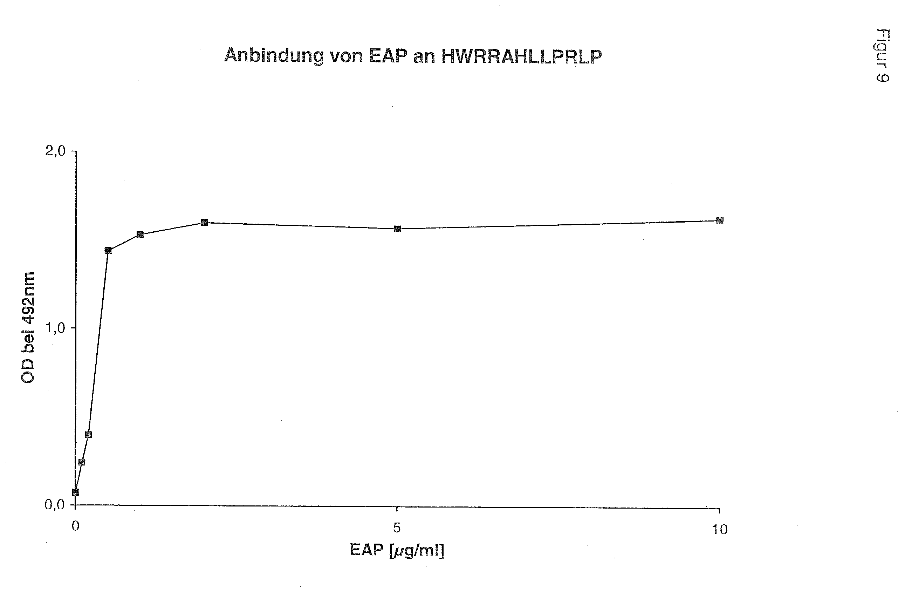

Biotinyliertes HWRRAHLLPRLP wurde für 1 h bei RT an eine vorgeblockte Streptavidinplatte gecoatet. Nach einem Waschschritt wurde in unterschiedlichen Konzentrationen EAP (inkl. Negativkontrolle) für 1 h bei RT zugegeben. Nach einem weiteren Waschschritt wurde rabbit anti EAP für 1h bei RT zugegeben. Nach einem erneuten Waschschritt wurde der entsprechende Sekundärantikörper, anti rabbit IgG POD für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen (Fig. 9).

### e) Detektion und Messung von missgefalteten Immunglobulinen (hier als Unterbeispiel KSCN behandeltes IgG, Urea-behandeltes IgG, Hitze-Behandeltes IgG, durch mehrfaches Einfrieren und Auftauen behandeltes IgG, HOCI gehandeltes IgG)

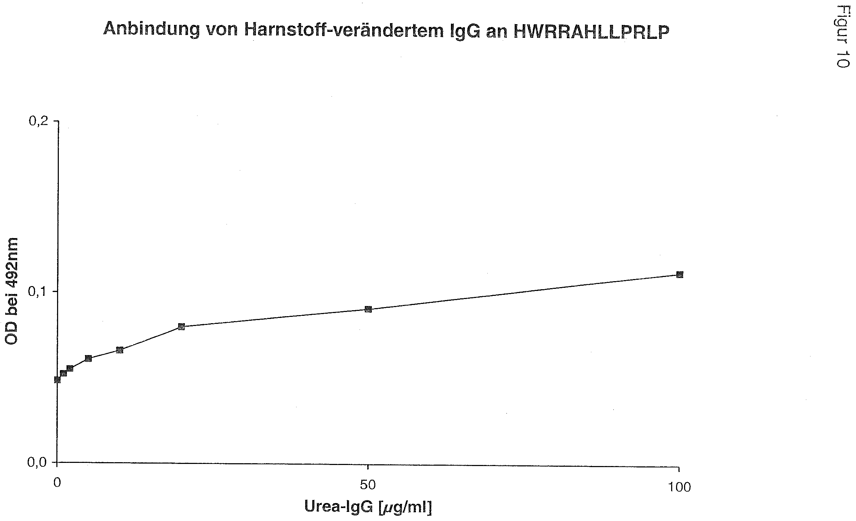

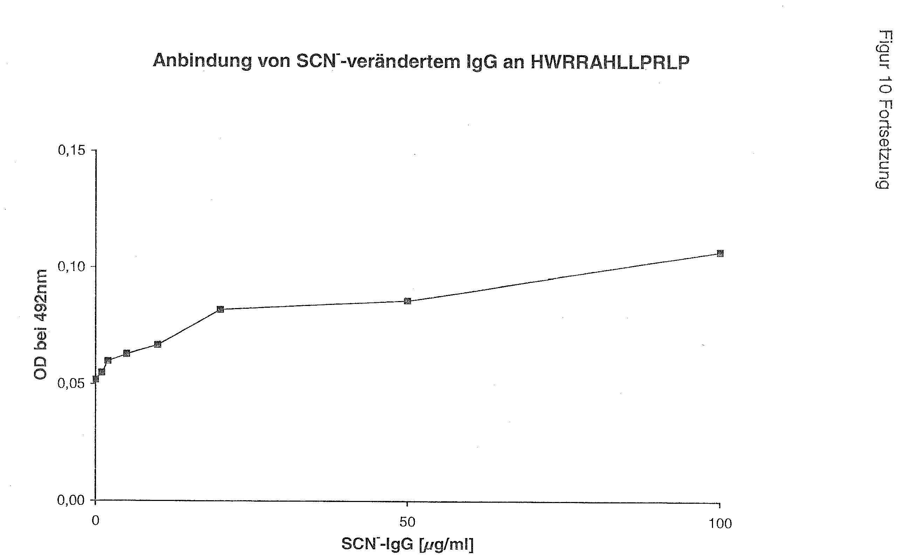

Biotinyliertes HWRRAHLLPRLP wurde für 1 h bei RT an eine vorgeblockte Streptavidinplatte gecoatet. Nach einem Waschschritt wurde in unterschiedlichen Konzentrationen SCN⁻-behandeltes IgG, oder Harnstoffbehandeltes IgG, oder Hitze-behandeltes IgG, oder durch mehrfaches Einfrieren und Auftauen-behandeltes IgG, oder HOCI-behandeltes IgG (inkl. Negativkontrolle) für 1 h bei RT zugegeben. Nach einem weiteren Waschschritt wurde anti human IgG-POD Antikörper für 1h bei RT zugesetzt. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen (Fig. 10).

### f) Detektion und Messung von Amyloid beta Peptid(1-42)

Biotinyliertes HWRRAHLLPRLP wurde für 1 h bei RT an eine vorgeblockte Streptavidinplatte gecoatet. Nach einem Waschschritt wurde in unterschiedlichen Konzentrationen beta Amyloid Peptid (1-42) (inkl. Negativkontrolle) für 1 h bei RT zugegeben. Nach einem weiteren Waschschritt wurde mouse anti beta Amyloid Peptid Antikörper für 1h bei RT zugegeben. Nach einem erneuten Waschschritt wurde der entsprechende Sekundärantikörper, anti mouse IgG POD für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen.

### g) Detektion und Messung von missgefaltetem Peptid aus Thrombospondin-1

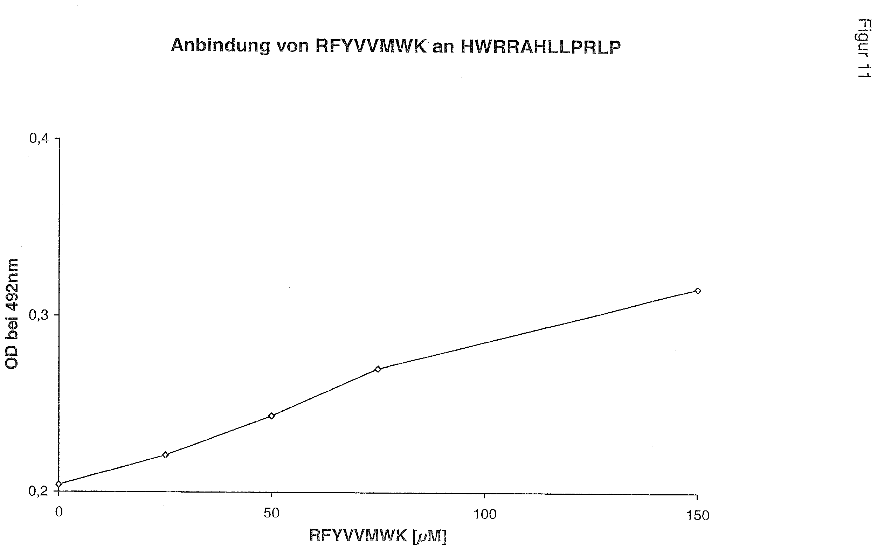

Biotinyliertes HWRRAHLLPRLP wurde für 1 h bei RT an eine vorgeblockte Streptavidinplatte gecoatet. Nach einem Waschschritt wurde in unterschiedlichen Konzentrationen TSP-1-Peptid RFYVVMWK (inkl. Negativkontrolle) für 1 h bei RT zugegeben. Nach einem weiteren Waschschritt wurde rabbit anti TSP-1 Antikörper für 1h bei RT zugegeben. Nach einem erneuten Waschschritt wurde der entsprechende Sekundärantikörper, anti rabbit IgG POD für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen (Fig. 11).

### h) Detektion und Messung von Amylin

Biotinyliertes HWRRAHLLPRLP wurde für 1 h bei RT an eine vorgeblockte Streptavidinplatte gecoatet. Nach einem Waschschritt wurde in unterschiedlichen Konzentrationen Amylin (inkl. Negativkontrolle) für 1 h bei RT zugegeben. Nach einem weiteren Waschschritt wurde human anti Amylin Antikörper für 1h bei RT zugegeben. Nach einem erneuten Waschschritt wurde der entsprechende Sekundärantikörper, anti human IgG POD für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen.

### i) Detektion und Messung von "scrambled" RNase

Biotinyliertes HWRRAHLLPRLP wurde für 1 h bei RT an eine vorgeblockte Streptavidinplatte gecoatet. Nach einem Waschschritt wurde in unterschiedlichen Konzentrationen scrambled RNase (inkl. Negativkontrolle) für 1 h bei RT zugegeben. Nach einem weiteren Waschschritt wurde rabbit anti RNase Antikörper für 1h bei RT zugegeben. Nach einem erneuten Waschschritt wurde der entsprechende Sekundärantikörper, anti rabbit IgG POD für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen.

### j) Detektion und Messung von "aktiviertem" alpha2 Makroglobulin

alpha 2 Makroglobulin wurde auf zwei unterschiedliche Arten aktiviert.
A) Humanes alpha 2 Makroglobulin wurde 2 h bei 37°C mit 2µM Trypsin inkubiert und die Reaktion durch Zugabe 100µM (Endkonzentration) von p-Nitrophenyl-p'-guanidinobenzoat-HCl gestoppt.
B) Humanes alpha 2 Makroglobulin wurde über Nacht bei 37°C mit 200mM Ammoniumcarbonat inkubiert und anschließend gegen PBS-Puffer dialysiert. Die so "aktivierten" alpha 2 Makroglobuline wurden in unterschiedlichen Konzentrationen (inkl. Negativkontrolle) auf eine mit biotinyliertem HWRRAHLLPRLP gecoateten, vorgeblockten Streptavidinplatte gegeben und für 1h bei RT inkubiert. Nach einem Waschschritt wurde rabbit anti alpha 2 Makroglobulin Antikörper für 1h bei RT zugegeben. Nach einem erneuten Waschschritt wurde der entsprechende Sekundärantikörper, anti rabbit IgG POD für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen.

### k) Detektion und Messung von sPla2 modifizierten LDL

Aus humanem Plasma isoliertes LDL wurde für 24 h bei 37°C mit sPLA2 inkubiert und die Reaktion durch Zugabe von 10mM EDTA. Auf diese Weise modifiziertes LDL wurde in unterschiedlichen Konzentrationen (inkl. Negativkontrolle) auf eine mit biotinyliertem HWRRAHLLPRLP gecoateten, vorgeblockten Streptavidinplatte gegeben und für 1h bei RT inkubiert. Nach einem Waschschritt wurde mouse anti Apo B Antikörper für 1h bei RT zugegeben. Nach einem erneuten Waschschritt wurde der entsprechende Sekundärantikörper, anti mouse IgG POD für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen.

### l) Detektion und Messung von missgefaltetem Apo B100 Lipoprotein

Missgefaltetes Apo B 100 Lipoprotein wurde in unterschiedlichen Konzentrationen (inkl. Negativkontrolle) auf eine mit biotinyliertem HWRRAHLLPRLP gecoateten, vorgeblockten Streptavidinplatte gegeben und für 1h bei RT inkubiert. Nach einem Waschschritt wurde mouse anti Apo B Antikörper für 1h bei RT zugegeben. Nach einem erneuten Waschschritt wurde der entsprechende Sekundärantikörper, anti mouse IgG POD für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen.

### m) Detektion und Messung von "gesheddeten" löslichen Membranproteinen (hier als Unterbeispiel sP-Selektin, sCD36, GPV-Fragment, GPVI-Fragment, GPIb-Fragment)

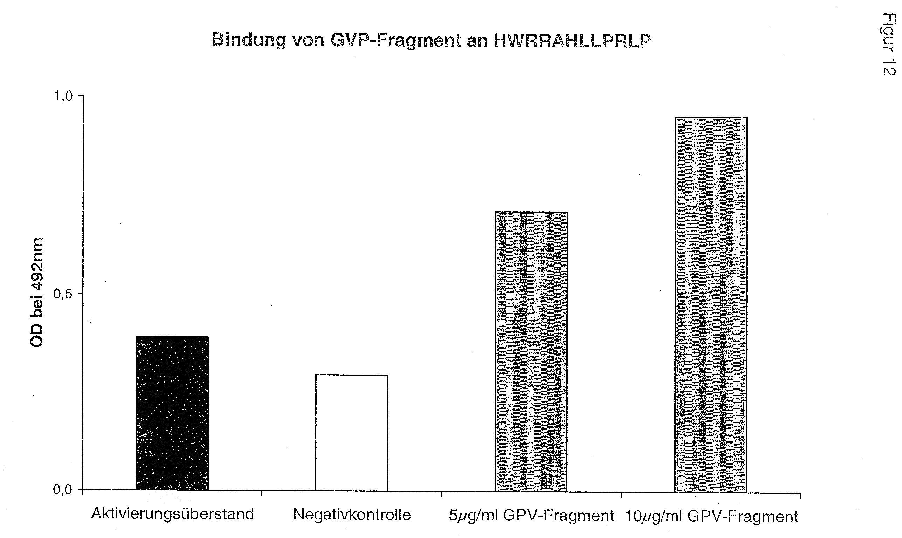

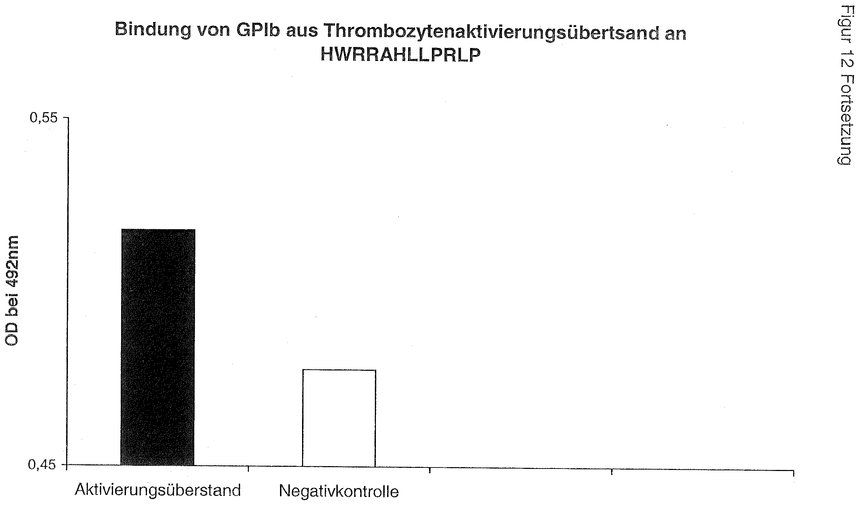

Lösliches P-Selektin, lösliches CD36, GPV-Fragment oder GPIb aus mit Kollagen und Thrombin aktivierten Thrombozyten (Aktivierungsüberstand) wurden in unterschiedlichen Konzentrationen (inkl. Negativkontrolle) auf eine mit biotinyliertem HWRRAHLLPRLP gecoateten, vorgeblockten Streptavidinplatte gegeben und für 1h bei RT inkubiert. Nach einem Waschschritt wurde P-Selektin mit mouse anti CD62P, CD36 mit mouse anti CD36, GPV mit rabbit anti GPV, oder GPIb mit rabbit anti GPIb Antikörper für 1h bei RT inkubiert. Nach einem erneuten Waschschritt wurde der entsprechende Sekundärantikörper, an den Peroxidase gekoppelt war, für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen (Fig. 12).

### n) Detektion und Messung von missgefaltetem beta2-Mikroglobulin

Biotinyliertes HWRRAHLLPRLP wurde für 1 h bei RT an eine vorgeblockte Streptavidinplatte gecoatet. Nach einem Waschschritt wurde in unterschiedlichen Konzentrationen missgefaltetes beta 2 Mikroglobulin (inkl. Negativkontrolle) für 1 h bei RT zugegeben. Nach einem weiteren Waschschritt wurde mouse anti beta 2 Mikroglobulin Antikörper für 1h bei RT zugegeben. Nach einem erneuten Waschschritt wurde der entsprechende Sekundärantikörper, anti mouse IgG POD für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen.

### o) Detektion und Messung von missgefalteten Serpinen (hier als Unterbeispiele Antithrombin III, alpha1 Antitrypsin

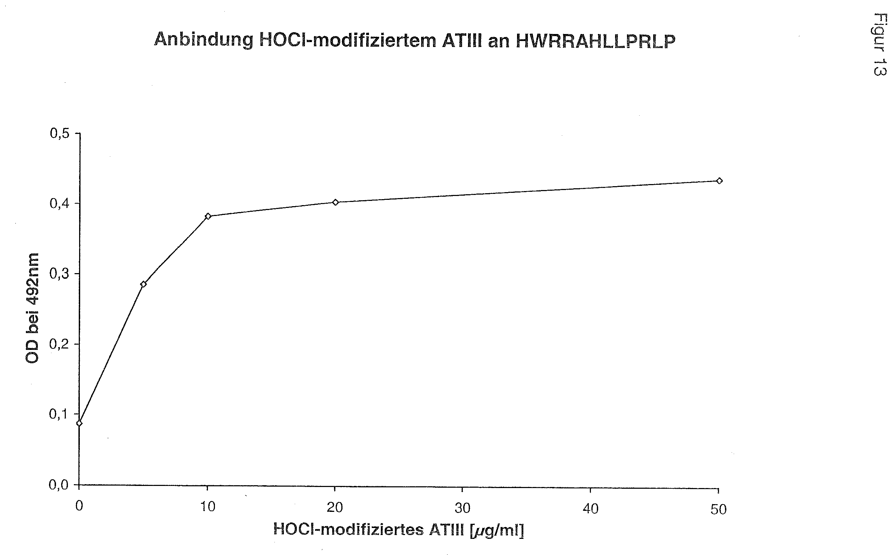

Biotinyliertes HWRRAHLLPRLP wurde für 1 h bei RT an eine vorgeblockte Streptavidinplatte gecoatet. Nach einem Waschschritt wurde in unterschiedlichen Konzentrationen HOCI-modifiziertes ATIII, oder HOCI-modifiziertes alpha 1 Antitrypsin (inkl. Negativkontrolle) für 1 h bei RT zugegeben. Nach einem weiteren Waschschritt wurde missgefaltetes Antithrombin mit rabbit anti ATIII und missgefaltetes alpha 1 Antitrypsin mit rabbit anti alpha 1 Antitrypsin Antikörper für 1h bei RT zugegeben. Nach einem erneuten Waschschritt wurde der entsprechende Sekundärantikörper, anti rabbit IgG POD, für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen (Fig. 13).

### 5. Detektion und Messung von missgefalteten Proteinen, welche FDP-Lysin-Reste tragen unter Nutzung von Biotin-PRKLYDY und HWRRAHLLPRLP-Biotin

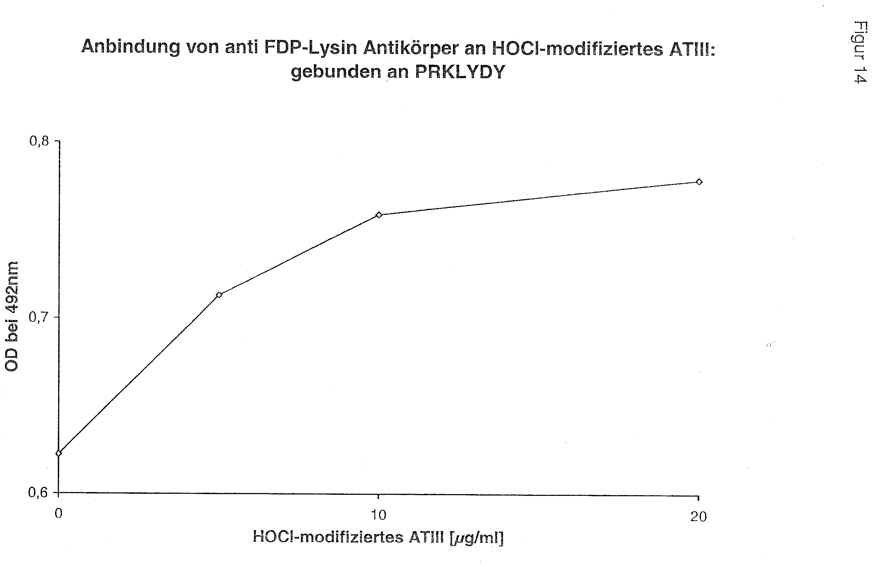

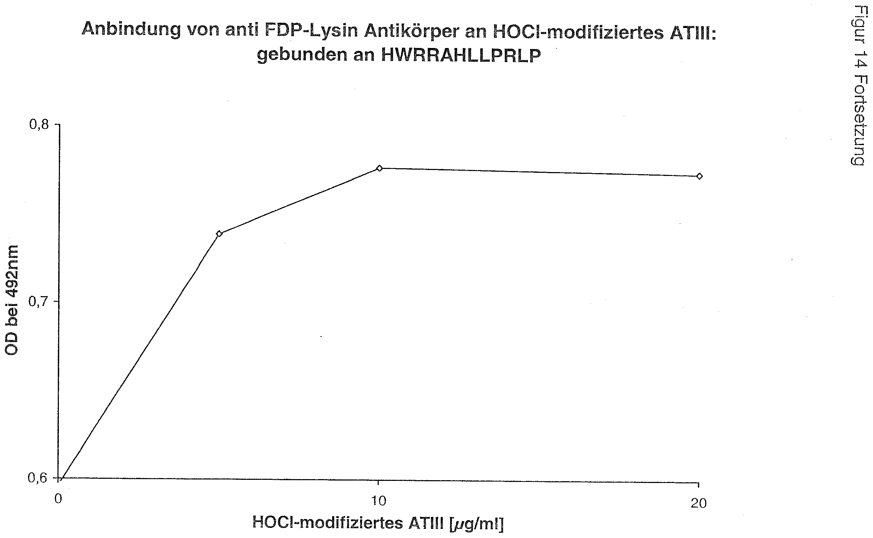

Biotinyliertes HWRRAHLLPRLP oder biotinyliertes PRKLYDY wurde für 1 h bei RT an eine vorgeblockte Streptavidinplatte gecoatet. Nach einem Waschschritt wurden in unterschiedlichen Konzentrationen missgefaltete Proteine (inkl. Negativkontrolle) für 1 h bei RT zugegeben (hier als Beispiel HOCI-modifiziertes Albumin und HOCI-modifiziertes Antithrombin). Nach einem weiteren Waschschritt wurde mouse anti FDP-Lysin Antikörper für 1h bei RT zugegeben. Nach einem erneuten Waschschritt wurde der entsprechende Sekundärantikörper, anti Maus IgG POD, für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen (Fig. 14).

### 6. Detektion und Messung von missgefalteten Proteinen im Komplexen mit alpha2-Makroglobulin, Clustrin, löslichem GRP78, Haptoglobin

Biotinyliertes HWRRAHLLPRLP oder biotinyliertes PRKLYDY wurde für 1 h bei RT an eine vorgeblockte Streptavidinplatte gecoatet. Faktor Vlla, oder Fibrinogen, oder HOCI-modifiziertes Albumin werden in Lösung mit alpha 2 Makroglobulin, oder Clustrin, oder GRP78, oder Haptoglobin, oder humanem Plasma inkubiert und anschließend in unterschiedlichen Konzentrationen (inkl. Negativkontrolle) für 1h bei RT auf die immobilisierten Peptide gegeben. Nach einem weiteren Waschschritt wurde entweder rabbit anti alpha 2 Makroglobulin, oder rat anti clusterin, oder rabbit anti GRP78/BiP, oder mouse anti Haptoglobin Antikörper für 1h bei RT zugegeben. Nach einem erneuten Waschschritt wurde der entsprechende Sekundärantikörper, an den Peroxidase gekoppelt war, für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen.

### 7. Detektion und Messung von missgefalteten Proteinen/Peptiden(total) durch Plasmon Resonanz

An einen Sensorchip, an dem Streptavidin kovalent an eine 2D Carboxymethyldextransulfat-Oberfläche gekoppelt war (SCB SAP), wurde in einem Biacore 1000 Gerät biotinyliertes PRKLYDY oder biotinyliertes HWRRAHLLPRLP in Boratpuffer gebunden. Über diese mit Peptid gekoppelten Oberflächen wurden mit einer Flussrate von 10µl/min unterschiedliche, missgefaltete Proteine gegeben und deren Anbindung durch Zunahme der Response Units sichtbar gemacht.

### 8. Detektion und Messung von missgefalteten Proteinen/Peptiden(total) in Körperflüssigkeiten durch Plasmon Resonanz

An einen Sensorchip, an dem Streptavidin kovalent an eine 2D Carboxymethyldextransulfat-Oberfläche gekoppelt war (SCB SAP), wurde in einem Biacore 1000 Gerät biotinyliertes PRKLYDY oder biotinyliertes HWRRAHLLPRLP in Boratpuffer gebunden. Über diese mit Peptid gekoppelten Oberflächen wurden mit einer Flussrate von 5µl/min unterschiedliche Körperflüssigkeiten gegeben und die Anbindung der missgefalteten Proteine in den Flüssigkeiten durch Zunahme der Response Units sichtbar gemacht.

### 9. Vergleich des Gehaltes von missgefalteten Proteinen im Plasma von gesunden Probanden und im Blut von Patienten (Beispiele: akuter Herzinfarkt, akuter Schlaganfall, schlecht eingestellte Diabetes, Alzheimer, Sepsis, DIC)

Aus Blut von Patienten (hier als Beispiel akuter Herzinfarkt, akuter Schlaganfall, schlecht eingestellter Diabetes, Alzheimer, Sepsis, DIC) wurde durch Zentrifugation frisches Plasma hergestellt. Parallel dazu wurde biotinyliertes HWRRAHLLPRLP oder biotinyliertes PRKLYDY für 1 h bei RT an eine vorgeblockte Strepatavidinplatte gecoatet.

Das frische Plasma der Patienten wurde mit den immobilisierten Peptiden für 1 h bei RT inkubiert. Nach einem Waschschritt wurde entweder rabbit anti Fibrinogen, oder rabbit anti humanalbumin, oder rabbit anti antithrombin für 1h bei RT zugegeben. Nach einem erneuten Waschschritt wurde der entsprechende Sekundärantikörper, an den Peroxidase gekoppelt war, für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen und die Menge an missgefaltetem Protein (hier untersucht: missgefaltetes Fibrinogen, missgefaltetes Humanalbumin, missgefaltetes Antithrombin) bestimmt.

### 10. Detektion und Messung von missgefalteten Proteinen in Protein-/Peptidhaltigen Medikamenten

Unterbeispiele :
a) missgefalteter FVIIa
b) missgefaltetes Fibrinogen
c) missgefalteter FVIII
d) missgefaltetes Insulin
e) missgefalteter C1 Inhibitor
f) missgefalteter GM-CSF
g) missgefaltete Proteine in Blutprodukten (Unterbeispiele: PPSB, FEIBA, gelagerte Plasmen, Faktorenkonzentrate, Immunglobulinkonzentrate, Humanalbumin)

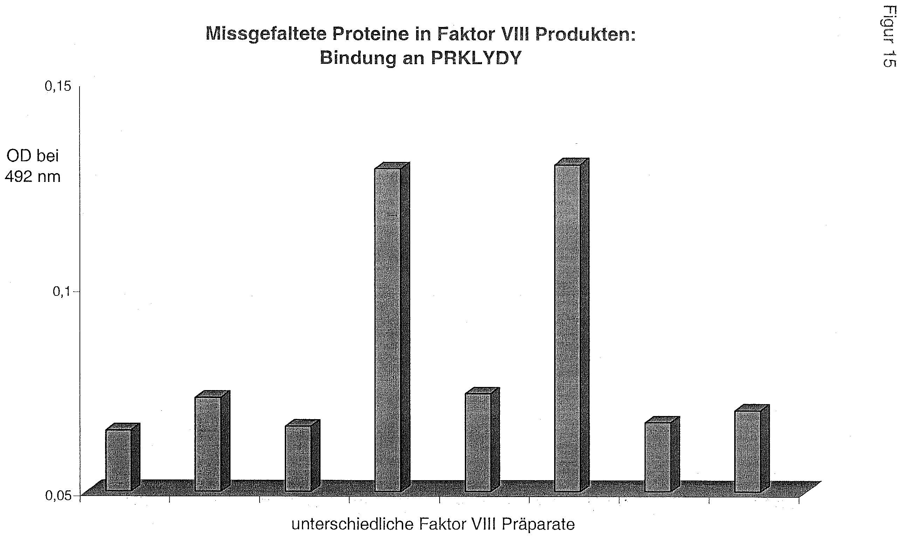

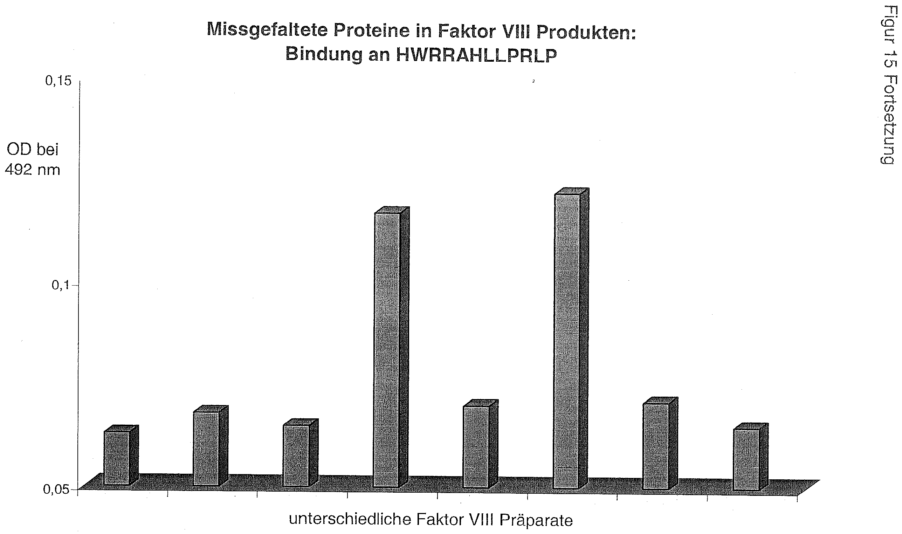

Biotinyliertes HWRRAHLLPRLP oder biotinyliertes PRKLYDY wurde für 1 h bei RT an eine vorgeblockte Streptavidinplatte gecoatet. Medikamente enthaltend a) Faktor Vlla, b) Fibrinogen, c) FVIII, d) Insulin, e) C1-Inhibitor, f) GM-CSF g) unterschiedliche Proteine aus Blutprodukten (hier untersucht PPSB-Konzentrate, Gerinnungsfaktorenkonzentrate, gelagerte Plasmen, Immunglobulinkonzentrate, Humanalbuminpräparate) wurden für 1 h bei RT mit den immobilisierten Peptiden inkubiert. Nach einem Waschschritt wurde im Faktor Vlla-Präparat anti FVIIa-Antikörper, im Fibrinogenpräparat anti Fibrinogen-Antikörper, in FVIII-Präparaten anti FVIII-Antikörper, in Insulinpräparaten anti Insulin-Antikörper, in C1-Inhibitor-Präparaten anti C1-Inhibitor-Antikörper, in GM-CSF anti GM-CSF-Antikörper und in Blutprodukten mit weiteren spezifischen Antikörpern für 1h bei RT zugegeben. Nach einem erneuten Waschschritt wurde der entsprechende Sekundärantikörper, an den Peroxidase gekoppelt war, für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen und die Menge an missgefalteten Proteinen in den Medikamenten bestimmt (Fig. 15).

Missgefaltete Proteine in Medikamenten/Blutprodukten wurden parallel in der Plasmon Resonanz untersucht. Dazu wurde an Sensorchips, an denen Streptavidin kovalent an eine 2D Carboxymethyldextransulfat-Oberfläche gekoppelt waren (SCB SAP), in einem Biacore 1000 Gerät biotinyliertes PRKLYDY oder biotinyliertes HWRRAHLLPRLP in Boratpuffer gebunden. Über diese mit Peptid gekoppelten Oberflächen wurden mit einer Flussrate von 5µl/min unterschiedliche Medikamente gegeben und die Anbindung der missgefalteten Proteine in den Medikamenten/Blutprodukten durch Zunahme der Response Units sichtbar gemacht.

### 11. Einfluss von Behandlungsbedingungen auf die Konzentration von missgefalteten Proteinen in Protein-/Peptidhaltigen Medikamenten

Beispiele: Fibrinogen und FVIIa
a) mehrmaliges Einfrieren Auftauen
b) Gegenwart von Ca⁺⁺
c) Einfluss von Thrombinspuren
d) Vortexen
e) Erhitzen auf 45 Grad über 24h

Fibrinogen und Faktor Vlla wurden a) 20 mal bei -80°C eingefroren und wieder aufgetaut, b) 2mM Ca²⁺ zugesetzt und 24 Stunden inkubiert, c) für 1h 0.05 U/ml alpha Thrombin zugesetzt, d) 2 Stunden gevortext oder e) für 24 Stunden auf 45°C erhitzt.

Diese unterschiedlich behandelten Proteine wurden auf biotinyliertes HWRRAHLLPRLP oder biotinyliertes PRKLYDY, welche zuvor für 1 h bei RT an eine vorgeblockte Strepatavidinplatte gecoatet wurden, gegeben. Nach einem Waschschritt wurde auf die Fibrinogen-Proben rabbit anti Fibrinogen und auf die Faktor Vlla-Proben sheep anti FVIIa für 1h bei RT gegeben. Nach einem erneuten Waschschritt wurde der entsprechende Sekundärantikörper, entweder anti rabbit IgG POD oder anti sheep IgG POD, für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen.

### 12. Detektion und Messung von missgefalteten Proteinen in Reagenzien, welche üblicherweise für die Diagnostik verwendet werden

### Unterbeispiele

a) Avidin, Streptavidin, Neutravidin
b) BSA
c) Magermilchpulver (Plasmon Resonanz)
d) sekundäre Antikörper

Missgefaltete Proteine in Avidin, Streptavidin, Neutravidin, oder in Rinderserumalbumin, oder Magermilchpulver, oder in sekundären Antikörpern wurden parallel in der Plasmon Resonanz untersucht. Dazu wurde an Sensorchips, an denen Streptavidin kovalent an eine 2D Carboxymethyldextransulfat-Oberfläche gekoppelt waren (SCB SAP), in einem Biacore 1000 Gerät biotinyliertes PRKLYDY oder biotinyliertes HWRRAHLLPRLP in Boratpuffer in sättigenden Konzentrationen gebunden. Alle noch freien Biotinbindungsstellen wurden abgedeckt. Über diese mit Peptid gekoppelten Oberflächen wurden mit einer Flussrate von 5µl/min die oben aufgeführten Substanzen gegeben und die Anbindung der missgefalteten Proteine in den Diagnostikreagentien durch Zunahme der Response Units sichtbar gemacht.

### 13. Detektion und Messung von missgefalteten Proteinen in Reagenzien, welche oft in der Forschung verwendet werden

a) Fetales Kälberserum (FCS), Serum neugeborener Kälber (NCS),
b) Humanserum
c) Proteinhaltiger/Peptidhaltiger Zellkulturmediumzusatz

Missgefaltete Proteine in fetalem Kälberserum, oder in Serum neugeborener Kälber, oder in Humanserum, oder in proteinhaltigen/peptidhaltigen Zellkulturmediumzusätzen wurden parallel in der Plasmon Resonanz untersucht. Dazu wurde an Sensorchips, an denen Streptavidin kovalent an eine 2D Carboxymethyldextransulfat-Oberfläche gekoppelt waren (SCB SAP), in einem Biacore 1000 Gerät biotinyliertes PRKLYDY oder biotinyliertes HWRRAHLLPRLP in Boratpuffer gebunden. Über diese mit Peptid gekoppelten Oberflächen wurden mit einer Flussrate von 5µl/min unterschiedliche Forschungsreagenzien gegeben und die Anbindung der missgefalteten Proteine in den Forschungsreagenzien durch Zunahme der Response Units sichtbar gemacht.

### 14. Detektion von missgefalteten Proteinen/Peptiden auf Biofilmen,

a) Undefinierte Biofilme abgerieben von Zähnen
b) Undefinierte Biofilme auf länger stehendem nährstoffhaltigem Wasser (Blumenwasser nach Entfernung der Blumen z.B.)
c) Biofilm von S. aureus

Die mit der Zeit entstandenen Biofilme wurden auf Folien übertragen, auf denen sie gut adhärierten. Zu den Biofilmen wurden entweder biotinyliertes PRKLYDY oder biotinyliertes HWRRAHLLPRLP gegeben und für 1 h bei RT inkubiert. Danach erfolgte ein gründlicher Waschschritt, und die Proben wurden auf zwei unterschiedliche Weisen analysiert:
1) Es wurde Avidin-Peroxidase für 1 h bei RT aufgegeben, anschließend gewaschen und Peroxidase-Substrat aufgegeben. Nach 30 minütiger Inkubation wurde die Reaktion mit 4N H₂SO₄ gestoppt, der gefärbte Überstand in eine ELISA-Platte übertragen und bei 492nm die optische Dichte gemessen. Mit Hilfe von Negativkontrollen und Standardreihen konnte die Menge an missgefalteten Proteinen/Peptiden bestimmt werden.
2) Es wurde Avidin-FITC für 1 h bei RT aufgegeben und nach einem Waschschritt im Fluoreszenzmikroskop untersucht.

### 15. Haemokompatibilität von Materialien

Vergleich Polystyrol, TiO2-Probekörper, mit Schutzsubstanz behandelte TiO2 Probekörper
a) Auf Blut eines gesunden Spenders, welches auf die unterschiedlichen Materialien (Probekörper) gegeben wurde, wurde 5 Minuten lang Scherstress ausgeübt (Cone-and-Plate-Aggregometer, 1700s⁻¹) (zur Kontrolle wurde ebenfalls Blut nur statisch mit den Oberflächen in Kontakt gebracht). Das Blut wurde vorsichtig abgenommen, die Probekörperchen gewaschen und mit biotinyliertem PRKLYDY, bzw. biotinyliertem HWRRAHLLPRLP inkubiert. Einem erneuten Waschschritt erfolgte die Inkubation mit Avidin-Peroxidase für 1 h bei RT. Nach einem weiteren Waschschritt wurde Peroxidase-Substrat aufgegeben und die Reaktion nach 30 Minuten mit 4N Schwefelsäure gestoppt. Der gefärbte Überstand wurde in eine ELISA-Platte übertragen und bei 492nm die optische Dichte gemessen. Mit Hilfe von Negativkontrollen und Standardreihen konnte die Menge an missgefalteten Proteinen/Peptiden bestimmt werden.
   Die Thrombozyten und die Leukozyten aus dem vorsichtig entfernten Blut wurden auf ihren Aktivierungszustand untersucht (Grundanbindung Fibrinogen, anti CD62P, anti CD63, Mikropartikel, Mikroaggregate, Mac-1 Expression und Assoziation untereinander), und das Plasma wurde nach Komplementfragmenten, Kallikrein und klassischen Gerinnungsparametern untersucht.
b) Eine Fibrinogenlösung wurde auf die Probekörperchen gegeben und für 5 Minuten bei 1700s⁻¹ geschert. Nach einem Waschschritt wurden diese mit biotinylertem PRKLYDY oder biotinyliertem HWRRAHLLPRLP für 1h bei RT inkubiert. Einem weiteren Waschschritt erfolgte die 1 stündige Inkubation mit Avidin-Peroxidase. Nach erneutem Waschen wurde Peroxidase-Substrat zugesetzt und die Reaktion nach 30 Minuten mit 4N Schwefelsäure gestoppt. Der gefärbte Überstand wurde in eine ELISA-Platte übertragen und bei 492nm die optische Dichte gemessen. Mit Hilfe von Negativkontrollen und Standardreihen konnte die Menge an missgefaltetem Fibrinogen bestimmt werden.

### 16. Optimierung von Medizingeräten

### Beispiel Herzunterstützungspumpen

Blut von gesunden Spendern wurde jeweils in zwei unterschiedliche Herzunterstützungssysteme gegeben und eine definierte Zeit lang durch die Kreisläufe gepumpt. Sowohl vor als auch 4 Stunden nach Durchlauf wurden 5ml Blut entnommen und durch Zentrifugation Plasma hergestellt.

Parallel dazu wurde biotinyliertes HWRRAHLLPRLP oder biotinyliertes PRKLYDY für 1 h bei RT an eine vorgeblockte Streptavidinplatte gecoatet.

Die frischen Plasmen wurden mit den immobilisierten Peptiden für 1 h bei RT inkubiert. Nach einem Waschschritt wurde entweder rabbit anti Fibrinogen, oder rabbit anti Humanalbumin, oder rabbit anti Antithrombin III für 1h bei RT zugegeben. Nach einem erneuten Waschschritt wurde der entsprechende Sekundärantikörper, an den Peroxidase gekoppelt war, für 1 h bei RT zugegeben. Nach einem letzten Waschschritt wurde mit o-Phenyldiamin-Substrat gefärbt und die Reaktion nach 30 Minuten mit 4N Schwefelsäure gestoppt. Es wurde die optische Dichte bei 492nm gemessen und die Menge an missgefalteten Proteinen (hier untersucht: missgefaltetes Fibrinogen, missgefaltetes Humanalbumin, missgefaltetes Antithrombin) bestimmt. Je weniger missgefaltete Proteine gefunden wurden, desto besser war das Herzunterstützungssystem konstruiert.

### 17. Bindung von Mikroorganismen an HWRRAHLLPRLP-Biotin und Biotin-PRKLYDY

Biotinyliertes HWRRAHLLPRLP oder biotinyliertes PRKLYDY wurde für 1 h bei RT an eine vorgeblockte Streptavidinplatte gecoatet. Nach einem Waschschritt wurden Bakterienkulturen, Bakterienmischungen, Staphylococcus aureus oder Candida albicans auf die immobilisierten Peptide gegeben (als Negativkontrolle wurden die Mikroorganismen auf Plastik gegeben, an dem keine Peptide gecoatet waren) und für 1h bei RT inkubiert. Nach einem gründlichen Waschschritt mit PBS-Puffer wurden die anhaftenden Zellen a) direkt und b) nach 1 Stunde angefärbt.

### 18. Bindung von missgefalteten Proteinen/Peptiden, die als Vorstufen für technisch nützlich Amyloide dienen

Biotinyliertes HWRRAHLLPRLP oder biotinyliertes PRKLYDY wurde für 1 h bei RT an eine vorgeblockte Streptavidinplatte gecoatet. Nach einem Waschschritt mit PBS-Puffer werden Lösungen aus Biomaterialien auf die immobilisierten Peptide gegeben, die potentiell missgefaltete Proteine/Peptide enthalten können. Als Beispiel haben wir die eine Lösung künstlich mit solchen potentiellen Werkstoffen, nämlich poly(ValGlyGlyLeuGly) oder poly(ValGlyGlyValGly) (beide werden heute schon im Sinne der Anmeldung, technisch genutzt) gespiked (versetzt). Die erfindungsgemäßen Peptide waren in der Lage, beide Werkstoffe zu binden, wie Surface Plasmon Resonanz zeigte.

Gebundene Peptide, Proteine oder Proteinfragmente können von solchen Chips oder anderem festen Material, auf dem unsere Nachweissubstanzen immobilisiert wurden, abgelöst und mittels Techniken der Proteomik, welche dem Fachmann gut bekannt sind, identifiziert werden.

### Referenzen

1. Stefani M. Generic cell dysfunction in neurodegenerative disorders: role of surfaces in early protein misfolding, aggregation, and aggregate cytotoxicity. Neuroscientist. 2007 Oct;13(5):519-31
2. Zhen Xu, Xi-Wei Liu, Yin-Sheng Ma and Hong-Wen Gao. Interaction of nano-TiO2 with lysozyme: insights into the enzyme toxicity of nanosized particles. Environ Sci Pollut Res Int. 2010 Mar;17(3):798-806. Epub 2009 Apr 24.
3. Onoue S, Ohshima K, Debari K, Koh K, Shioda S, Iwasa S, Kashimoto K, Yajima T. Mishandling of the therapeutic peptide glucagon generates cytotoxic amyloidogenic fibrils. Pharm. Res. 21:1274-1283
4. Bouma B, Kroon-Batenburg LM, Wu YP, Brünies B, Posthuma G, Kranenburg O, de Groot PG, Voest EE, Gebbink MF. Glycation induces formation of amyloid cross-beta structure in albumin. J Biol Chem 2003;278:41810-9
5. Maas C, Govers-Riemslag JW, Bouma B, Schiks B, Hazenberg BP, Lokhorst HM, Hammarström P, ten Cate H, de Groot PG, Bouma BN, Gebbink MF. Misfolded proteins activate Factor XII in humans, leading to kallikrein formation without initiating coagulation. J Clin Invest. 2008;118(9):3208-3218)
6. Rakhit R, Cunningham P, Furtos-Matei A, Dahan S, Qi XF, Crow JP, Cashman NR, Kondejewski LH, Chakrabartty A. Oxidation-induced Misfolding and Aggregation of Superoxide Dismutase and Its Implications for Amyotrophic Lateral Sclerosis, J Biol Chem 2002; 277 (49), 47551-47556
7. Marina Ramirez-Alvarado, Jeffery W. Kelly, Christopher M. Dobson, Johanna C. Scheinost,Daniel P. Witter, Grant E. Boldt, Paul Wentworth. Chapter 28. Role of Oxidative Stress in Protein Misfolding and/or Amyloid Formation. 2010
8. Hoffstrom BG, Kaplan A, Letso R, Schmid RS, Turmel GJ, Lo DC, Stockwell BR. Inhibitors of protein disulfide isomerase suppress apoptosis induced by misfolded proteins. Nature Chemical Biology 2010; 6,900-906
9. Park SY, Ferreira A. The generation of a 17 kDa neurotoxic fragment: an alternative mechanism by which tau mediates beta-amyloidinduced neurodegeneration. J Neurosci 2005;25:5365-75
10. Stewart N. Loh. The missing Zinc: p53 misfolding and cancer Metallomics, 2010, 2, 442-449
11. Byström R, Aisenbrey C, Borowik T, Bokvist M, Lindström F, Sani MA, Olofsson A, Gröbner G. Disordered proteins: biological membranes as two-dimensional aggregation matrices. Cell Biochem Biophys. 2008;52(3):175-89
12. Taylor DR, Hooper NM. Role of lipid rafts in the processing of the pathogenic prion and Alzheimer's amyloid-beta proteins. Semin Cell Dev Biol. 2007 Oct;18(5):638-48
13. Gebbink MF, Claessen D, Bouma B, Diikhuizen L, Wösten HA. Amyloids--a functional coat for microorganisms. Nat Rev Microbiol 2005; 3:333-41
14. Horn M, Bertling A, Brodde MF, Schulte A, Roth J, Van Aaken H, Jurk K, Stürzel C, Heilmann C, Peters G, Kehrel BE. Human neutrophil alpha defensins activate platelets. Zur Publikation eingereicht
15. Levinthal JD, Rubin H. Serum induced changes in the fine structure of primary chick embryo cultures. 1968 Exp Cell Res. 1968 Oct;52(2):667-72
16. Anfinsen, CB. Untersuchungen über die Ursachen der Faltung von Proteinketten (Nobel-Vortrag). Angewandte Chemie, 1973, 85: 1065-1074
17. Scott H. Protein-misfolding in neurodegenerative disease. The Internet Journal of Neurology. 2009 Volume 11 Number 2
18. Chelbi ST, Mondon F, Jammes H, Buffat C, Mignot TM, Tost J, Busato F, Gut I, Rebourcet R, Laissue P, Tsatsaris V, Goffinet F, Rigourd V, Carbonne B, Ferré F, Vaiman D. Expressional and Epigenetic Alterations of Placental Serine Protease Inhibitors SERPINA3 Is a Potential Marker of Preeclampsia. Hypertension. 2007;49:76
19. Maas C, Hermeling S, Bouma B, Jiskoot W, Gebbink MF. A role for protein misfolding in immunogenicity of biopharmaceuticals. J Biol Chem 2007; 282:2229-36
20. Brignull HR, Morley JF, Morimoto RI. The stress of misfolded proteins: C. elegans models for neurodegenerative disease and aging. Adv. Exp. Med. Biol. 2007; 594, 167-189
21. Maas C, Schiks B, Strangi RD, Hackeng TM, Bouma BN, Gebbink MF, Bouma B. Identification of fibronectin type I domains as amyloid-binding modules on tissue-type plasminogen activator and three homologs. Amyloid 2008; 15:166-80
22. Maas C, Govers-Riemslag JW, Bouma B, Schiks B, Hazenberg BP, Lokhorst HM, Hammarström P, ten Cate H, de Groot PG, Bouma BN, Gebbink MF. Misfolded proteins activate factor XII in humans, leading to kallikrein formation without initiating coagulation. J Clin Invest 2008; 118:3208-18
23. Gebbink MF, Bouma B, Maas C, Bouma BN. Physiological responses to protein aggregates: Fibrinolysis, coagulation and inflammation (new roles for old factors). FEBS Lett. 2009 Aug 20;583(16):2691-9
24. Kranenburg O, Bouma B, Kroon-Batenburg LM, Reijerkerk A, Wu YP, Voest EE, Gebbink MF. Tissue-type Plasminogen activator is a multiligand cross-beta structure receptor. Current Biology 2002; 12, 1833-1839
25.Mintz PJ, Kim J, Do KA, Wang X, Zinner RG, Cristofanilli M, Arap MA, Hong WK, Troncoso P, Logothetis CJ, Pasqualini R, Arap W. Fingerprinting the circulating repertoire of antibodies from cancer patients. Nat. Biotechnol 2003; 21: 57-63
26. Liu C, Bhattacharjee G, Boisvert W, Dilley R, Edgington T. In vivo interrogation of the molecular display of atherosclerotic lesion surfaces. Am J Pathol 2003; 163: 1859-71
27. Zhou J, Werstuck GH, Lhoták S, de Koning AB, Sood SK, Hossain GS, Møller J, Ritskes-Hoitinga M, Falk E, Dayal S, Lentz SR, Austin RC. Association of multiple cellular stress pathways with accelerated atherosclerosis in hyperhomocysteinemic apolipoprotein E-deficient mice. Circulation 2004; 110: 207-213
28. Feaver RE, Hastings NE, Pryor A, Blackman BR. GRP78 upregulation by atheroprone shear stress via p38-, alpha2beta1-dependent mechanism in endothelial cells. ATVB 2008; 28: 1534-41
29.Chen JC, Wu ML, Huang KC, Lin WW. HMG-CoA reductase inhibitors activate the unfolded protein response and induce cytoprotective GRP78 expression. Cardiovasc. Res. 2008; 80: 138-150
30. Watson LM, Chan AK, Berry LR, Li J, Sood SK, Dickhout JG, Xu L, Werstuck GH, Bajzar L, Klamut HJ, Austin RC. Overexpression of the 78-kda glucose-regulated protein/immunoglobulin-binding protein (GRP78/BIP) inhibits tissue factor procoagulant activity. J. Biol. Chem. 2003; 278:17438-17447
31. Bhattacharjee G, Ahamed J, Pedersen B, EI-Sheikh A, Mackman N, Ruf W, Liu C, Edgington TS. Regulation of tissue factor-mediated initiation of the coagulation cascade by cell surface grp78. ATVB 2005; 25:1737-1743
32. Molins B, Peña E, Padro T, Casani L, Mendieta C, Badimon L. Glucose-regulated protein 78 and platelet deposition. ATVB 2010; 30: 1246-1252
33. Bertling A, Niemann S, Hussain M, Brodde MF, Pohl S, Schifferdecker T, Roth J, Jurk K, Müller A, Peters G, Heilmann C, Kehrel BE. Staphylococcal extracellular adherence protein (Eap) induces platelet adhesion, agglutination, aggregation and procoagulant activity. zur Publikation eingereicht
34. Herczenik E, Bouma B, Korporaal SJ, Strangi R, Zeng Q, Gros P, Van Eck M, Van Berkel TJ, Gebbink MF, Akkerman JW. Activation of Human Platelets by Misfolded Proteins. Arterioscler Thromb Vasc Biol. 2007 Jul;27(7):1657-65
35. Bertling A, Brodde MF, Van Aken H , Müller A, Hussain M, Roth J, Peters G, Heilmann C, Kehrel BE. Staphylococcal extracellular adherence protein (Eap) acts as a misfolded protein and interacts with the cell surface chaperone BiP. zur Publikation eingereicht
36. Horn M, Bertling A, Brodde MF, Schulte A, Roth J, Stürzel C, Heilmann C, Peters G, Van Aken H, Jurk K, Kehrel BE. Human neutrophil alpha-defensins activate platelets. zur Publikation eingereicht
37. Marcinowski M, Höller M, Feige MJ, Baerend D, Lamb DC, Buchner J. Substrate discrimination of the chaperone BiP by autonomous and cochaperone-regulated conformational transitions. Nat Struct Mol Biol. 2011 Feb;18(2):150-8
38. Deng WG, Ruan KH, Du M, Saunders MA, Wu KK. Aspirin and salicylate bind to immunoglobulin heavy chain binding protein (BiP) and inhibit its ATPase activity in human fibroblasts. FASEB J. 2001 Nov;15(13):2463-70
39.Sugiyama S, Kugiyama K, Aikawa M, Nakamura S, Ogawa H, Libby P. Hypochlorous Acid, a Macrophage Product, Induces Endothelial Apoptosis and Tissue Factor Expression. Involvement of Myeloperoxidase-Mediated Oxidant in Plaque Erosion and Thrombogenesis. Arterioscler Thromb Vasc Biol. 2004; 1309-1314
40. Baldus S, Heeschen C, Meinertz T, Zeiher AM, Eiserich JP, Münzel T, Simoons ML, Hamm CW; CAPTURE Investigators. Circulation Myeloperoxidase Serum Levels Predict Risk in Patients With Acute Coronary Syndromes. Circulation. 2003 Sep 23;108(12):1440-5
41. Matthijsen RA, Huugen D, Hoebers NT, de Vries B, Peutz-Kootstra CJ, Aratani Y, Daha MR, Tervaert JW, Buurman WA, Heeringa P. Myeloperoxidase Is Critically Involved in the Induction of Organ Damage after Renal Ischemia Reperfusion Am. J. Pathol. 2007; 171(6): 1743 - 1752
42. Meuwese MC, Stroes ES, Hazen SL, van Miert JN, Kuivenhoven JA, Schaub RG, Wareham NJ, Luben R, Kastelein JJ, Khaw KT, Boekholdt SM. Serum Myeloperoxidase Levels Are Associated With the Future Risk of Coronary Artery Disease in Apparently Healthy Individuals: The EPIC-Norfolk Prospective Population Study. J. Am. Coll. Cardiol, 2007; 50(2): 159 - 165
43. Mocatta TJ, Pilbrow AP, Cameron VA, Senthilmohan R, Frampton CM, Richards AM, Winterbourn CC. Winterbourn Plasma Concentrations of Myeloperoxidase Predict Mortality After Myocardial Infarction. J. Am. Coll. Cardiol. 2007; 49(20): 1993 - 2000
44. Ferrante G, Nakano M, Prati F, Niccoli G, Mallus MT, Ramazzotti V, Montone RA, Kolodgie FD, Virmani R, Crea F. High Levels of Systemic Myeloperoxidase Are Associated With Coronary Plaque Erosion in Patients With Acute Coronary Syndromes: A Clinicopathological Study ; Circulation, 2010; 122(24): 2505 - 2513
45. Brennan ML, Reddy A, Tang WH, Wu Y, Brennan DM, Hsu A, Mann SA, Hammer PL, Hazen SL. Comprehensive Peroxidase-Based Hematologic Profiling for the Prediction of 1-Year Myocardial Infarction and Death Circulation. 2010; 122(1): 70-79
46. Naruko T, Furukawa A, Yunoki K, Komatsu R, Nakagawa M, Matsumura Y, Shirai N, Sugioka K, Takagi M, Hozumi T, Itoh A, Haze K, Yoshiyama M, Becker AE, Ueda M. Increased expression and plasma levels of myeloperoxidase are closely related to the presence of angiographically-detected complex lesion morphology in unstable angina Heart. 2010; 96(21): 1716 - 1722
47. Van der Zwan LP, Scheffer PG, Dekker JM, Stehouwer CD, Heine RJ, Teerlink T. Hyperglycemia and Oxidative Stress Strengthen the Association Between Myeloperoxidase and Blood Pressure. Hypertension. 2010; 55(6): 1366 - 1372
48. Giovannini S, Onder G, Leeuwenburgh C, Carter C, Marzetti E, Russo A, Capoluongo E, Pahor M, Bernabei R, Landi F. Myeloperoxidase Levels and Mortality in Frail Community-Living Elderly Individuals. J Gerontol A Biol Sci Med Sci. 2010; 65A(4): 369 - 376
49. Ali Z, Sarcia P, Mosley TH Jr, Kondragunta V, Kullo IJ. Association of serum myeloperoxidase with the ankle-brachial index and peripheral arterial disease Vascular Medicine. 2009; 14(3): 215 - 220
50. Liu HR, Tao L, Gao E, Qu Y, Lau WB, Lopez BL, Christopher TA, Koch W, Yue TL, Ma XL. Rosiglitazone inhibits hypercholesterolaemia-induced myeloperoxidase upregulation--a novel mechanism for the cardioprotective effects of PPAR agonists. Cardiovasc Res. 2009; 81(2): 344 - 352
51. Rudolph V, Andrie RP, Rudolph TK, Friedrichs K, Klinke A, Hirsch-Hoffmann B, Schwoerer AP, Lau D, Fu X, Klingel K, Sydow K, Didié M, Seniuk A, von Leitner EC, Szoecs K, Schrickel JW, Treede H, Wenzel U, Lewalter T, Nickenig G, Zimmermann WH, Meinertz T, Böger RH, Reichenspurner H, Freeman BA, Eschenhagen T, Ehmke H, Hazen SL, Willems S, Baldus S. Myeloperoxidase acts as a profibrotic mediator of atrial fibrillation Nature Medicine. 2010; 16, 470-474
52. Tang WH, Wu Y, Nicholls SJ, Hazen SL. Plasma Myeloperoxidase Predicts Incident Cardiovascular Risks in Stable Patients Undergoing Medical Management for Coronary Artery Disease. Clin. Chem. 2011; 57(1): 33 - 39
53. Davidson DJ, Haskell C, Maiest S, Kherzai A, Egan DA, Walter KA, Schneider A, Gubbins EF, Solomon L, Chen Z, Lesniewski R, Henkin J. Kringle 5 of Human Plasminogen Induces Apoptosis of Endothelial and Tumor Cells through Surface-Expressed Glucose-Regulated Protein 78. Cancer Res. 2005; 65(11): 4663-72
54. McFarland BC, Stewart J Jr, Hamza A, Nordal R, Davidson DJ, Henkin J, Gladson CL. Plasminogen kringle 5 induces apoptosis of brain microvessel endothelial cells: sensitization by radiation and requirement for GRP78 and LRP1. Cancer Res. 2009 Jul 1;69(13):5537-45
55. Raiter A, Weiss C, Bechor Z, Ben-Dor I, Battler A, Kaplan B, Hardy B. Activation of GRP78 on endothelial cell membranes by an ADAM15-derived peptide induces angiogenesis. J Vasc Res. 2010;47(5):399-411
56. Misra UK, Pizzo SV. Heterotrimeric Galphaq11 co-immunoprecipitates with surface-anchored GRP78 from plasma membranes of alpha2M*-stimulated macrophages. J cell Biochem. 2008; 104, 96-104
57. Lu MC, Lai NS, Yu HC, Huang HB, Hsieh SC, Yu CL. Anti-citrullinated protein antibodies bind surface-expressed citrullinated Grp78 on monocyte/macrophages and stimulate tumor necrosis factor α production. Arthritis Rheum. 2010 May;62(5):1213-23.
58. Reijerkerk A, Mosnier LO, Kranenburg O, Bouma BN, Carmeliet P, Drixler T, Meijers JC, Voest EE, Gebbink MF. Amyloid endostatin induces endothelial cell detachment by stimulation of the plasminogen activation system. Mol Cancer Res. 2003 Jun;1(8):561-8
59. Zhang Y, Liu R, Ni M, Gill P, Lee AS. Cell Surface Relocalization of the Endoplasmic Reticulum Chaperone and Unfolded Protein Response Regulator GRP78/BiP J. Biol. Chem. 2010; 285:15065-15075
60. Hotamisligil GS. Endoplasmic reticulum stress and atherosclerosis. Nature Medicine 2010; 16, 396-399
61.Myoishi M, Hao H, Minamino T, Watanabe K, Nishihira K, Hatakeyama K, Asada Y, Okada K, Ishibashi-Ueda H, Gabbiani G, Bochaton-Piallat M, Mochizuki N, Kitakaze M. Increased endoplasmic reticulum stress in atherosclerotic plaques associated with acute coronary syndrome. Circulation. 2007; 116: 1226-1233
62.Zhou J, Lhoták S, Hilditch BA, Austin RC. Activation of the unfolded protein response occurs at all stages of atherosclerotic lesion development in apolipoprotein E-deficient mice. Circulation. 2005; 111: 1814-1821,
63.Özcan U, Yilmaz E, Özcan L, Furuhashi M, Vaillancourt E, Smith RO, Görgün CZ, Hotamisligil GS. Chemical chaperones reduce ER stress and restore glucose homeostasis in a mouse model of type 2 diabetes. Science. 2006; 313: 1137-1140
64. Feaver RE, Hastings NE, Pryor A, Blackman BR. GRP78 upregulation by atheroprone shear stress via p38-, alpha2beta1-dependent mechanism in endothelial cells. Arterioscler Thromb Vasc Biol. 2008 Aug;28(8):1534-41
65.Ye R, Jung DY, Jun JY, Li J, Luo S, Ko HJ, Kim JK, Lee AS. Grp78 Heterozygosity Promotes Adaptive Unfolded Protein Response and Attenuates Diet-Induced Obesity and Insulin Resistance. Diabetes. 2010 Jan;59(1):6-16
66. Dickhout JG, Colgan SM, Lhoták S, Austin RC. Increased Endoplasmic Reticulum Stress in Atherosclerotic Plaques Associated With Acute Coronary Syndrome A Balancing Act Between Plaque Stability and Rupture. Circulation. 2007 Sep 11;116(11):1214-6
67.Wilson MR, Yerbury JJ, Poon S. Potential roles of abundant extracellular chaperones in the control of amyloid formation and toxicity. Mol. BioSyst. 2008; 4, 42-52
68. Naiki H, Nagai Y. Molecular Pathogenesis of Protein Misfolding Diseases: Pathological Molecular Environments Versus Quality Control Systems Against Misfolded Proteins. J Biochem. 2009; 146 (6): 751-756
69.Yerbury JJ, Rybchyn MS, Easterbrook-Smith SB, Henriques C, Wilson MR. The acute phase protein haptoglobin is a mammalian extracellular chaperone with an action similar to clusterin. Biochemistry 2005;44: 10914-10925
70.Yerbury JJ, Stewart EM, Wyatt AR, Wilson MR. Quality control of protein folding in extracellular space. EMBO Reports 2005;6: 1131-1136
71.Wilhelmus MM, de Waal RM, Verbeek MM. Heat Shock Proteins and Amateur Chaperones in Amyloid-Beta Accumulation and Clearance in Alzheimer's Disease. Mol Neurobiol. 2007 June; 35(3): 203-216
72.Strittmatter WJ, Saunders AM, Schmechel D, Pericak-Vance M, Enghild J, Salvesen GS, Roses AD. Apolipoprotein E: high-avidity binding to beta-amyloid and increased frequency of type 4 allele in late-onset familial Alzheimer disease. Proc Natl Acad Sci USA 1993; 90:1977-1981
73.Strittmatter WJ, Weisgraber KH, Huang DY, Dong LM, Salvesen GS, Pericak-Vance M, Schmechel D, Saunders AM, Goldgaber D, Roses AD. Binding of human apolipoprotein E to synthetic amyloid beta peptide: isoform-specific effects and implications for late-onset Alzheimer disease. Proc Natl Acad Sci USA 1993; 90:8098-8102
74.Casserly I, Topol E. Convergence of atherosclerosis and Alzheimer's disease: inflammation, cholesterol, and misfolded proteins, Lancet 2004; 363: 1139-46
75. Baldus S, Heeschen C, Meinertz T, Zeiher AM, Eiserich JP, Münzel T, Simoons ML, Hamm CW; CAPTURE Investigators. Myeloperoxidase Serum Levels Predict Risk in Patients With Acute Coronary Syndromes. Circulation. 2003 Sep 23;108(12):1440-5
76. Mallat Z, Lambeau G, Tedgui A. Lipoprotein-Associated and Secreted Phospholipases A2 in Cardiovascular Disease Roles as Biological Effectors and Biomarkers. Circulation. 2010 Nov 23;122(21):2183-200
77.Greco G, Balogh G, Brunelli R, Costa G, De Spirito M, Lenzi L, Mei G, Ursini F, Parasassi T. Generation in Human Plasma of Misfolded, Aggregation-Prone Electronegative Low Density Lipoprotein. Biophys J. 2009 Jul 22;97(2):628-35
78.Matsuzaki T, Sasaki K, Tanizaki Y, Hata J, Fujimi K, Matsui Y, Sekita A, Suzuki SO, Kanba S, Kiyohara Y, Iwaki T. Insulin resistance is associated with the pathology of Alzheimer disease: the Hisayama study. Neurology. 2010 Aug 31;75(9):764-70
79. Ho L, Qin W, Pompl PN, Xiang Z, Wang J, Zhao Z, Peng Y, Cambareri G, Rocher A, Mobbs CV, Hof PR, Pasinetti GM. Diet-induced insulin resistance promotes amyloidosis in a transgenic mouse model of Alzheimer's disease. FASEB J. 2004 May;18(7):902-4
80.Sie MP, van der Wiel HE, Smedts FM, de Boer AC. Human recombinant insulin and amyloidosis:an unexpected association. Neth J Med. 2010 Mar;68(3):138-40
81.Yumlu S, Barany R, Eriksson M, Röcken C. Localized insulin-derived amyloidosis in patients with diabetes mellitus: a case report. Hum Pathol. 2009;40(11):1655-60
82. Brange J, Andersen L, Laursen ED, Meyn G, Rasmussen E. Toward understanding insulin fibrillation. J Pharm Sci. 1997 May;86(5):517-25
83. Ratner RE, Phillips TM, Steiner M. Persistent cutaneous insulin allergy resulting from high-molecular-weight insulin aggregates. Diabetes. 1990 Jun;39(6):728-33
84.Xu S. Aggregation drives "misfolding" in protein amyloid fiber formation. Amyloid. 2007 Jun;14(2):119-31.
85. Maurer-Stroh S, Debulpaep M, Kuemmerer N, Lopez de la Paz M, Martins IC, Reumers J, Morris KL, Copland A, Serpell L, Serrano L, Schymkowitz JW, Rousseau F. Exploring the sequence determinants of amyloid structure using position-specific scoring matrices. Nat Methods. 2010 Mar;7(3):237-42
86. Puchtler H, Sweat F, Levine M. On the binding of Congo red by amyloid. J Histochem Cytochem 1962; 10: 355-64
87.Westermark GT, Johnson KH, Westermark P. Staining methods for identification of amyloid in tissue. Meth Enzymol 1999; 309: 3-25
88. Merlini G, Bellotti V. Molecular mechanisms of amyloidosis. N Engl J Med 2003; 349: 583-596
89. Merlini G, Westermark P. The systemic amyloidoses: clearer understanding of the molecular mechanisms offers hope for more effective therapies. J Intern Med. 2004 Feb;255(2):159-78
90. Glenner GG, Terry W, Harada M, Isersky C, Page D. Amyloid fibril proteins: proof of homology with immunoglobulin light chains by sequence analysis. Science 1971; 172: 1150-1
91.Gillmore JD, Lovat LB, Persey MR, Pepys MB, Hawkins PN. Amyloid load and clinical outcome in AA amyloidosis in relation to circulating concentration of serum amyloid A protein. Lancet 2001; 358: 24-9
92. Westermark P, Sletten K, Johansson B, Cornwell GG III. Fibril in senile systemic amyloidosis is derived from normal transthyretin. Proc Natl Acad Sci USA 1990; 87: 2843-5
93.Obici L, Bellotti V, Mangione P, Stoppini M, Arbustini E, Verga L, Zorzoli I, Anesi E, Zanotti G, Campana C, Viganò M, Merlini G. The new apolipoprotein A-I variant Leu(174) -> Ser causes hereditary cardiac amyloidosis, and the amyloid fibrils are constituted by the 93-residue N-terminal polypeptide. Am J Pathol 1999; 155: 695-702
94. Kaved R, Bernhagen J, Greenfield N, Sweimeh K, Brunner H, Voelter W, Kapurniotu A. Conformational transtitions of islet amyloid polypeptide (IAPP) in amyloid formation in vitro. J Mol Biol 1999; 287: 781-96
95.Janson J, Ashley RH, Harrison D, Mclntyre S, Butler PC. The mechanism of islet amyloid polypeptide toxicity is membrane disruption by intermediate-sized toxic amyloid particles. Diabetes 1999; 48: 491-8
96. Benson MD. LECT2 amyloidosis. Kidney Int. 2010 May;77(9):757-9.
97.Selkoe DJ. The origins of Alzheimer disease: a is for amyloid. JAMA 2000; 283: 1615-7
98.Cohen FE, Pan K-M, Huang Z, Baldwin M, Fletterick RJ, Prusiner SB. Structural clues to prion replication. Science 1994; 264: 530-1
99.Wille H, Baldwin MA, Cohen FE, DeArmond SJ, Prusiner SB. Prion protein amyloid: separation of scrapie infectivity from PrP polymers. Ciba Found Symp. 1996;199:181-99; discussion 199-201.
100. Chichester: John Wiley & Sons, 1996; 181-201
101. Drüeke TB. Beta2-microglobulin and amyloidosis. Nephrol Dial Transplant. 2000;15 Suppl 1:17-24
102. Wilson MR, Yerbury JJ, Poon S. Potential roles of abundant extracellular chaperones in the control of amyloid formation and toxicity. Mol Biosyst. 2008 Jan;4(1):42-52
103. Herczenik E, Bouma B, Korporaal SJ, Strangi R, Zeng Q, Gros P, Van Eck M, Van Berkel TJ, Gebbink MF, Akkerman JW. Activation of Human Platelets by Misfolded Proteins. Arterioscler Thromb Vasc Biol. 2007 Jul;27(7):1657-65
104. Sweeny JM, Gorog DA, Fuster V. Antiplatelet drug 'resistance'. Part 1: mechanisms and clinical measurements. Nat Rev Cardiol. 2009 Apr;6(4):273-82
105. Henry P, Vermillet A, Boval B, Guyetand C, Petroni T, Dillinger JG, Sideris G, Bal Dit Sollier C, Drouet L. 24-hour time-dependent aspirin efficacy in patients with stable coronary artery disease. Thromb Haemost. 2011 Feb 1;105(2):336-44.
106. Rothwell PM, Fowkes FG, Belch JF, Ogawa H, Warlow CP, Meade TW.Effect of daily aspirin on long-term risk of death due to cancer: analysis of individual patient data from randomised trials. Lancet. 2011 Jan 1;377(9759):31-41
107. Topol EJ, Schork NJ. Catapulting clopidogrel pharmacogenomics forward. Nat Med. 2011 Jan;17(1):40-1
108. Bouman HJ, Schömig E, van Werkum JW, Velder J, Hackeng CM, Hirschhäuser C, Waldmann C, Schmalz HG, ten Berg JM, Taubert D. Paraoxonase-1 is a major determinant of clopidogrel efficacy. Nat Med. 2011 Jan;17(1):110-6
109. Sutherland WH, de Jong SA, Walker RJ. Hypochlorous acid and low serum paraoxonase activity in haemodialysis patients: an in vitro study. Nephrol Dial Transplant. 2004 Jan;19(1):75-82
110. Kastelein JJ, Hack CE, Khaw KT. Serum levels of type II secretory phospholipase A2 and the risk of future coronary artery disease in apparently healthy men and women: the EPIC-Norfolk prospective population study. Arterioscler Thromb Vasc Biol 2005;25:839-846
111. Doi H, Sugiyama S, Yasue H. Circulating levels of secretory type II phospholipase A2 predict coronary events in patients with coronary artery disease. Circulation 1999;100:1280-1284
112. Liu PY, Li YH, Tsai WC, Chao TH, Tsai LM, Wu HL, Chen JH. Prognostic value and the changes of plasma levels of secretory type II phospholipase A2 in patients with coronary artery disease undergoing percutaneous coronary intervention. Eur Heart J 2003;24:1824-1832
113. Kugiyama K, Ota Y, Sugiyama S, Kawano H, Doi H, Soejima H, Miyamoto S, Ogawa H, Takazoe K, Yasue H. Prognostic value of plasma levels of secretory type II phospholipase A2 in patients with unstable angina pectoris. Am J Cardiol 2000;86:718-722
114. Porela P, Pulkki K, Voipio-Pulkki LM, Pettersson K, Leppänen V, Nevalainen TJ. Circulating phospholipase A2 in prediction of the prognosis of patients with suspected myocardial infarction. Basic Res Cardiol 2000;95:413-417.
115. Mattsson N, Magnussen CG, Rönnemaa T, Mallat Z, Benessiano J, Jula A, Taittonen L, Kähönen M, Juonala M, Viikari JS, Raitakari OT. Metabolic Syndrome and Carotid Intima-Media Thickness in Young Adults: Roles of Apolipoprotein B, Apolipoprotein A-I, C-Reactive Protein, and Secretory Phospholipase A2: The Cardiovascular Risk in Young Finns Study. Arterioscler Thromb Vasc Biol. 2010 Sep;30(9):1861-6
116. Partrick DA, Moore EE, Silliman CC, Barnett CC, Kuypers FA. Secretory phospholipase A2 activity correlates with postinjury multiple organ failure. Crit Care Med. 2001 May;29(5):989-93
117. Lausevic Z, Lausevic M, Trbojevic-Stankovic J, Krstic S, Stojimirovic B. Predicting multiple organ failure in patients with severe trauma. Can J Surg. 2008 Apr;51(2):97-102
118. Jaross W, Eckey R, Menschikowski M. Biological effects of secretory phospholipase A(2) group IIA on lipoproteins and in atherogenesis. Eur J Clin Invest. 2002 Jun;32(6):383-93
119. Rosenson RS, Elliott M, Stasiv Y, Hislop C; for the PLASMA II Investigators. Randomized trial of an inhibitor of secretory phospholipase A2 on atherogenic lipoprotein subclasses in statintreated patients with coronary heart disease. Eur Heart J. 2010 Nov 16. [Epub ahead of print]
120. Greco G, Balogh G, Brunelli R, Costa G, De Spirito M, Lenzi L, Mei G, Ursini F, Parasassi T. Generation in human plasma of misfolded, aggregation-prone electronegative low density lipoprotein. Biophys J. 2009 Jul 22;97(2):628-35
121. Asatryan L, Hamilton RT, Isas JM, Hwang J, Kaved R, Sevanian A. LDL phospholipid hydrolysis produces modified electronegative particles with an unfolded apoB-100 protein. J Lipid Res. 2005 Jan;46(1):115-22
122. Lee E, Nichols P, Spicer D, Groshen S, Yu MC, Lee AS. GRP78 as a novel predictor of responsiveness to chemotherapy in breast cancer. Cancer Res 2006; 66: 7849-53
123. Misra UK, Payne S, Pizzo SV. Ligation of prostate cancer cell surface GRP78 activates a proproliferative and antiapoptotic feedback loop: a role for secreted prostate-specific antigen. J Biol Chem. 2011 Jan 14;286(2):1248-59
124. de Ridder GG, Gonzalez-Gronow M, Ray R, Pizzo SV. Autoantibodies against cell surface GRP78 promote tumor growth in a murine model of melanoma. Melanoma Res. 2010 Dec 15. [Epub ahead of print]
125. Hardy B, Raiter A. Peptide-binding heat shock protein GRP78 protects cardiomyocytes from hypoxia-induced apoptosis. J Mol Med. 2010 Nov;88(11):1157-67
126. Thuerauf DJ, Marcinko M, Gude N, Rubio M, Sussman MA, Glembotski CC. Activation of the unfolded protein response in infarcted mouse heart and hypoxic cultured cardiac myocytes. Circ Res. 2006 Aug 4;99(3):275-82
127. Davidson DJ, Haskell C, Majest S, Kherzai A, Egan DA, Walter KA, Schneider A, Gubbins EF, Solomon L, Chen Z, Lesniewski R, Henkin J. Kringle 5 of human plasminogen induces apoptosis of endothelial and tumor cells through surface-expressed glucoseregulated protein 78. Cancer Res 2005; 65: 4663-72
128. Gebbink MF, Voest EE, Reijerkerk A. Do antiangiogenic protein fragments have amyloid properties? Blood. 2004 Sep 15;104(6):1601-5
129. Koch CG, Li L, Sessler DI, Figueroa P, Hoeltge GA, Mihaljevic T, Blackstone EH. Duration of Red-Cell Storage and Complications after Cardiac Surgery. N Engl J Med. 2008 Mar 20;358(12):1229-39.
130. Hermeling S, Crommelin DJ, Schellekens H, Jiskoot W. Strukture-Immmunogenicity relationships of therapeutic proteins. Pharm Res. 2004 Jun;21(6):897-903
131. Rosenberg AS. Effects of protein aggregates. An Immunologic Perspective. AAPS J. 2006 Aug 4;8(3):E501-7
132. Joubert MK, Hokom M, Xie J, Deshpande M, Kaliyaperumal A, Chirmule N, Juan G, Goltz T, Narhi L, Jawa V, An innate immune response was induced from PBMCs following challenge with aggregated biotherapeutics. 2010 Workshop On Protein Aggregation and Immunogenicity, Posterpresentation
133. Gagnon P, Arakawa T. Aggregation detection and removal in biopharmaceutical proteins. Curr Pharm Biotechnol. 2009 Jun;10(4):347
134. Wang W. Protein aggregation and its inhibition in biopharmaceutics. Int J Pharm. 2005 Jan 31;289(1-2):1-30
135. Stefani M, Dobson CM. Protein aggregation and aggregate toxicity: new insights into protein folding, misfolding diseases and biological evolution. J Mol Med. 2003 Nov;81(11):678-99
136. Cromwell M, Hilario E, Jacobson F. Protein Aggregation and Bioprocessing. AAPS Journal. 2006;8(3):E572-E579
137. Dische FE, Wernstedt C, Westermark GT, Westermark P, Pepys MB, Rennie JA, Gilbey SG, Watkins PJ. Insulin as an amyloid-fibril protein at sites of repeated insulin injections in a diabetic patient. Diabetologia. 1988;31(3):158-61
138. Ahmad A, Millett IS, Doniach S, Uversky VN, Fink AL. Partially folded intermediates in insulin fibrillation. Biochemistry. 2003;42(39):11404-16
139. Philo, J. S. A critical review of methods for size characterization of non-particulate protein aggregates. Curr Pharm Biotechnol. 2009 Jun;10(4):359-72.
140. Costerton JW, Lewandowski Z, Caldwell DE, Korber DR, Lappin-Scott HM. Microbial biofilms. Annu Rev Microbiol. 1995;49:711-45
141. Hall-Stoodley L, Costerton JW, Stoodley P. Bacterial Biofilms: from the natural environment to infectious diseases. Nat. Rev. Microbiol. 2004; 2: 95-108
142. Larsen P, Nielsen JL, Dueholm MS, Wetzel R, Otzen D, Nielsen PH.. Amyloid adhesins are abundant in natural biofilms. Environ Microbiol. 2007 Dec;9(12):3077-90.
143. Williams D. Revisiting the definition of biocompatibility. Med Device Technol. 2003 Oct;14(8):10-3
144. Ratner BD. The catastrophe revisited: Blood compatibility in the 21st Century. Biomaterials. 2007 Dec;28(34):5144-7
145. Wintermantel E, Ha S-W: Medizintechnik - Life Science Engineering, Springer-Verlag Berlin Heidelberg, 2009
146. Merritt K, Edwards CR, Brown SA. Use of an enzyme linked immunosorbent assay (ELISA) for quantification of proteins on the surface of materials. J Biomed Mater Res. 1988 Feb;22(2):99-109
147. Cherny I, Gazit E. Amyloide: nicht nur pathologische Substanzen, sondern auch geordnete Nanomaterialien. Angewandte ChemieVolume 120, Issue 22, 2008
148. Reches M, Gazit E. Assembly of Peptide Nanostructures: Mechanism of Association and Potential Uses Current Nanoscience, 2006, 2, 105-111
149. Hamedi M, Herland A, Karlsson RH, Inganäs O. Electrochemical devices made from conducting nanowire networks self-assembled from amyloid fibrils and alkoxysulfonate PEDOT. Nano Lett. 2008 Jun;8(6):1736-40
150. Scheibel T, Parthasarathy R, Sawicki G, Lin XM, Jaeger H, Lindguist SL. Conducting nanowires built by controlled self-assembly of amyloid fibers and selective metal deposition. Proc Natl Acad Sci U S A. 2003 Apr 15;100(8):4527-32
151. Gebbink MF, Claessen D, Bouma B, Dijkhuizen L, Wösten HA. Amyloids - a functional coat for microorganisms. Nat Rev Microbiol. 2005 Apr;3(4):333-41

## Patentansprüche

1. Verwendung einer Bindesubstanz, ausgewählt aus der Gruppe, bestehend aus Peptiden, welche die Sequenz His, Trp, Arg, Arg (HWRR), die Sequenz His, Trp, Arg, Arg, Pro (HWRRP), die Sequenz Ala, Val, Thr, Leu, Glu, Asn, Phe, Leu, His, Trp, Arg, Arg entsprechend den Aminosäuren 278-289 der ADAM15 Sequenz, die Sequenz Glu, Asn, Phe, Leu, His, Trp, Arg, Arg, Ala, His, Leu, Leu (ENFLHWRRAHLL) entsprechend den Aminosäuren 282-293 der ADAM 15 Sequenz oder die Sequenz His, Trp, Arg, Arg, Ala, His, Leu, Leu, Pro, Arg, Leu, Pro (HWRRAHLLPRLP) entsprechend den Aminosäuren 286-297 der ADAM15 Sequenz umfassen, der Metalloproteasedomäne von ADAM15 und ADAM15 (Metargidin), *in vitro* zur Anbindung missgefalteter Proteine oder Peptide, die bei Erkrankungen eine Rolle in der Pathogenese spielen.

2. Verwendung nach Anspruch 1,
wobei die Bindesubstanz an einer Festphase immobilisiert ist, und wobei vorzugsweise die Festphase ausgewählt ist aus einer Mikrotiterplatte, einem Chip für eine Oberflächen-Plasmon-Resonanz-Untersuchung, einem Mikroarray-Chip, einem Filter wie Nitrocellulose, Nylon oder PVDF, einer Membran, einem Teststreifen, einem magnetischen oder Fluorophor-markierten Kügelchen, einem Silizium-Wafer, Glas, Metall, Kunststoff, einem Chip, einem massenspektrometrischen Target oder einer Matrix, sowie Kügelchen (Beads) für Untersuchungen in Durchflusszytometern.

3. Verwendung nach einem der vorhergehenden Ansprüche,
zur Bestimmung von missgefalteten Proteinen oder Peptiden in einer Probe,
wobei vorzugsweise die Bestimmung einen qualitativen oder/und quantitativen Nachweis umfasst, oder
zur Entfernung oder Bereitstellung von missgefalteten Proteinen oder Peptiden aus einer Probe,
wobei die Probe ausgewählt ist aus Körperflüssigkeiten oder Gewebeauszügen wie beispielsweise Blut, Serum Blutplasma, Lymphe, Sperma, Vaginalflüssigkeit, Fruchtwasser, Cerebrospinalflüssigkeit, Synovialflüssigkeit, Urin, Sputum, Flüssigkeit aus Lavagen, wie z.B. bronchioalveoläre Lavageflüssigkeit und Peritoneallavageflüssigkeit, oder
wobei die Probe ausgewählt ist aus Medizinprodukten wie Arzneimitteln, Arzneizusatzstoffen, pharmazeutischen Zusammensetzungen, Reagenzien und Hilfsreagenzien für Diagnostische Tests, Forschungsreagenzien, Lebensmitteln, Genussmitteln, Nahrungsergänzungsmitteln, Trink-und Brauchwasser und Biofilmen.

4. Verfahren zur Bestimmung von missgefalteten Proteinen oder Peptiden in einer Probe *in vitro,* umfassend
a) Inkontaktbringen der Probe mit einer oder mehreren Bindesubstanzen, ausgewählt aus der Gruppe, bestehend aus Peptiden, welche die Sequenz His, Trp, Arg, Arg (HWRR), die Sequenz His, Trp, Arg, Arg, Pro (HWRRP), die Sequenz Ala, Val, Thr, Leu, Glu, Asn, Phe, Leu, His, Trp, Arg, Arg entsprechend den Aminosäuren 278-289 der ADAM15 Sequenz, die Sequenz Glu, Asn, Phe, Leu, His, Trp, Arg, Arg, Ala, His, Leu, Leu (ENFLHWRRAHLL) entsprechend den Aminosäuren 282-293 der ADAM 15 Sequenz oder die Sequenz His, Trp, Arg, Arg, Ala, His, Leu, Leu, Pro, Arg, Leu, Pro (HWRRAHLLPRLP) entsprechend den Aminosäuren 286-297 der ADAM15 Sequenz umfassen, der Metalloproteasedomäne von ADAM15 und ADAM15 (Metargidin),
b) qualitative oder/und quantitative Bestimmung der gebundenen missgefalteten Proteine oder Peptide.

5. Verfahren nach Anspruch 4, wobei die Probe ausgewählt ist aus Körperflüssigkeiten oder Gewebeauszügen wie beispielsweise Blut, Serum Blutplasma, Lymphe, Sperma, Vaginalflüssigkeit, Fruchtwasser, Cerebrospinalflüssigkeit, Synovialflüssigkeit, Urin, Sputum, Flüssigkeit aus Lavagen, wie z.B. bronchioalveoläre Lavageflüssigkeit und Peritoneallavageflüssigkeit, oder
wobei die Probe ausgewählt ist aus Medizinprodukten wie Arzneimitteln, Arzneizusatzstoffen, pharmazeutischen Zusammensetzungen, Reagenzien und Hilfsreagenzien für Diagnostische Tests, Forschungsreagenzien, Lebensmitteln, Genussmitteln, Nahrungsergänzungsmitteln, Trink-und Brauchwasser und Biofilmen.

6. Verfahren nach Anspruch 4 oder 5,
wobei die Bestimmung der gebundenen missgefalteten Proteine oder Peptide die Umsetzung mit einem spezifischen Detektionsreagenz für das missgefaltete Protein oder Peptid, vorzugsweise einem spezifischen Antikörper gegen das Protein oder Peptid, umfasst,
wobei vorzugsweise das Detektionsreagenz eine weitere Bindesubstanz für die gebundenen missgefalteten Proteine oder Peptide, vorzugsweise ausgewählt aus Chaperonen, Scavenger Rezeptoren, t-Pa, FXII, HGFA, Kongorot oder Thioflavin, umfasst oder/und vorzugsweise das Detektionsreagenz
a) durch Umsetzung mit einer Indikatorsubstanz nachgewiesen werden kann, wie beispielsweise Proteinmodifikationen durch Einwirkung von Acrolein und anderen elektrophilen Substanzen, Glykierungen, proteolytische Spaltung, Phosphorylierung, Dephosphorylierung, Glykosylierung, Acetylierung, S-Nitrosylierung, Citrullinierung oder Sulfatierung, oder
b) mit einem Erkennungslabel markiert ist, insbesondere mit Markierungen oder Teilen von Markierungen, beispielsweise einer Komponente eines spezifischen Bindungspaares,
wobei vorzugsweise das Erkennungslabel ausgewählt ist aus Fluoreszenzmarkern, Biotin, einem HIS-Tag, einem GST-Tag, einem SEAP-Tag, einem Maltose binding protein-Tag (MBP-Tag), einem FLAG-Tag, Digoxigenin, einem paramagnetischen Atom, einem radioaktiven Atom, wie z.B. Kohlenstoff-11, Jod-125/123, ^{99m}Tc, Cu-64 oder ¹¹¹In, sowie Reporter-Enzymen wie alkalische Phosphatase, Meerrettichperoxidase, β-Galactosidase, Glukoseoxidase, Luciferase, β-Lactamase, Urease oder Lysozym.

7. Verfahren nach einem der Ansprüche 4 bis 6
zur Kontrolle des Herstellungsprozesses von Arzneimitteln, pharmazeutischen Zusammensetzungen, Reagenzien und Hilfsreagenzien für Diagnostische Tests, Forschungsreagenzien, Lebensmitteln oder Biofilmen, oder/und
zur Hygiene- und Qualitätskontrolle im Bereich Forschung, Gesundheitswesen, Lebensmittelgewinnung, -lagerung und -handel.

8. Verfahren zur Entfernung von missgefalteten Proteinen oder Peptiden aus einer Probe oder einem Produkt *in vitro,* umfassend
a) Inkontaktbringen der Probe oder des Produkts mit einer oder mehreren Bindesubstanzen, ausgewählt aus der Gruppe, bestehend aus Peptiden, welche die Sequenz His, Trp, Arg, Arg (HWRR), die Sequenz His, Trp, Arg, Arg, Pro (HWRRP), die Sequenz Ala, Val, Thr, Leu, Glu, Asn, Phe, Leu, His, Trp, Arg, Arg entsprechend den Aminosäuren 278-289 der ADAM15 Sequenz, die Sequenz Glu, Asn, Phe, Leu, His, Trp, Arg, Arg, Ala, His, Leu, Leu (ENFLHWR-RAHLL) entsprechend den Aminosäuren 282-293 der ADAM 15 Sequenz oder die Sequenz His, Trp, Arg, Arg, Ala, His, Leu, Leu, Pro, Arg, Leu, Pro (HWRRAHLLPRLP) entsprechend den Aminosäuren 286-297 der ADAM15 Sequenz umfassen, der Metalloproteasedomäne von ADAM15 und ADAM15 (Metargidin),
b) Abtrennen der gebundenen missgefalteten Proteine oder Peptide.

9. Verfahren nach Anspruch 8, wobei die Probe ausgewählt ist aus Körperflüssigkeiten oder Gewebeauszügen wie beispielsweise Blut, Serum Blutplasma, Lymphe, Sperma, Vaginalflüssigkeit, Fruchtwasser, Cerebrospinalflüssigkeit, Synovialflüssigkeit, Urin, Sputum, Flüssigkeit aus Lavagen, wie z.B. bronchioalveoläre Lavageflüssigkeit und Peritoneallavageflüssigkeit, oder
wobei die Probe oder das Produkt ausgewählt ist aus Medizinprodukten wie Arzneimitteln, Arzneizusatzstoffen, pharmazeutischen Zusammensetzungen, Reagenzien und Hilfsreagenzien für Diagnostische Tests, Forschungsreagenzien, Lebensmitteln, Genussmitteln, Nahrungsergänzungsmitteln, Trink- und Brauchwasser und Biofilmen.

10. Verfahren zur Bereitstellung von missgefalteten Proteinen oder Peptiden aus einer Probe *in vitro,* umfassend
a) Inkontaktbringen der Probe mit einer oder mehreren Bindesubstanzen, ausgewählt aus der Gruppe, bestehend aus Peptiden, welche die Sequenz His, Trp, Arg, Arg (HWRR), die Sequenz His, Trp, Arg, Arg, Pro (HWRRP), die Sequenz Ala, Val, Thr, Leu, Glu, Asn, Phe, Leu, His, Trp, Arg, Arg entsprechend den Aminosäuren 278-289 der ADAM15 Sequenz, die Sequenz Glu, Asn, Phe, Leu, His, Trp, Arg, Arg, Ala, His, Leu, Leu (ENFLHWRRAHLL) entsprechend den Aminosäuren 282-293 der ADAM 15 Sequenz oder die Sequenz His, Trp, Arg, Arg, Ala, His, Leu, Leu, Pro, Arg, Leu, Pro (HWRRAHLLPRLP) entsprechend den Aminosäuren 286-297 der ADAM15 Sequenz umfassen, der Metalloproteasedomäne von ADAM15 und ADAM15 (Metargidin),
b) Abtrennen der gebundenen missgefalteten Proteine oder Peptide, und
c) Isolieren der missgefalteten Proteine oder Peptide,
wobei vorzugsweise die Probe ausgewählt ist aus Körperflüssigkeiten oder Gewebeauszügen wie beispielsweise Blut, Serum Blutplasma, Lymphe, Sperma, Vaginalflüssigkeit, Fruchtwasser, Cerebrospinalflüssigkeit, Synovialflüssigkeit, Urin, Sputum, Flüssigkeit aus Lavagen, wie z.B. bronchioalveoläre Lavageflüssigkeit und Peritoneallavageflüssigkeit, oder
wobei die Probe ausgewählt ist aus Medizinprodukten wie Arzneimitteln, Arzneizusatzstoffen, pharmazeutischen Zusammensetzungen, Reagenzien und Hilfsreagenzien für Diagnostische Tests, Forschungsreagenzien, Lebensmitteln, Genussmitteln, Nahrungsergänzungsmitteln, Trink- und Brauchwasser und Biofilmen.

11. Verfahren zur Diagnose, Stratifizierung oder/und Überwachung von Erkrankungen, an denen missgefaltete Proteine oder Peptide beteiligt sind, *in vitro* umfassend die Schritte
a) qualitative oder/und quantitative Bestimmung von missgefalteten Proteinen oder Peptiden in einer bereitgestellten Probe nach Inkontaktbringen der Probe mit einer oder mehreren Bindesubstanzen, ausgewählt aus der Gruppe, bestehend aus Peptiden, welche die Sequenz His, Trp, Arg, Arg (HWRR), die Sequenz His, Trp, Arg, Arg, Pro (HWRRP), die Sequenz Ala, Val, Thr, Leu, Glu, Asn, Phe, Leu, His, Trp, Arg, Arg entsprechend den Aminosäuren 278-289 der ADAM15 Sequenz, die Sequenz Glu, Asn, Phe, Leu, His, Trp, Arg, Arg, Ala, His, Leu, Leu (ENFLHWRRAHLL) entsprechend den Aminosäuren 282-293 der ADAM 15 Sequenz oder die Sequenz His, Trp, Arg, Arg, Ala, His, Leu, Leu, Pro, Arg, Leu, Pro (HWRRAHLLPRLP) entsprechend den Aminosäuren 286-297 der ADAM15 Sequenz umfassen, der Metalloproteasedomäne von ADAM15 und ADAM15 (Metargidin),
b) Vergleich des Ergebnisses der Bestimmung in Schritt a) mit einem oder mehreren Referenzwerten.

12. Verfahren nach Anspruch 11 ,
zum Monitoring oder/und Überwachen einer medikamentösen Therapie, wobei vorzugsweise die medikamentöse Therapie eine Behandlung mit Acetylsalicylsäure, Thienopyridin wie etwa Clopidogrel, Prasugrel oder Ticlopidin, PEGylierten Liposomen und/oder sPLA2 Inhibitoren umfasst.

13. Diagnostischer Kit für die Bestimmung von missgefalteten Proteinen oder Peptiden, umfassend
a) eine erste Bindesubstanz, ausgewählt aus der Gruppe, bestehend aus Peptiden, welche die Sequenz His, Trp, Arg, Arg (HWRR), die Sequenz His, Trp, Arg, Arg, Pro (HWRRP), die Sequenz Ala, Val, Thr, Leu, Glu, Asn, Phe, Leu, His, Trp, Arg, Arg entsprechend den Aminosäuren 278-289 der ADAM15 Sequenz, die Sequenz Glu, Asn, Phe, Leu, His, Trp, Arg, Arg, Ala, His, Leu, Leu (ENFLHWR-RAHLL) entsprechend den Aminosäuren 282-293 der ADAM 15 Sequenz oder die Sequenz His, Trp, Arg, Arg, Ala, His, Leu, Leu, Pro, Arg, Leu, Pro (HWRRAHLLPRLP) entsprechend den Aminosäuren 286-297 der ADAM15 Sequenz umfassen, der Metalloproteasedomäne von ADAM15 und ADAM15 (Metargidin), und
b) ein spezifisches Detektionsreagenz für das missgefaltete Protein oder Peptid, wobei das Detektionsreagenz vorzugsweise eine weitere Bindesubstanz, ausgewählt aus der Gruppe, bestehend aus Peptiden, welche die Sequenz His, Trp, Arg, Arg (HWRR), die Sequenz His, Trp, Arg, Arg, Pro (HWRRP), die Sequenz Ala, Val, Thr, Leu, Glu, Asn, Phe, Leu, His, Trp, Arg, Arg entsprechend den Aminosäuren 278-289 der ADAM15 Sequenz, die Sequenz Glu, Asn, Phe, Leu, His, Trp, Arg, Arg, Ala, His, Leu, Leu (ENFLHWRRAHLL) entsprechend den Aminosäuren 282-293 der ADAM 15 Sequenz oder die Sequenz His, Trp, Arg, Arg, Ala, His, Leu, Leu, Pro, Arg, Leu, Pro (HWRRAHLLPRLP) entsprechend den Aminosäuren 286-297 der ADAM15 Sequenz umfassen, der Metalloproteasedomäne von ADAM15 und ADAM15 (Metargidin), oder ausgewählt aus Chaperonen, Scavenger Rezeptoren, t-Pa, FXII, HGFA, Kongorot oder Thioflavin, oder einen spezifischen Antikörper gegen das Protein oder Peptid umfasst,
wobei eine der Komponenten a) und b) eine nachweisbare Markierung trägt und die andere der Komponenten a) und b) an einer Festphase immobilisiert ist.

14. Verfahren zur Bestimmung der Biokompatibilität eines Materials *in vitro,* umfassend die Schritte:
a) Inkontaktbringen des Materials mit einer Testsubstanz, die Proteine oder Peptide umfasst,
b) qualitative oder/und quantitative Bestimmung von missgefalteten Proteinen oder Peptiden in der Testsubstanz, nach einem Verfahren wie in einem der Ansprüche 4 und 5 definiert,
c) Vergleich der Menge der in der Testsubstanz enthaltenen missgefalteten Proteine oder Peptide vor und nach dem Inkontaktbringen mit dem Material und
d) Bestimmung, ob das Material in Proteinen oder Peptiden der Testsubstanz Missfaltungen erzeugt.

15. Verfahren zur Aufkonzentrierung von Mikroorganismen, welche missgefaltete Proteine auf ihrer Oberfläche tragen, aus einer Probe *in vitro,* umfassend die Schritte:
a) Inkontaktbringen der Probe mit einer oder mehreren Bindesubstanzen, ausgewählt aus der Gruppe, bestehend aus Peptiden, welche die Sequenz His, Trp, Arg, Arg (HWRR), die Sequenz His, Trp, Arg, Arg, Pro (HWRRP), die Sequenz Ala, Val, Thr, Leu, Glu, Asn, Phe, Leu, His, Trp, Arg, Arg entsprechend den Aminosäuren 278-289 der ADAM15 Sequenz, die Sequenz Glu, Asn, Phe, Leu, His, Trp, Arg, Arg, Ala, His, Leu, Leu (ENFLHWRRAHLL) entsprechend den Aminosäuren 282-293 der ADAM 15 Sequenz oder die Sequenz His, Trp, Arg, Arg, Ala, His, Leu, Leu, Pro, Arg, Leu, Pro (HWRRAHLLPRLP) entsprechend den Aminosäuren 286-297 der ADAM15 Sequenz umfassen, der Metalloproteasedomäne von ADAM15 und ADAM15 (Metargidin),
b) Abtrennen der gebundenen Mikroorganismen aus der Probe.

## Claims

1. Use of a bonding substance, selected from the group consisting of peptides, which comprise the sequence His, Trp, Arg, Arg (HWRR), the sequence His, Trp, Arg, Arg, Pro (HWRRP), the sequence Ala, Val, Thr, Leu, Glu, Asn, Phe, Leu, His, Trp, Arg, Arg corresponding to amino acids 278-289 of the ADAM15 sequence, the sequence Glu, Asn, Phe, Leu, His, Trp, Arg, Arg, Ala, His, Leu, Leu (ENFLHWRRAHLL) corresponding to amino acids 282-293 of the ADAM 15 sequence or the sequence His, Trp, Arg, Arg, Ala, His, Leu, Leu, Pro, Arg, Leu, Pro (HWRRAHLLPRLP) corresponding to amino acids 286-297 of the ADAM15 sequence, the metalloprotease domain of ADAM15 and ADAM15 (metargidin), *in vitro* for bonding misfolded proteins or peptides that play a role for the pathogenesis of diseases.

2. Use according to claim 1,
wherein the bonding substance is immobilised in a solid phase, and wherein the solid phase is preferably selected from a micro-titre plate, a chip for a surface plasmon resonance examination, a micro-array chip, a filter such as nitrocellulose, nylon or PVDF, a membrane, a test strip, a magnetic or fluorophore marked bead, a silicon wafer, glass, metal, plastic, a chip, a mass-spectrometric target or a matrix, as well as beads (beads) for examinations in flow cytometers.

3. Use according to one of the preceding claims,
for determining misfolded proteins or peptides in a sample,
wherein the determination preferably comprises qualitative or/and quantitative evidence,
or for removing or providing misfolded proteins or peptides from a sample,
wherein the sample is selected from body fluids or tissue extracts such as for example blood, serum, blood plasma, lymph, sperm, vaginal fluid, amniotic fluid, cerebrospinal fluid, synovial fluid, urine, sputum, fluid from lavages such as for example bronchioalveolar lavage fluid and peritoneal lavage fluid, or
wherein the sample is selected from medical products such as drugs, drug additives, pharmaceutical compositions, reagents and auxiliary reagents for diagnostic tests, research reagents, foodstuffs, drinks and tobacco products, food additives, potable and grey water and biofilms.

4. Method for determining misfolded proteins or peptides in a sample *in vitro*, comprising
a) bringing into contact the sample with one or more bonding substances, selected from the group consisting of peptides, which comprise the sequence His, Trp, Arg, Arg (HWRR), the sequence His, Trp, Arg, Arg, Pro (HWRRP), the sequence Ala, Val, Thr, Leu, Glu, Asn, Phe, Leu, His, Trp, Arg, Arg corresponding to amino acids 278-289 of the ADAM15 sequence, the sequence Glu, Asn, Phe, Leu, His, Trp, Arg, Arg, Ala, His, Leu, Leu (ENFLHWRRAHLL) corresponding to amino acids 282-293 of the ADAM 15 sequence or the sequence His, Trp, Arg, Arg, Ala, His, Leu, Leu, Pro, Arg, Leu, Pro (HWRRAHLLPRLP) corresponding to amino acids 286-297 of the ADAM15 sequence, the metalloprotease domain of ADAM15 and ADAM15 (metargidin),
b) qualitative or/and quantitative determination of the bonded misfolded proteins or peptides.

5. Method according to claim 4, wherein the sample is selected from body fluids or tissue extracts such as for example blood, serum, blood plasma, lymph, sperm, vaginal fluid, amniotic fluid, cerebrospinal fluid, synovial fluid, urine, sputum, fluid from lavages such as for example bronchioalveolar lavage fluid and peritoneal lavage fluid, or
wherein the sample is selected from medical products such as drugs, drug additives, pharmaceutical compositions, reagents and auxiliary reagents for diagnostic tests, research reagents, foodstuffs, drinks and tobacco products, food additives, potable and grey water and biofilms.

6. Method according to claim 4 or 5,
wherein the determination of the bonded misfolded protein or peptides comprises the conversion with a specific detection reagent for the misfolded protein or peptide, preferably a specific antibody against the protein or the peptide,
wherein the detection reagent preferably comprises a further bonding substance for the bonded misfolded proteins or peptides, preferably selected from chaperones, scavenger receptors, t-Pa, FXII, HGFA, Congo red or thioflavin or/and preferably the detection reagent
a) that can be proven through conversion with an indicator substance, such as for example protein modifications through influencing with acrolein and other electrophilic substances, glycations, proteolytic cleavage, phosphorylisation, dephosphorylisation, glycosylisation, acetylisation, S-nitrosylisation, citrullination or sulfation, or
b) is marked with an identification tag, in particular with markings or parts of markings, for example a component of a specific bonding pair,
wherein the identification tag is preferably selected from fluorescent markers, biotin, a HIS tag, a GST tag, a SEAP tag, a maltose bonding protein tag (MBP tag), a FLAG tag, digoxigenin, a paramagnetic atom, a radioactive atom, such as for example carbon 11, iodine 125/123, ^{99m}Tc, Cu-64 or ¹¹¹In, as well as reporter enzymes such as alkaline phosphatase, horseradish peroxidase, β-galactosidase, glucoseoxidase, luciferase, β-lactamase, urease or lysozyme.

7. Method according to one of the claims 4 to 6
for controlling the manufacturing process of drugs, pharmaceutical compositions, reagents and auxiliary reagents for diagnostic tests, research reagents, foodstuffs or biofilms,
or/and
for hygiene and quality control within the areas of research, health care, food production, storage and retailing.

8. Method for removing misfolded proteins or peptides from a sample or a product *in vitro*, comprising
a) bringing into contact the sample or the product with one or more bonding substances, selected from the group consisting of peptides, which comprise the sequence His, Trp, Arg, Arg (HWRR), the sequence His, Trp, Arg, Arg, Pro (HWRRP), the sequence Ala, Val, Thr, Leu, Glu, Asn, Phe, Leu, His, Trp, Arg, Arg corresponding to amino acids 278-289 of the ADAM15 sequence, the sequence Glu, Asn, Phe, Leu, His, Trp, Arg, Arg, Ala, His, Leu, Leu (ENFLHWRRAHLL) corresponding to amino acids 282-293 of the ADAM 15 sequence or the sequence His, Trp, Arg, Arg, Ala, His, Leu, Leu, Pro, Arg, Leu, Pro (HWRRAHLLPRLP) corresponding to amino acids 286-297 of the ADAM15 sequence, the metalloprotease domain of ADAM15 and ADAM15 (metargidin),
b) separating the bonded misfolded proteins or peptides.

9. Method according to claim 8, wherein the sample is selected from body fluids or tissue extracts such as for example blood, serum, blood plasma, lymph, sperm, vaginal fluid, amniotic fluid, cerebrospinal fluid, synovial fluid, urine, sputum, fluid from lavages such as for example bronchioalveolar lavage fluid and peritoneal lavage fluid, or
wherein the sample is selected from medical products such as drugs, drug additives, pharmaceutical compositions, reagents and auxiliary reagents for diagnostic tests, research reagents, foodstuffs, drinks and tobacco products, food additives, potable and grey water and biofilms.

10. Method for providing misfolded proteins or peptides from a sample *in vitro*, comprising
a) bringing into contact the sample with one or more bonding substances, selected from the group consisting of peptides, which comprise the sequence His, Trp, Arg, Arg (HWRR), the sequence His, Trp, Arg, Arg, Pro (HWRRP), the sequence Ala, Val, Thr, Leu, Glu, Asn, Phe, Leu, His, Trp, Arg, Arg corresponding to amino acids 278-289 of the ADAM15 sequence, the sequence Glu, Asn, Phe, Leu, His, Trp, Arg, Arg, Ala, His, Leu, Leu (ENFLHWRRAHLL) corresponding to amino acids 282-293 of the ADAM 15 sequence or the sequence His, Trp, Arg, Arg, Ala, His, Leu, Leu, Pro, Arg, Leu, Pro (HWRRAHLLPRLP) corresponding to amino acids 286-297 of the ADAM15 sequence, the metalloprotease domain of ADAM15 and ADAM15 (metargidin),
b) separating the bonded misfolded proteins or peptides, and
c) isolating the misfolded proteins or peptides,
wherein the sample is preferably selected from body fluids or tissue extracts such as for example blood, serum, blood plasma, lymph, sperm, vaginal fluid, amniotic fluid, cerebrospinal fluid, synovial fluid, urine, sputum, fluid from lavages such as for example bronchioalveolar lavage fluid and peritoneal lavage fluid, or
wherein the sample is selected from medical products such as drugs, drug additives, pharmaceutical compositions, reagents and auxiliary reagents for diagnostic tests, research reagents, foodstuffs, drinks and tobacco products, food additives, potable and grey water and biofilms.

11. Method for the diagnosis, stratification or/and monitoring of diseases in which misfolded proteins or peptides play a role, *in vitro* comprising the steps:
a) qualitative or/and quantitative determination of the bonded misfolded proteins or peptides in a provided sample after bringing into contact the sample with one or more bonding substances, selected from the group consisting of peptides, which comprise the sequence His, Trp, Arg, Arg (HWRR), the sequence His, Trp, Arg, Arg, Pro (HWRRP), the sequence Ala, Val, Thr, Leu, Glu, Asn, Phe, Leu, His, Trp, Arg, Arg corresponding to amino acids 278-289 of the ADAM15 sequence, the sequence Glu, Asn, Phe, Leu, His, Trp, Arg, Arg, Ala, His, Leu, Leu (ENFLHWRRAHLL) corresponding to amino acids 282-293 of the ADAM 15 sequence or the sequence His, Trp, Arg, Arg, Ala, His, Leu, Leu, Pro, Arg, Leu, Pro (HWRRAHLLPRLP) corresponding to amino acids 286-297 of the ADAM15 sequence, the metalloprotease domain of ADAM15 and ADAM15 (metargidin),
b) comparing the results of the determination in step a) with one or more reference values.

12. Method according to claim 11,
for monitoring or/and observing a medicinal therapy, wherein the medicinal therapy preferably comprises a treatment with acetylsalicylic acid, thienopyridine such as for example clopidogrel, prasugrel or ticlopidin, PEGylated liposomes and/or sPLA2 inhibitors.

13. Diagnostic kit for the determination of misfolded proteins or peptides, comprising
a) a first binding substance, selected from the group consisting of peptides, which comprise the sequence His, Trp, Arg, Arg (HWRR), the sequence His, Trp, Arg, Arg, Pro (HWRRP), the sequence Ala, Val, Thr, Leu, Glu, Asn, Phe, Leu, His, Trp, Arg, Arg corresponding to amino acids 278-289 of the ADAM15 sequence, the sequence Glu, Asn, Phe, Leu, His, Trp, Arg, Arg, Ala, His, Leu, Leu (ENFLHWRRAHLL) corresponding to amino acids 282-293 of the ADAM 15 sequence or the sequence His, Trp, Arg, Arg, Ala, His, Leu, Leu, Pro, Arg, Leu, Pro (HWRRAHLLPRLP) corresponding to amino acids 286-297 of the ADAM15 sequence, the metalloprotease domain of ADAM15 and ADAM15 (metargidin), and
b) a specific detection reagent for the misfolded protein or peptide, wherein the detection reagent is preferably a further bonding substance, selected from the group consisting of peptides, which comprise the sequence His, Trp, Arg, Arg (HWRR), the sequence His, Trp, Arg, Arg, Pro (HWRRP), the sequence Ala, Val, Thr, Leu, Glu, Asn, Phe, Leu, His, Trp, Arg, Arg corresponding to amino acids 278-289 of the ADAM15 sequence, the sequence Glu, Asn, Phe, Leu, His, Trp, Arg, Arg, Ala, His, Leu, Leu (ENFLHWRRAHLL) corresponding to amino acids 282-293 of the ADAM 15 sequence or the sequence His, Trp, Arg, Arg, Ala, His, Leu, Leu, Pro, Arg, Leu, Pro (HWRRAHLLPRLP) corresponding to amino acids 286-297 of the ADAM15 sequence, the metalloprotease domain of ADAM15 and ADAM15 (metargidin), or selected from chaperones, scavenger receptors, t-Pa, FXII, HGFA, Congo red or thioflavin, or a specific antibody against the protein or peptide,
wherein one of the components a) and b) bears a traceable marking and the other of the components a) and b) is immobilised in a solid phase.

14. Method for determining the biocompatibility of a material *in vitro*, comprising the steps:
a) bringing into contact the material with a test substance comprising proteins or peptides,
b) qualitative or/and quantitative determination of misfolded proteins or peptides in the test substance, according to a method as defined in claims 4 and 5,
c) comparison of the quantity of the misfolded proteins or peptides contained in the test substance prior to and after bringing into contact with the material, and
d) determination of whether the material generates misfoldings in proteins or peptides of the test substance.

15. Method for concentrating micro-organisms, which carry misfolded proteins on their surface, from a sample *in vitro*, comprising the steps:
a) bringing into contact the sample with one or more bonding substances, selected from the group consisting of peptides, which comprise the sequence His, Trp, Arg, Arg (HWRR), the sequence His, Trp, Arg, Arg, Pro (HWRRP), the sequence Ala, Val, Thr, Leu, Glu, Asn, Phe, Leu, His, Trp, Arg, Arg corresponding to amino acids 278-289 of the ADAM15 sequence, the sequence Glu, Asn, Phe, Leu, His, Trp, Arg, Arg, Ala, His, Leu, Leu (ENFLHWRRAHLL) corresponding to amino acids 282-293 of the ADAM 15 sequence or the sequence His, Trp, Arg, Arg, Ala, His, Leu, Leu, Pro, Arg, Leu, Pro (HWRRAHLLPRLP) corresponding to amino acids 286-297 of the ADAM15 sequence, the metalloprotease domain of ADAM15 and ADAM15 (metargidin),
b) separating the bonded micro-organisms from the sample.

## Revendications

1. Utilisation d'une substance de liaison choisie dans le groupe consistant en peptides qui comprennent la séquence His, Trp, Arg, Arg (HWRR), la séquence His, Trp, Arg, Arg, Pro (HWRRP), la séquence Ala, Val, Thr, Leu, Glu, Asn, Phe, Leu, His, Trp, Arg, Arg correspondant aux acides aminés 278-289 de la séquence de ADAM15, la séquence Glu, Asn, Phe, Leu, His, Trp, Arg, Arg, Ala, His, Leu, Leu (ENFLHWRRAHLL) correspondant aux acides aminés 282-293 de la séquence de ADAM15 ou la séquence His, Trp, Arg, Arg, Ala, His, Leu, Leu, Pro, Arg, Leu, Pro (HWRRAHLLPRLP) correspondant aux acides aminés 286-297 de la séquence de ADAM15, le domaine métalloprotéase de ADAM15 et ADAM15 (métargidine), *in vitro* pour la fixation de protéines ou de peptides mal repliés qui jouent un rôle dans la pathogenèse lors de maladies.

2. Utilisation selon la revendication 1,
où la substance de liaison est immobilisée sur une phase solide, et où de préférence la phase solide est choisie parmi une plaque de microtitrage, une puce pour un examen de résonance plasmonique de surface, une puce à microarray, un filtre tel que la nitrocellulose, le Nylon ou le PVDF, une membrane, une bandelette de test, une bille magnétique ou marquée avec un fluorophore, une plaquette de silicium, du verre, du métal, du plastique, une puce, une cible de spectrométrie de masse ou une matrice, ainsi que des billes (beads) pour des examens dans des cytomètres en flux.

3. Utilisation selon l'une des revendications précédentes, pour la détermination de protéines ou de peptides mal repliés dans un échantillon,
où de préférence la détermination comprend une mise en évidence qualitative et/ou quantitative, ou
pour l'élimination ou la fourniture de protéines ou de peptides mal repliés à partir d'un échantillon,
où l'échantillon est choisi parmi les fluides corporels ou les extraits de tissus comme par exemple le sang, le plasma sanguin sérique, la lymphe, le sperme, le liquide vaginal, le liquide amniotique, le liquide céphalo-rachidien, le liquide synovial, l'urine, les expectorations, le liquide provenant de lavages, comme par exemple le liquide de lavage bronchioalvéolaire et le liquide de lavage péritonéal, ou bien
où l'échantillon est choisi parmi les produits médicaux tels que les médicaments, les additifs de médicaments, les compositions pharmaceutiques, les réactifs et les réactifs auxiliaires pour les tests de diagnostic, les réactifs de recherche, les aliments, les produits de consommation de luxe, les compléments alimentaires, l'eau potable et industrielle et les biofilms.

4. Procédé pour la détermination de protéines ou de peptides mal repliés dans un échantillon *in vitro*, comprenant
a) la mise en contact de l'échantillon avec une ou plusieurs substances de liaison choisies dans le groupe consistant en peptides qui comprennent la séquence His, Trp, Arg, Arg (HWRR), la séquence His, Trp, Arg, Arg, Pro (HWRRP), la séquence Ala, Val, Thr, Leu, Glu, Asn, Phe, Leu, His, Trp, Arg, Arg correspondant aux acides aminés 278-289 de la séquence de ADAM15, la séquence Glu, Asn, Phe, Leu, His, Trp, Arg, Arg, Ala, His, Leu, Leu (ENFLHWRRAHLL) correspondant aux acides aminés 282-293 de la séquence de ADAM15 ou la séquence His, Trp, Arg, Arg, Ala, His, Leu, Leu, Pro, Arg, Leu, Pro (HWRRAHLLPRLP) correspondant aux acides aminés 286-297 de la séquence de ADAM15, le domaine métalloprotéase de ADAM15 et ADAM15 (métargidine),
b) la détermination qualitative et/ou quantitative des protéines ou des peptides mal repliés liés.

5. Procédé selon la revendication 4, où l'échantillon est choisi parmi les fluides corporels ou les extraits de tissus comme par exemple le sang, le plasma sanguin sérique, la lymphe, le sperme, le liquide vaginal, le liquide amniotique, le liquide céphalo-rachidien, le liquide synovial, l'urine, les expectorations, le liquide provenant de lavages, comme par exemple le liquide de lavage bronchioalvéolaire et le liquide de lavage péritonéal, ou bien
où l'échantillon est choisi parmi les produits médicaux tels que les médicaments, les additifs de médicaments, les compositions pharmaceutiques, les réactifs et les réactifs auxiliaires pour les tests de diagnostic, les réactifs de recherche, les aliments, les produits de consommation de luxe, les compléments alimentaires, l'eau potable et industrielle et les biofilms.

6. Procédé selon la revendication 4 ou 5,
où la détermination des protéines ou des peptides mal repliés liés comprend la réaction avec un réactif de détection spécifique pour la protéine ou le peptide mal replié, de préférence un anticorps spécifique contre la protéine ou le peptide,
où de préférence le réactif de détection comprend une autre substance de liaison pour les protéines ou les peptides mal repliés liés, de préférence choisie parmi les chaperonnes, les récepteurs piégeurs, t-Pa, FXII, HGFA, le rouge Congo ou la thioflavine, et/ou de préférence le réactif de détection
a) peut être mis en évidence par réaction avec une substance indicatrice, comme par exemple les modifications de protéines par l'action de l'acroléine et d'autres substances électrophiles, les glycations, le clivage protéolytique, la phosphorylation, la déphosphorylation, la glycosylation, l'acétylation, la S-nitrosylation, la citrullination ou la sulfatation, ou
b) est marqué avec un marqueur d'identification, en particulier avec des marquages ou des parties de marquages, par exemple un composant d'une paire de liaison spécifique,
où de préférence le marqueur d'identification est choisi parmi les marqueurs fluorescents, la biotine, une étiquette HIS, une étiquette GST, une étiquette SEAP, une étiquette de protéine de liaison au maltose (étiquette MBP), une étiquette FLAG, la digoxigénine, un atome paramagnétique, un atome radioactif, comme par exemple le carbone-11, l'iode-125/123, ^{99m}Tc, Cu-64 ou ¹¹¹In, ainsi que les enzymes rapporteurs telles que la phosphatase alcaline, la peroxydase de raifort, la β-galactosidase, la glucose-oxydase, la luciférase, la β-lactamase, l'uréase ou le lysozyme.

7. Procédé selon l'une des revendications 4 à 6 pour le contrôle du processus de fabrication de médicaments, de compositions pharmaceutiques, de réactifs et de réactifs auxiliaires pour les tests de diagnostic, de réactifs de recherche, d'aliments ou de biofilms, et/ou
pour le contrôle de l'hygiène et de la qualité dans le domaine de la recherche, des soins de santé, de la production, du stockage et du commerce des aliments.

8. Procédé pour éliminer des protéines ou des peptides mal repliés d'un échantillon ou d'un produit *in vitro*, comprenant
a) la mise en contact de l'échantillon ou du produit avec une ou plusieurs substances de liaison choisies dans le groupe consistant en peptides qui comprennent la séquence His, Trp, Arg, Arg (HWRR), la séquence His, Trp, Arg, Arg, Pro (HWRRP), la séquence Ala, Val, Thr, Leu, Glu, Asn, Phe, Leu, His, Trp, Arg, Arg correspondant aux acides aminés 278-289 de la séquence de ADAM15, la séquence Glu, Asn, Phe, Leu, His, Trp, Arg, Arg, Ala, His, Leu, Leu (ENFLHWR-RAHLL) correspondant aux acides aminés 282-293 de la séquence de ADAM15 ou la séquence His, Trp, Arg, Arg, Ala, His, Leu, Leu, Pro, Arg, Leu, Pro (HWRRAHLLPRLP) correspondant aux acides aminés 286-297 de la séquence de ADAM15, le domaine métalloprotéase de ADAM15 et ADAM15 (métargidine),
b) la séparation des protéines ou des peptides mal repliés liés.

9. Procédé selon la revendication 8, où l'échantillon est choisi parmi les fluides corporels ou les extraits de tissus comme par exemple le sang, le plasma sanguin sérique, la lymphe, le sperme, le liquide vaginal, le liquide amniotique, le liquide céphalo-rachidien, le liquide synovial, l'urine, les expectorations, le liquide provenant de lavages comme par exemple le liquide de lavage bronchioalvéolaire et le liquide de lavage péritonéal, ou bien
où l'échantillon ou le produit est choisi parmi les produits médicaux comme les médicaments, les additifs de médicaments, les compositions pharmaceutiques, les réactifs et les réactifs auxiliaires pour les tests de diagnostic, les réactifs de recherche, les produits alimentaires, les produits de consommation de luxe, les compléments alimentaires, l'eau potable et industrielle et les biofilms.

10. Procédé pour fournir des protéines ou des peptides mal repliés à partir d'un échantillon *in vitro*, comprenant
a) la mise en contact de l'échantillon avec une ou plusieurs substances de liaison choisies dans le groupe consistant en peptides qui comprennent la séquence His, Trp, Arg, Arg (HWRR), la séquence His, Trp, Arg, Arg, Pro (HWRRP), la séquence Ala, Val, Thr, Leu, Glu, Asn, Phe, Leu, His, Trp, Arg, Arg correspondant aux acides aminés 278-289 de la séquence de ADAM15, la séquence Glu, Asn, Phe, Leu, His, Trp, Arg, Arg, Ala, His, Leu, Leu (ENFLHWRRAHLL) correspondant aux acides aminés 282-293 de la séquence de ADAM15 ou la séquence His, Trp, Arg, Arg, Ala, His, Leu, Leu, Pro, Arg, Leu, Pro (HWRRAHLLPRLP) correspondant aux acides aminés 286-297 de la séquence de ADAM15, le domaine métalloprotéase de ADAM15 et ADAM15 (métargidine),
b) la séparation des protéines ou des peptides mal repliés liés, et
c) l'isolement des protéines ou des peptides mal repliés,
où de préférence l'échantillon est choisi parmi les fluides corporels ou les extraits de tissus comme par exemple le sang, le plasma sanguin sérique, la lymphe, le sperme, le liquide vaginal, le liquide amniotique, le liquide céphalo-rachidien, le liquide synovial, l'urine, les expectorations, le liquide provenant de lavages, comme par exemple le liquide de lavage bronchioalvéolaire et le liquide de lavage péritonéal, ou bien
où l'échantillon est choisi parmi les produits médicaux comme les médicaments, les additifs de médicaments, les compositions pharmaceutiques, les réactifs et les réactifs auxiliaires pour les tests de diagnostic, les réactifs de recherche, les produits alimentaires, les produits de consommation de luxe, les compléments alimentaires, l'eau potable et industrielle et les biofilms.

11. Procédé pour diagnostiquer, stratifier et/ou surveiller des maladies dans lesquelles des protéines ou des peptides mal repliés sont impliqués, *in vitro*, comprenant les étapes
a) détermination qualitative et/ou quantitative de protéines ou de peptides mal repliés dans un échantillon fourni après mise en contact de l'échantillon avec une ou plusieurs substances de liaison choisies dans le groupe consistant en peptides qui comprennent la séquence His, Trp, Arg, Arg (HWRR), la séquence His, Trp, Arg, Arg, Pro (HWRRP), la séquence Ala, Val, Thr, Leu, Glu, Asn, Phe, Leu, His, Trp, Arg, Arg correspondant aux acides aminés 278-289 de la séquence de ADAM15, la séquence Glu, Asn, Phe, Leu, His, Trp, Arg, Arg, Ala, His, Leu, Leu (ENFLHWRRAHLL) correspondant aux acides aminés 282-293 de la séquence de ADAM15 ou la séquence His, Trp, Arg, Arg, Ala, His, Leu, Leu, Pro, Arg, Leu, Pro (HWRRAHLLPRLP) correspondant aux acides aminés 286-297 de la séquence de ADAM15, le domaine métalloprotéase de ADAM15 et ADAM15 (métargidine),
b) comparaison du résultat de la détermination dans l'étape a) avec une ou plusieurs valeurs de référence.

12. Procédé selon la revendication 11,
pour suivre et/ou surveiller une thérapie médicamenteuse, où de préférence la thérapie médicamenteuse comprend un traitement avec l'acide acétylsalicylique, une thiénopyridine comme par exemple le clopidogrel, le prasugrel ou la ticlopidine, des liposomes PEGylés et/ou des inhibiteurs de sPLA2.

13. Kit de diagnostic pour la détermination des protéines ou de peptides mal repliés, comprenant
a) une première substance de liaison choisie dans le groupe consistant en peptides qui comprennent la séquence His, Trp, Arg, Arg (HWRR), la séquence His, Trp, Arg, Arg, Pro (HWRRP), la séquence Ala, Val, Thr, Leu, Glu, Asn, Phe, Leu, His, Trp, Arg, Arg correspondant aux acides aminés 278-289 de la séquence de ADAM15, la séquence Glu, Asn, Phe, Leu, His, Trp, Arg, Arg, Ala, His, Leu, Leu (ENFLHWRRAHLL) correspondant aux acides aminés 282-293 de la séquence de ADAM15 ou la séquence His, Trp, Arg, Arg, Ala, His, Leu, Leu, Pro, Arg, Leu, Pro (HWRRAHLLPRLP) correspondant aux acides aminés 286-297 de la séquence de ADAM15, le domaine métalloprotéase de ADAM15 et ADAM15 (métargidine), et
b) un réactif de détection spécifique pour la protéine ou le peptide mal replié, où le réactif de détection comprend de préférence une autre substance de liaison choisie dans le groupe consistant en peptides qui comprennent la séquence His, Trp, Arg, Arg (HWRR), la séquence His, Trp, Arg, Arg, Pro (HWRRP), la séquence Ala, Val, Thr, Leu, Glu, Asn, Phe, Leu, His, Trp, Arg, Arg correspondant aux acides aminés 278-289 de la séquence de ADAM15, la séquence Glu, Asn, Phe, Leu, His, Trp, Arg, Arg, Ala, His, Leu, Leu (ENFLHWRRAHLL) correspondant aux acides aminés 282-293 de la séquence de ADAM15 ou la séquence His, Trp, Arg, Arg, Ala, His, Leu, Leu, Pro, Arg, Leu, Pro (HWRRAHLLPRLP) correspondant aux acides aminés 286-297 de la séquence de ADAM15, le domaine métalloprotéase de ADAM15 et ADAM15 (métargidine), ou choisie parmi les chaperonnes, les récepteurs piégeurs, t-Pa, FXII, HGFA, le rouge Congo ou la thioflavine, ou un anticorps spécifique contre la protéine ou le peptide,
où l'un des composants a) et b) porte un marqueur pouvant être mis en évidence et l'autre des composants a) et b) est immobilisé sur une phase solide.

14. Procédé pour la détermination de la biocompatibilité d'un matériau *in vitro*, comprenant les étapes:
a) mise en contact du matériau avec une substance de test qui comprend des protéines ou des peptides,
b) détermination qualitative et/ou quantitative de protéines ou de peptides mal repliés dans la substance de test, selon un procédé tel que défini dans l'une des revendications 4 et 5,
c) comparaison de la quantité des protéines ou des peptides mal repliés contenus dans la substance de test avant et après la mise en contact avec le matériau et
d) détermination de ce que le matériau produit des mauvais repliements dans des protéines ou des peptides de la substance de test.

15. Procédé pour la concentration de micro-organismes qui portent des protéines mal repliées sur leur surface à partir d'un échantillon *in vitro*, comprenant les étapes:
a) mise en contact de l'échantillon avec une ou plusieurs substances de liaison choisies dans le groupe consistant en peptides qui comprennent la séquence His, Trp, Arg, Arg (HWRR), la séquence His, Trp, Arg, Arg, Pro (HWRRP), la séquence Ala, Val, Thr, Leu, Glu, Asn, Phe, Leu, His, Trp, Arg, Arg correspondant aux acides aminés 278-289 de la séquence de ADAM15, la séquence Glu, Asn, Phe, Leu, His, Trp, Arg, Arg, Ala, His, Leu, Leu (ENFLHWRRAHLL) correspondant aux acides aminés 282-293 de la séquence de ADAM15 ou la séquence His, Trp, Arg, Arg, Ala, His, Leu, Leu, Pro, Arg, Leu, Pro (HWRRAHLLPRLP) correspondant aux acides aminés 286-297 de la séquence de ADAM15, le domaine métalloprotéase de ADAM15 et ADAM15 (métargidine),
b) séparation des micro-organismes liés de l'échantillon.
